# EUROPEAN PATENT SPECIFICATION

(11) **EP 2 254 462 B1**
(45) Date of publication and mention of the grant of the patent: **20.05.2015**
(21) Application number: 08850612.6
(22) Date of filing: 12.11.2008
(51) Int. Cl.: A61B 5/00, A61N 1/05, A61N 1/36

(54) **APPARATUS FOR PROGRAMMING OF AUTONOMIC NEUROMODULATION FOR THE TREATMENT OF OBESITY**
GERÄT ZUR PROGRAMMIERUNG DER AUTONOMEN NEUROMODULATION ZUR BEHANDLUNG VON ADIPOSITAS
APPAREIL DE PROGRAMMATION D'UNE NEUROMODULATION AUTONOME POUR LE TRAITEMENT DE L'OBÉSITÉ

(30) Priority: 12.11.2007 US 2735 P; 19.11.2007 US 3686 P
(43) Date of publication of application: 01.12.2010
(73) Proprietor: Dilorenzo, Daniel J., Houston, TX 77230 (US)
(72) Inventor: Dilorenzo, Daniel J., Houston, TX 77230 (US)
(74) Representative: Roberts, Mark Peter
(86) International application number: PCT/US2008/012696
(87) International publication number: WO 2009/064408

(56) References cited:
- WO-A2-2006/087712
- WO-A2-2007/007339
- US-A1- 2003 018 367
- US-A1- 2005 049 655
- US-A1- 2005 131 493
- US-A1- 2005 149 146
- US-A1- 2005 149 146
- US-A1- 2005 182 467
- US-A1- 2006 116 736
- US-A1- 2007 016 274
- US-B2- 6 600 953
- US-B2- 6 600 953

## Description

### Background Of The Invention

The present invention relates generally to metabolic disease and neuropsychiatric disease and, more particularly, to stimulation of gastric and autonomic including sympathetic and parasympathetic-neural tissue for the treatment of disease, including but not limited to obesity, eating disorders including anorexia and bulimia, depression, anxiety, epilepsy, metabolic conditions, diabetes, hyperglycemia, hypoglycemia, irritable bowel syndrome, immunological conditions, asthma, respiratory conditions, cardiovascular conditions, cardiac conditions, vascular conditions, headaches, substance abuse, substance addiction, smoking cessation, drug withdrawal, hyperhidrosis, reflex sympathetic dystrophy, pain, and other medical and neurological and psychiatric conditions.

### Related Art

Physiologic studies have demonstrated the presence of a sympathetic nervous system afferent pathway transmitting gastric distention information to the hypothalamus. [Barone, Zarco de Coronado et al. (1995). Gastric distension modulates hypothalamic neurons via a sympathetic afferent path through the mesencephalic periaqueductal gray. Brain Research Bulletin. 38: 239-51.] However, prior techniques have generally not addressed the problems associated with satiety, morbidity, mortality of intracranial modulation and the risk of ulcers. Unlike prior techniques, by specifically targeting sympathetic afferent fibers, the present invention effects the sensation of satiety and avoids the substantial risks of morbidity and mortality of intracranial modulation, particularly dangerous in the vicinity of the hypothalamus. Furthermore, this invention avoids the risk of ulcers inherent in vagus nerve stimulation.

A. Satiety Stimulation of intracranial structures has been proposed and described for the treatment of obesity (US. Patent No. 5,782,798). Stimulation of the left ventromedial hypothalamic (VMH) nucleus resulted in delayed eating by dogs who had been food deprived. Following 24 hours of food deprivation, dogs with VMH stimulation waited between 1 and 18 hours after food presentation before consuming a meal. Sham control dogs ate immediately upon food presentation. Dogs that received 1 hour of stimulation every 12 hours for 3 consecutive days maintained an average daily food intake of 35% of normal baseline levels. [Brown, Fessler et al. (1984). Changes in food intake with electrical stimulation of the ventromedial hypothalamus in dogs. Journal of Neurosurgery.] 60: 1253-7. B. Candidate Peripheral Nerve Pathways for Modulating Satiety.

B1. Sympathetic Afferents. The effect of gastric distension on activity in the lateral hypothalamus-lateral preoptic area-medial forebrain bundle (LPA-LH-MFB) was studied to determine the pathways for this gastric afferent input to the hypothalamus. [Barone, Zarco de Coronado et al. (1995). Gastric distension modulates hypothalamic neurons via a sympathetic afferent path through the mesencephalic periaqueductal gray. Brain Research Bulletin. 38: 239-51.]The periaqueductal gray matter (PAG) was found to be a relay station for this information. [Barone, Zarco de Coronado et al. (1995). Gastric distension modulates hypothalamic neurons via a sympathetic afferent path through the mesencephalic periaqueductal gray. Brain Research Bulletin. 38: 239-51.] This modulation of the hypothalamus was attenuated but not permanently eliminated by bilateral transection of the vagus nerve. This modulation was, however, significantly reduced or eliminated by bilateral transection of the cervical sympathetic chain or spinal transection at the first cervical level. [Barone, Zarco de Coronado et al. (1995). Gastric distension modulates hypothalamic neurons via a sympathetic afferent path through the mesencephalic periaqueductal gray. Brain Research Bulletin. 38:239-51.] These signals containing gastric distension and temperature stimulation are mediated to a large degree by sympathetic afferents, and the PAG is a relay station for this gastric afferent input to the hypothalamus. [Barone, Zarco de Coronado et al. (1995). Gastric distension modulates hypothalamic neurons via a sympathetic afferent path through the mesencephalic periaqueductal gray. Brain Research Bulletin. 38: 239-51.] For example, in the LPA-LH-MFB study, 26.1% of the 245 neurons studied were affected by gastric stimulation, with 17.6% increasing in firing frequency and 8.6% decreasing during gastric distension. [Barone, Zarco de Coronado et al. (1995). Gastric distension modulates hypothalamic neurons via a sympathetic afferent path through the mesencephalic periaqueductal gray. Brain Research Bulletin. 38: 239-51.] The response of 8 of 8 neurons sensitive to gastric distension were maintained, though attenuated after bilateral vagus nerves were cut. In 2 of these 8 cells, the effect was transiently eliminated for 2-4 minutes after left vagus transection, and then activity recovered. In 3 LH-MFB cells, two increased and the other decreased firing rate with gastric distension. Following bilateral sympathetic ganglion transection, the response of two were eliminated, and the third (which increased firing with distension) had a significantly attenuated response. [Barone, Zarco de Coronado etal. (1995). Gastric distension modulates hypothalamic neurons via a sympathetic afferent path through the mesencephalic periaqueductal gray. Brain Research Bulletin. 38: 239-51.] Vagus stimulation resulted in opposite or similar responses as gastric distension on the mesencephalic cells.

B2. Vagus Nerve Afferents. Gastric vagal input to neurons throughout the hypothalamus has been characterized. [Yuan and Barber (1992). Hypothalamic unitary responses to gastric vagal input from the proximal stomach. American Journal of Physiology. 262: G74-80.] Nonselective epineural vagus nerve stimulation (VNS) has been described for the treatment of Obesity (US Patent 5,188,104). This suffers from several significant limitations that are overcome by the present invention.

The vagus nerve is well known to mediate gastric hydrochloric acid secretion. Dissection of the vagus nerve off the stomach is often performed as part of major gastric surgery for ulcers. Stimulation of the vagus nerve may pose risks for ulcers in patients, of particular concern, as obese patients often have gastroesophageal reflux disease (GERD); further augmentation of gastric acid secretion would only exacerbate this condition.

C. Assessment of Sympathetic and Vagus Stimulation. The present invention teaches a significantly more advanced neuroelectric interface technology to stimulate the vagus nerve and avoid the efferent vagus side effects, including speech and cardiac side effects common in with existing VNS technology as well as the potential ulcerogenic side effects. However, since sympathetic afferent activity appears more responsive to gastric distension, this may represent a stronger channel for modulating satiety. Furthermore, by pacing stimulating modulators on the greater curvature of the stomach, one may stimulate the majority of the circular layer of gastric musculature, thereby diffusely increasing gastric tone.

D. Neuromuscular Stimulation. The muscular layer of the stomach is comprised of 3 layers: (1) an outer longitudinal layer, (2) a circular layer in between, and (3) a deeper oblique layer. [Gray (1974). Gray's Anatomy. T. Pick and R. Howden. Philadelphia, Running Press.] The circular fibers, which lie deep to the superficial longitudinal fibers, would appear to be the layer of choice for creating uniform and consistent gastric contraction with elevated wall tension and luminal pressure. Therefore, modulators should have the ability to deliver stimulation through the longitudinal layer. If the modulator is in the form of an electrode, then the electrodes should have the ability to deliver current through the longitudinal layer.

Gray's Anatomy describes innervation as including the right and left pneumogastric nerves (not the vagus nerves), being distributed on the back and front of the stomach, respectively. A great number of branches from the sympathetic nervous system also supply the stomach. [Gray (1974). Gray's Anatomy. T. Pick and R. Howden. Philadelphia, Running Press.] Metabolic Modulation (Efferent) Electrical stimulation of the VMH enhances lipogenesis in the brown adipose tissue (BAT), preferentially over the white adipose tissue (WAT) and liver, probably through a mechanism involving activation of the sympathetic innervation of the BAT. [Takahashi and Shimazu (1982). Hypothalamic regulation of lipid metabolism in the rat: effect of hypothalamic stimulation on lipogenesis. Journal of the Autonomic Nervous System. 6: 225-35.] The VMH is a hypothalamic component of the sympathetic nervous system. [Ban (1975). Fiber connections in the hypothalamus and some autonomic functions. Pharmacology, Biochemistry & Behavior. 3: 3-13.] A thermogenic response in BAT was observed with direct sympathetic nerve stimulation. [Flaim, Horwitz et al. (1977). Coupling of signals to brown fat: a- and b-adrenergic responses in intact rats. Amer. J. Physiol. 232: R101-R109.] The BAT had abundant sympathetic innervation with adrenergic fibers that form nest-like networks around every fat cell, [Derry, Schonabum et al. (1969). Two sympathetic nerve supplies to brown adipose tissue of the rat. Canad. J. Physiol. Pharmacol. 47: 57-63.] whereas WAT has no adrenergic fibers in direct contact with fat cells except those related to the blood vessels. [Daniel and Derry (1969). Criteria for differentiation of brown and white fat in the rat. Canad. J. Physiol. Pharmacol. 47: 941-945.]

### Summary of the Invention

The present invention teaches apparatus and methods for treating a multiplicity of diseases, including obesity, depression, epilepsy, diabetes, and other diseases. The invention taught herein employs a variety of energy modalities to modulate central nervous system structures, peripheral nervous system structures, and peripheral tissues and to modulate physiology of neural structures and other organs, including gastrointestinal, adipose, pancreatic, and other tissues. The methods for performing this modulation, including the sites of stimulation and the modulator configurations are described. The apparatus for performing the stimulation are also described. This invention teaches a combination of novel anatomic approaches and apparatus designs for direct and indirect modulation of the autonomic nervous system, which is comprised of the sympathetic nervous system and the parasympathetic nervous system.

For the purposes of this description the term GastroPace™ should be interpreted to mean the devices constituting the system of the present embodiment of this invention, including the obesity application as well as others described, implied, enabled, facilitated, and derived from those taught in the present invention.

A. Obesity and Eating Disorders. The present invention teaches several mechanisms, including neural modulation and direct contraction of the gastric musculature, to effect the perception of satiety. This modulation is useful in the treatment of obesity and eating disorders, including anorexia nervosa and bulimia.

Direct stimulation of the gastric musculature increases the intraluminal pressure within the stomach; and this simulates the physiologic condition of having a full stomach, sensed by stretch receptors in the muscle tissue and transmitted via neural afferent pathways to the hypothalamus and other central nervous system structures, where the neural activity is perceived as satiety.

This may be accomplished with the several alternative devices and methods taught in the present invention. Stimulation of any of the gastric fundus, greater curvature of stomach, pyloric antrum, or lesser curvature of stomach, or other region of the stomach or gastrointestinal tract, increases the intraluminal pressure. Increase of intraluminal pressure physiologically resembles fullness of the respective organ, and satiety is perceived.

The present invention also includes the restriction of the flow of food to effect satiety. This is accomplished by stimulation of the pylorus. The pylorus is the sphincter-like muscle at the distal juncture of the stomach with the duodenum, and it regulates food outflow from the stomach into the duodenum. By stimulating contraction of the pylons, food outflow from the stomach is slowed or delayed. The presence of a volume of food in the stomach distends the gastric musculature and causes the person to experience satiety.

B. Depression and Anxiety. An association has been made between depression and overeating, particularly with the craving of carbohydrates; and is believed to be an association between the sense of satiety and relief of depression. Stimulation of the gastric tissues, in a manner that resembles or is perceived as satiety, as described above, provides relief from this craving and thereby relief from some depressive symptoms. There are several mechanisms, including those taught above for the treatment of obesity that are applicable to the treatment of depression, anxiety, agoraphobia, social anxiety, panic attacks, and other neurological and psychiatric conditions.

An object of the present invention, as taught in the parent case, is the modulation of the autonomic nervous system for physiologic modulation, including modulation of limbic physiology, which has efficacy in the treatment of depression, anxiety and other psychiatric conditions. By altering the level of sympathetic nervous system activity, or the level of parasympathetic nervous system activity, or the ratio of sympathetic to parasympathetic nervous system activity (as reflected in metrics such as the autonomic index), the level of activity n the locus ceruleus, solitary nucleus, cingulate nucleus, the limbic system, the supraorbital cortex, and other regions may be modulated, thereby influencing affect or mood as well as level of anxiety. Furthermore, the reduction of systemic sympathetic activity may be used to alleviate the symptoms of anxiety, which is employed in both the treatment of anxiety and in the conditioning of patients to control anxiety.

C. Epilepsy. The present invention includes electrical stimulation of peripheral nervous system and other structures and tissues to modulate the activity in the central nervous system to control seizure activity.

This modulation takes the form of peripheral nervous system stimulation using a multiplicity of novel techniques and apparatus. Direct stimulation of peripheral nerves is taught; this includes stimulation of the vagus, trigeminal, accessory, and sympathetic nerves. Indiscriminate stimulation of the vagus nerves has been described for some disorders, but the limitations in this technique are substantial, including cardiac rhythm disruptions, speech difficulties, and gastric and duodenal ulcers. The present invention overcomes these persistent limitations by teaching a method and apparatus for the selective stimulation of structures, including the vagus nerve as well as other peripheral nerves, and other neural, neuromuscular, and other tissues.

The present invention further includes noninvasive techniques for neural modulation. This includes the use of tactile stimulation to activate peripheral or cranial nerves. This noninvasive stimulation includes the use of tactile stimulation, including light touch, pressure, vibration, and other modalities that may be used to activate the peripheral or cranial nerves. Temperature stimulation, including hot and cold, as well as constant or variable temperatures, are included in the present invention.

D. Diabetes. The response of the gastrointestinal system, including the pancreas, to a meal includes several phases. The first phase, the anticipatory stage, is neurally mediated. Prior to the actual consumption of a meal, saliva production increases and the gastrointestinal system prepares for the digestion of the food to be ingested. Innervation of the pancreas, in an analogous manner, controls production of insulin.

Modulation of pancreatic production of insulin may be performed by modulation of at least one of afferent or efferent neural structures. Afferent modulation of at least one of the vagus nerve, the sympathetic structures innervating the gastrointestinal tissue, the sympathetic trunk, and the gastrointestinal tissues themselves is used as an input signal to influence central and peripheral nervous system control of insulin secretion.

E. Irritable bowel Syndrome. An object of the present invention, as taught in the parent case, is the modulation of the autonomic nervous system for physiologic modulation, including modulation of gastrointestinal physiology, which has efficacy in the treatment of irritable bowel syndrome. By altering the level of sympathetic nervous system activity, or the level of parasympathetic nervous system activity, or the ratio of sympathetic to parasympathetic nervous system activity (as reflected in metrics such as the autonomic index), the level of gastrointestinal motility and absorption may be modulated.

Modulation including down-regulation of the activity of the gastrointestinal tract, through autonomic modulation, as taught in the parent case has application to the treatment of irritable bowel syndrome. Said autonomic modulation includes but is not limited to inhibition or blocking of sympathetic nervous system activity and to enhancement or stimulation of parasympathetic nervous system activity.

The response of the gastrointestinal system to sympathetic stimulation, such as that induced by stress or sympathomimetic agents including caffeine, may include symptoms such as elevated motility and altered absorption. Modulation of gastrointestinal physiology is taught for applications including but not limited to the maintenance of baseline levels of gastrointestinal motility, secretion, absorption, and hormone release. Modulation of gastrointestinal physiology is also taught for applications including but not limited to the real-time control of levels of gastrointestinal motility, secretion, absorption, and hormone release, in response to physiological needs as well as in response to perturbations. Such external perturbation that can induce symptoms that are alleviated by the present invention include but are not limited to stress, consumption of caffeine, alcohol, or other substance, consumption of allergenic substance, or consumption of infectious or toxic agent. By intervening with the application of autonomic modulation to counter these undesirable autonomic responses to external agents, these side effects are reduced or prevented.

F. Immmunomodulation. An object of the present invention, as taught in the parent case, is the modulation of the autonomic nervous system for physiologic modulation, including modulation of immune system physiology. By altering the level of sympathetic nervous system activity, or the level of parasympathetic nervous system activity, or the ratio of sympathetic to parasympathetic nervous system activity (as reflected in metrics such as the autonomic index), the level of activity of the immune system may be modulated. Both polarities of modulation have efficacy in the treatment of disease as well as in prophylactic applications.

Modulation, including up-regulation of the immune system, through autonomic modulation, as taught in the parent case invention has application to the treatment of infection, cancer, autoimmune immunodeficiency syndrome (AIDS), human immunodeficiency virus) infection (HIV), severe combined immunodeficiency (SCID), other causes of immunodeficiency, other causes of immunosuppression, mitigation of effects of iatrogenic immunosupppression (including that used with organ transplantation or for treating autoimmune disorders), and other causes of decreased immune system activity.

Modulation, including down-regulation, of the immune system, through autonomic modulation, as taught in the parent case invention has application to the treatment of autoimmune disease, including but not limited to multiple sclerosis, reflex sympathetic dystrophy (RSD), type I diabetes (the pathophysiology of which may include an autoimmune component), rheumatoid arthritis, graft versus host disease, psoriasis, allergic reactions, dermatitis, other allergic conditions, other diseases involving signs or symptoms due to an autoimmune or other immune pathology, and other diseases with untoward effects arising from excessive or detrimental immune responses.

Modulation, including down-regulation, of the immune system, through autonomic modulation, as taught in the parent case invention has application to the treatment of some complications from infection, including but not limited to lyme disease, streptococcal pharyngitis (strep throat), rheumatic heart disease, fungal infections, parasitic infections, bacterial infections, viral infections, other infections, and other exposures to infectious or allergenic agents.

Modulation, including down-regulation, of the immune system, through autonomic modulation, as taught in the parent case invention has application to the augmentation of other therapies, and may be used to suppress immune function in patients with organ transplantation.

G. Asthma An object of the present invention, as taught in the parent case, is the modulation of the autonomic nervous system for physiologic modulation, including modulation of pulmonary physiology. By altering the level of sympathetic nervous system activity, or the level of parasympathetic nervous system activity, or the ratio of sympathetic to parasympathetic nervous system activity (as reflected in metrics such as the autonomic index), the level of activity of the immune system may be modulated. Both polarities of modulation have efficacy in the treatment of disease as well as in prophylactic applications.

Modulation, including stimulation of the sympathetic nervous system, as taught in the parent case invention has application to the treatment of asthma, including exercise induced asthma and other forms of asthma. Through stimulation of the sympathetic nervous system, the beta-2 efferent pathways of the sympathetic nervous system are activated, effecting bronchodiulation, providing a therapeutic action opposing the bronchoconstrictive process that underlies the increased airway resistance which results in the potentially life-threatening signs and symptoms of this disease. This same therapy is also applied to the treatment of bronchospasm and laryngospasm, in which elevated sympathetic efferent activity mitigates the constrictive effects on the airway.

Modulation, including stimulation of the sympathetic nervous system and stimulation of the parasympathetic nervous system, as taught in the parent case invention has application to the treatment of asthma, including exercise induced asthma through an additional mechanism. Through inhibition of the sympathetic nervous system, the activity of the immune system may be down-regulated, reducing the sensitivity of the pulmonary mast cells to allergens, thereby reducing the susceptibility to and the severity of asthma signs and symptoms.

H. Cardiovascular Disease - Cardiac. An object of the present invention, as taught in the parent case, is the modulation of the autonomic nervous system for physiologic modulation, including modulation of cardiovascular physiology, including cardiac physiology in particular. By altering the level of sympathetic nervous system activity, or the level of parasympathetic nervous system activity, or the ratio of sympathetic to parasympathetic nervous system activity (as reflected in metrics such as the autonomic index), cardiac parameters may be modulated. Both polarities of modulation have efficacy in the treatment of cardiac disease as well as in prophylactic applications.

Modulation, including stimulation of the sympathetic nervous system, inhibition of the parasympathetic system, or increase in the autonomic index, as taught in the parent case invention has application to the treatment of cardiac disease, including hear failure and bradycardia. Through stimulation of the sympathetic nervous system, the beta-1 efferent pathways of the sympathetic nervous system are activated, effecting increase inotropic activity, providing a therapeutic action to mitigate decreased myocardial contractility found in cardiac disease, including congestive heart failure, post myocardial infarction sequelae, and other cardiac disorders. Sympathetic stimulation is also used to effect increased chronotropic behavior, thereby elevating heart rate. This has application to numerous cardiac conditions, including bradycardia and heart block. This has further application to the treatment of hypotension and to neurogenic shock, which may be augmented by autonomic neuromodulation directed toward the vascular system, as described below.

Modulation, including inhibition of the sympathetic nervous system, stimulation of the parasympathetic system, or decrease in the autonomic index, as taught in the parent case invention has application to the treatment of cardiac disease. The negative inotropic effect of such autonomic modulation has application to cardiac disease, including among others, diastolic disease, in which the heart muscle does not fully relax, thereby impairing proper atrial and ventricular filling during the diastolic portion of the cardiac cycle. This additionally has application to the treatment of hypertension, through each of negative inotropic and negative chronotropic effects. This further has application to the prevention and control of the progression of congestive heart failure, through the reduction of the normal sympathetic physiologic response to heart failure, which itself contributes to progression of the disease. The negative chronotropic effect of such modulation also has application to the treatment of tachycardia and other cardiac rhythm abnormalities.

I. Cardiovascular Disease - Vascular. An object of the present invention, as taught in the parent case, is the modulation of the autonomic nervous system for physiologic modulation, including modulation of cardiovascular physiology including vascular physiology in particular. By altering the level of sympathetic nervous system activity, or the level of parasympathetic nervous system activity, or the ratio of sympathetic to parasympathetic nervous system activity (as reflected in metrics such as the autonomic index), the level of activity including the muscular tone of the vascular system may be modulated. Both polarities of modulation have efficacy in the treatment of disease as well as in prophylactic applications.

Modulation, including stimulation of the sympathetic nervous system, inhibition of the parasympathetic nervous system, or increase in the autonomic index, as taught in the parent case invention has application to the treatment of hypotension and neurogenic shock, and other conditions in which vascular tone or blood pressure is below normal. This further has application to therapeutically increase vascular tone or blood pressure, including to levels above normal, such as in the treatment of cerebral vasospasm, ischemic stroke, peripheral vascular disease, or other condition. Through stimulation of the sympathetic nervous system, the alpha-1 efferent pathways of the sympathetic nervous system are activated, effecting vasoconstriction, providing a therapeutic action to correct low blood pressure as well as to provide a normalizing to correct low vascular tone characterizing neurogenic shock as well as to elevate blood pressure to treat the above listed conditions. A particular advantage of this therapy is conveyed by the ability to selectively rather than systemically induce vasoconstriction, thereby elevating systemic blood pressure while avoiding vasoconstridion in selected circulatory regions, as desired in the treatment of cerebral vasospasm.

Modulation, including inhibition of the sympathetic nervous system, stimulation of the parasympathetic nervous system, or decrease in the autonomic index, as taught in the parent case invention has application to the treatment of hypertension, including essential hypertension, renally mediated hypertension, atherosderosis mediated hypertension, other forms of systemic hypertension, and pulmonary hypertension. Through this therapy, vasodilation is achieved, which is also used to treat coronary artery disease, peripheral vascular disease, cerebral vascular disease, myocardial infarction, and stroke. This has further use in other therapy in which enhanced circulation is desired, such as for enhanced circulation and drug delivery in the treatment of infections and as an adjuvant to accelerate healing processes, such as ulcers, postoperative wounds, trauma, and other conditions.

J. Headaches. An object of the present invention, as taught in the parent case, is the modulation of the autonomic nervous system for physiologic modulation, including modulation of cerebral vascular physiology, including intraparenchymal circulation and meningeal circulation. By altering the level of sympathetic nervous system activity, or the level of parasympathetic nervous system activity, or the ratio of sympathetic to parasympathetic nervous system activity (as reflected in metrics such as the autonomic index), the level of activity of the cerebral vascular system may be modulated. Both polarities of modulation have efficacy in the treatment of headaches as well as in prophylactic applications.

Modulation, including stimulation of the sympathetic nervous system, inhibition of the parasympathetic nervous system, or increase in the autonomic index, as taught in the parent case invention has application to the treatment of headaches, including migraine headaches, cluster headaches, and other headaches. Through stimulation of the sympathetic nervous system, the alpha-1 efferent pathways of the sympathetic nervous system are activated, effecting cerebral vasoconstriction, providing decrease in the blood volume within the intracranial vascular structures as well as the remainder of the intracranial compartment. This acts through additional mechanisms including but not limited to reduction of the mechanical tension on the dura, reduction of the intracranial pressure, and alteration in the blood flow and neural activity within the brain, altering neural and vascular patterns that can progress to generate headaches or other undesirable neural states.

Modulation, including inhibition of the sympathetic nervous system, stimulation of the parasympathetic nervous system, or decrease in the autonomic index, as taught in the parent case invention has application to the prophylaxis and treatment of headaches, including migraine headaches, cluster headaches, and other headaches. Through inhibition of the sympathetic nervous system, the activity of alpha-1 efferent pathways of the sympathetic nervous system are reduced, effecting cerebral vasodilation, providing variation in the vascular tone as well as altered blood flow and neural activity, which has application to disrupt neural and vascular patterns that can generate headaches or other undesirable neural states.

K. Smoking Cessation and Drug Withdrawal. An object of the present invention, as taught in the parent case, is the modulation of the autonomic nervous system, which has application to stabilize or oppose the physiologic response to the introduction or withdrawal of pharmacological or other bioactive agents, including nicotine, caffeine, stimulants, depressants, and other medical and recreational drugs.

When patients cease smoking, the nicotine plasma levels drop, reducing the level of stimulation of the nicotinic receptors in the sympathetic nervous system. This alteration causes a physiologic response characterized by significant levels of anxiety and a withdrawal response in the person. By modulating the sympathetic nervous system activity using the method and apparatus taught in the parent case or using variants thereof, this response can be mitigated. This has application to controlling addiction to nicotine and in the facilitation of smoking cessation.

When patients cease intake of alcohol, narcotics, sedatives, hypnotics, or other drugs to which they may be addicted, a withdrawal response ensues. This response can be life threatening. In alcohol withdrawal, delirium tremens can be accompanied by dangerous elevations in heart rate. By modulating sympathetic and/or parasympathetic activity to control the autonomic index, this response can be reduced or prevented.

L. Hyperhidrosis. An object of the present invention, as taught in the parent case, is the modulation of the autonomic nervous system, which has application to prevent or control the symptoms of hyperhidrosis.

In hyperhidrosis, a abnormally active or responsive sympathetic nervous system results is excessive perspiration, typically most problematic when involving the hands and axillae. Current treatments employ surgical ablation for the corresponding region of the sympathetic trunk, which results in irreversible cessation of sympathetic activity in the corresponding anatomical region. By modulating the sympathetic nervous system activity using the method and apparatus taught in the parent case or using variants thereof, the symptoms arising from this condition can be prevented or reduced.

M. Reflex Sympathetic Dystropy and Pain. An object of the present invention, as taught in the parent case, is the modulation of the autonomic nervous system, which has application to prevent the development or progression of reflex sympathetic dystrophy and to control the symprtoms once the condition has developed.

Reflex sympathetic dystrophy is a potentially debilitating condition that typically develops following trauma to a peripheral nerve, in which a crush or transection injury disrupts the afferent pain fibers and the sympathetic efferent fibers. The most widely accepted theory as to the etiology underlying this condition holds that during the healing phase, sympathetic efferent fibers develop connections with the pain carrying afferent fibbers, resulting in the perception of pain in response to sympathetic activity. Cureent therapy involves pharmacologal agents and is largely ineffective, leaving a population of otherwise often healthy people who are debiliatated by severe chronic medication refractory pain. By modulating the sympathetic nervous system activity using the method and apparatus taught in the parent case orusing variants thereof, the symptoms arising from reflex sympathetic dystrophy can be prevented or reduced.

Inhibition of sympathetic system activity is used to reduce the level of neural activity that is pathologically fed back into pain afferent fibers, thereby reducing symptoms. This therapy may be applied preventatively to modulate sympathetic nervous system activity and minimize the degree of neural connection between the sympathetic efferent neurons and the pain carrying afferent neurons.

N. General - Control and Temporal Modulation. Various forms of temporal modulation may be performed to achieve the desired efficacy in the treatment of these and other diseases, conditions, or augmentation applications. Constant intensity modulation, time varying modulation, cyclical modulation, altering polarity modulation, up-regulation interspersed with down-regulation, intermittent modulation, and other permutations are include in the present invention. The use of a single or multiplicity of these temporal profiles provides resistance of the treatment or enhancement to habituation by the nervous system, thereby preserving or prolonging the effect of the modulation. The use of a multiplicity of modulation sites provides resistance of the treatment or enhancement to habituation by the nervous system, thereby preserving or prolonging the effect of the modulation; by distributing or varying the intensity of the neuromodulation among a plurality of sites enables the delivery of therapy or augmentation that is more resistant to adaptation or habituation by the nervous system. Furthermore, the control of neural state, including level of sympathetic nervous system activity, level of parasympathetic nervous system activity, autonomic index, or other characteristic or metric of neural function in either or both of an open-loop or closed-loop manner is taught herein. The use of open-loop or closed-loop control to maintain at least one neural state at a constant or time varying target level is used to better control physiology, reduce habituation, reduce side effects, apportion side effect to preferable time windows such as while sleeping), and optimize response to therapy.

### Brief Description Of Drawings

Figure 1 depicts GastroPace™ implanted along the Superior Greater Curvature of the stomach for both Neural Afferent and Neuromuscular Modulation.
Figure 2 depicts GastroPace™ implanted along the Inferior Greater Curvature of the stomach for both Neural Afferent and Neuromuscular Modulation.
Figure 3 depicts GastroPace™ implanted along the PyloricAntrum of the stomach for both Neural Afferent and Neuromuscular Modulation.
Figure 4 depicts GastroPace™ implanted adjacent to the Gastric Pylorus for modulation of pylorus activity and consequent control of gastric food efflux and intraluminal pressure.
Figure 5 depicts GastroPace™ implanted along the PyloricAntrum of the stomach with modulators positioned for stimulation of Neural and Neuromuscular structures of the Pylorus and Pyloric Antrum of the Stomach.
Figure 6 depicts GastroPace™ implanted along the PyloricAntrum of the stomach with modulators positioned for stimulation of Neural and Neuromuscular structures of the Pylorus, Pyloric Antrum, Greater Curvature, and Lesser Curvature of the Stomach.
Figure 7 depicts the Nerve Cuff Electrode, comprising the Epineural Electrode Nerve Cuff Design.
Figure 8 depicts the Nerve Cuff Electrode, comprising the Axial Electrode Blind End Port Design.
Figure 9 depicts the Nerve Cuff Electrode, comprising the Axial Electrode Regeneration Port Design.
Figure 10 depicts the Nerve Cuff Electrode, comprising the Axial Regeneration Tube Design.
Figure 11 depicts GastroPace™ implanted along the Pyloric Antrum of the stomach with modulators positioned for stimulation of Afferent Neural Structures, including sympathetic and parasympathetic fibers.
Figure 12 depicts GastroPace™ implanted along the Pyloric Antrum of the stomach with modulators positioned for stimulation of Neural and Neuromuscular structures of the Pylorus, Pyloric Antrum, Greater Curvature, and Lesser Curvature of the Stomach and with modulators positioned for stimulation of Afferent Neural Structures, including sympathetic and parasympathetic fibers.
Figure 13 depicts the Normal Thoracoabdominal anatomy as seen via a saggital view of an open dissection.
Figure 14 depicts modulators for GastroPace™ positioned on the sympathetic trunk and on the greater and lesser splanchnic nerves, both supradiaphragmatically and infradiaphragmatically, for afferent and efferent neural modulation.
Figure 15 depicts GastroPace™ configured with multiple pulse generators, their connecting cables, and multiple modulators positioned on the sympathetic trunk and on the greater and lesser splanchnic nerves, both supradiaphragmatically and infradiaphragmatically, for afferent and efferent neural modulation.
Figure 16 depicts GastroPace™ configured with multiple pulse generators, their connecting cables, and multiple modulators positioned on the sympathetic trunk and on the greater and lesser splanchnic nerves, both supradiaphragmatically and infradiaphragmatically, for afferent and efferent neural modulation and with modulators positioned for stimulation of Neural and Neuromuscular structures of the Pylorus, Pyloric Antrum, Greater Curvature, and Lesser Curvature of the Stomach.
Figure 17 depicts the Normal Spinal Cord Anatomy, shown in Transverse Section.
Figure 18 depicts GastroPace™ implanted with multiple modulators positioned for modulation of Spinal Cord structures
Figure 19 depicts the three muscle layers of the stomach.
Figure 20 depicts GastroPace™ with modulators implanted along the surface of the stomach.
Figure 21 depicts GastroPace™ with an array of modulators implanted along the surface of the stomach.
Figure 22 depicts a GastroPace™ array, with multiple pulse generators implanted. This figure is exemplary, with two pulse generators shown each in the thorax and abdomen, each connected to modulators.
Figure 23 depicts GastroPace™, with two pulse generators shown in an exemplary configuration in the abdomen, each connected to modulators.
Figure 24 depicts GastroPace™, in a close up view of modulators implanted in the abdomen.
Figure 25 depicts GastroPace™, in a close up view of modulators implanted in the abdomen.
Figure 26 depicts GastroPace™, in a close up view of modulators and modulator arrays implanted in the abdomen.
Figure 27 depicts GastroPace™, in a close up view of the modulators implanted adjacent to the spinal cord, spinal nerves, dorsal root ganglia, and adjacent structures.
Figure 28 depicts GastroPace™, in a detailed view of that shown in the parent case in Figure 15, with more detail of the modulators shown. This figure shows exemplary modulators of the design shown in Figure 7.
Figure 29 depicts GastroPace™, in a detailed view of that shown in the parent case in Figure 15, with more detail of the modulators shown. This figure shows exemplary modulators similar to the catheter design shown in Figure 35.
Figure 30 depicts GastroPace™, in a detailed view of that shown in the parent case in Figure 15, with more detail of the modulators shown. This figure shows exemplary modulators a wireless catheter design.
Figure 31 depicts GastroPace™, in a detailed view of that shown in the parent case in Figure 15, with more detail of the modulators shown. This figure shows exemplary modulators a wireless cylindrical or injectable implant design.
Figure 32 depicts GastroPace™, in a detailed view of that shown in the parent case in Figure 15, with more detail of the modulators shown. This figure shows exemplary modulators similar to the catheter design shown in Figure 35.
Figure 33 depicts electrode catheter being implanted with surgical tools.
Figure 34 depicts electrode catheter being implanted with surgical tools.
Figure 35 depicts neuromodulatory interface array catheter in detailed view.
Figure 36 depicts neurophysiological effects of GastroPace™ functions, with view of time course of response of autonomic index to modulation of at least one of sympathetic and parasympathetic nervous systems.
Figure 37 is a schematic diagram of one embodiment of the present invention implanted in a human patient.
Figure 38 is an architectural block diagram of one embodiment of the neurological control system of the present invention.
Figure 39 is a schematic diagram of electrical stimulation waveforms for neural modulation.
Figure 40 is a schematic diagram of one example of the recorded waveforms.
Figure 41 is a plot showing metabolic parameters and weight versus time.
Figure 42 is a plot showing satiety parameters and weight versus time.
Figure 43 is a diagram of one implementation of an Autonomic Neuromodulation Programmer.
Figure 44 is a diagram of one implementation of an Autonomic Neuromodulation Patient Interface.
Figure 45 is a diagram of one implementation of an Autonomic Neuromodulation Patient Interface.

### Detailed Description Of The Invention

The present invention encompasses a multimodality technique, method, and apparatus for the treatment of several diseases, including but not limited to obesity, eating disorders, depression, epilepsy, and diabetes.

These modalities may be used for diagnostic and therapeutic uses, and these modalities include but are not limited to stimulation of gastric tissue, stimulation of gastric musculature, stimulation of gastric neural tissue, stimulation of sympathetic nervous tissue, stimulation of parasympathetic nervous tissue, stimulation of peripheral nervous tissue, stimulation of central nervous tissue, stimulation of cranial nervous tissue, stimulation of skin receptors, including Pacinian corpuscles, nociceptors, golgi tendons, and other sensory tissues in the skin, subcutaneous tissue, muscles, and joints.

Stimulation may be accomplished by electrical means, optical means, electromagnetic means, radiofrequency means, electrostatic means, magnetic means, vibrotactile means, pressure means, pharmacologic means, chemical means, electrolytic concentration means, thermal means, or other means for altering tissue activity.

Already encompassed in the above description are several specific applications of this broad technology. These specific applications include electrical stimulation of gastric tissue, including at least one of muscle and neural, for the control of appetite and satiety, and for the treatment of obesity. Additional specific uses include electrical stimulation of gastric tissue for the treatment of depression. Further uses include electrical stimulation of pancreatic tissue for the treatment of diabetes.

### A. Satiety Modulation.

A1. Sympathetic Afferent Stimulation. Selected stimulation of the sympathetic nervous system is an objective of the present invention. A variety of modulator designs and configurations are included in the present invention and other designs and configurations may be apparent to those skilled in the art and these are also included in the present invention. Said modulator may take the form of electrode or electrical source, optical source, electromagnetic source, radiofrequency source, electrostatic source, magnetic source, vibrotactile source, pressure source, pharmacologic source, chemical source, electrolyte source, thermal source, or other energy or stimulus source.

One objective of the modulator design for selective sympathetic nervous system stimulation is the avoidance of stimulation of the vagus nerve. Stimulation of the vagus nerve poses the risk enhanced propensity for development of gastric or duodenal ulcers.

Other techniques in which electrical stimulation has been used for the treatment of obesity have included stimulation of central nervous system structures or peripheral nervous system structures. Other techniques have used sequential stimulation of the gastric tissue to interrupt peristalsis; however, this broad stimulation of gastric tissue necessarily overlaps regions heavily innervated by the vagus nerve and consequently poses the same risks of gastric and duodenal ulcers that stimulation of the vagus nerve does.

One objective of the present invention is the selective stimulation of said afferent neural fibers that innervate gastric tissue. Avoidance of vagus nerve stimulation is an object of this modulator configuration. Other alternative approaches to gastric pacing involving gastric muscle stimulation secondarily cause stimulation of the vagus nerve as well as stimulation of gastric tissues in acid-secreting regions, consequently posing the undesirable side effects of gastric and duodenal ulcers secondary to activation of gastric acid stimulation.

There are a number of approaches to selective stimulation of the sympathetic nervous system. This invention includes stimulation of the sympathetic fibers at sites including the zones of innervation of the stomach, the gastric innervation zones excluding those innervated by vagus branches, the distal sympathetic branches proximal to the stomach, the sympathetic trunk, the intermediolateral nucleus, the locus ceruleus, the hypothalamus, and other structures comprising or influencing sympathetic afferent activity.

Stimulation of the sympathetic afferent fibers elicits the perception of satiety, and achievement of chronic, safe, and efficacious modulation of sympathetic afferents is one of the major objectives of the present invention.

Alternating and augmenting stimulation of the sympathetic nervous system and vagus nerve is included in the present invention. By alternating stimulation of the vagus nerve and the sympathetic afferent fibers, one may induce the sensation of satiety in the implanted patient while minimizing the potential risk for gastric and duodenal ulcers.

Since vagus and sympathetic afferent fibers carry information that is related to gastric distention, a major objective of the present invention is the optimization stimulation of the biggest fibers, the afferent sympathetic nervous system fibers, and other afferent pathways such that a maximal sensation of satiety is perceived in the implanted individual and such that habituation of this sensation of satiety is minimized. This optimization is performed in any combination of matters including temporal patterning of the individual signals to each neural pathway, including but not limited to the vagus nerve and sympathetic afferents, as well as temporal patterning between a multiplicity of stimulation channels involving the same were neural pathways The present invention teaches a multiplicity of apparatus and method for stimulation of afferent sympathetic fibers, as detailed below. Other techniques and apparatus may become apparent to those skilled in the art, without departing from the present invention.

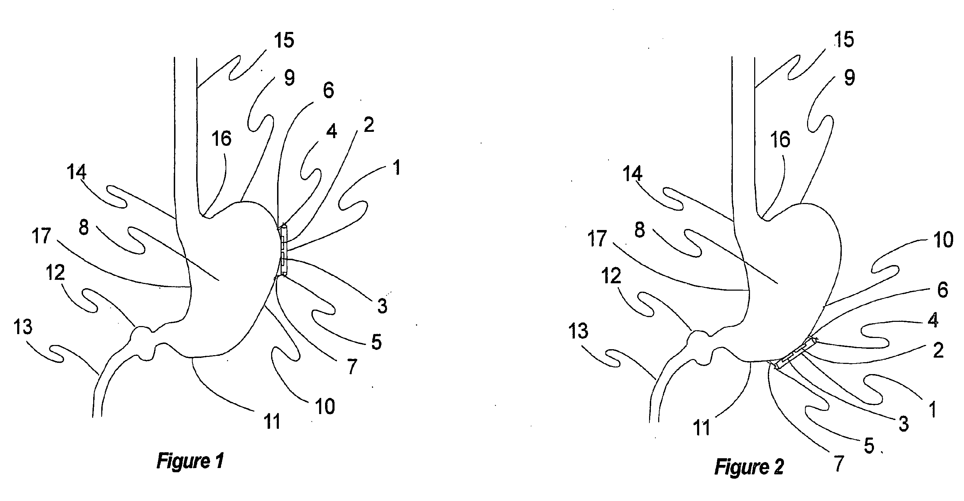

A1a. Sympathetic Afferents - Gastric Region. Figure 1 through Figure 3 demonstrate stimulation of gastric tissue, including at least one of neural and muscular tissue. Anatomical structures include esophagus 15, lower esophageal sphincter 14, stomach 8, cardiac notch of stomach 16, gastric fundus 9, greater curvature of stomach 10, pyloric antrum 11, lesser curvature of stomach 17, pylorus 12, and duodenum 13.

Implantable pulse generator 1 is shown with modulator 2 and modulator 3 in contact with the corresponding portion of stomach 8 in the respective figures, detailed below. Implantable pulse generator further comprises attachment fixture 4 and attachment fixture 5. Additional or fewer attachment fixtures may be included without departing from the present invention. Attachment means 6 and attachment means 7 are used to secure attachment fixture 4 and attachment fixture 5, respectively to appropriate portion of stomach 8. Attachment means 6 and attachment means 7 may be comprised from surgical suture material, surgical staples, adhesives, or other means without departing from the present invention.

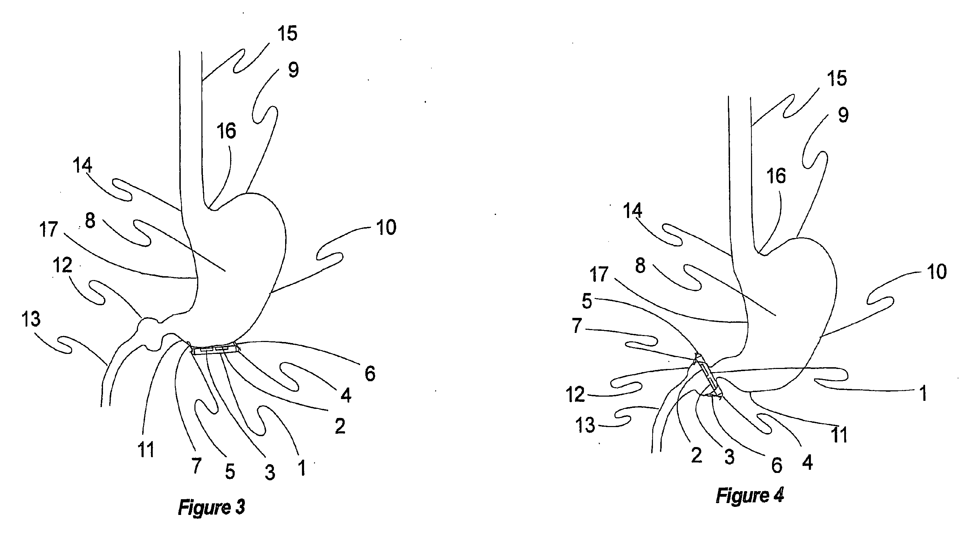

Figures 1, 2, and 3 show implantable pulse generator 1 in several anatomical positions. In Figure 1, implantable pulse generator 1 is shown positioned along the superior region of the greater curvature of stomach 10, with modulator 2 and modulator 3 in contact with the tissues comprising the greater curvature of stomach 10. In Figure 2, implantable pulse generator 1 is shown positioned along the inferior region of the greater curvature of stomach 10, with modulator 2 and modulator 3 in contact with the tissues comprising the greater curvature of stomach 10. In Figure 3, implantable pulse generator 1 is shown positioned along the pyloric antrum 11, with modulator 2 and modulator 3 in contact with the tissues comprising the pyloric antrum 11.

Modulator 2 and modulator 3 are used to stimulate at least one of gastric longitudinal muscle layer, gastric circular muscle layer, gastric nervous tissue, or other tissue. Modulator 2 and modulator 3 may be fabricated from nonpenetrating material or from penetrating material, including needle tips, arrays of needle tips, wires, conductive sutures, other conductive material, or other material, without departing from the present invention.

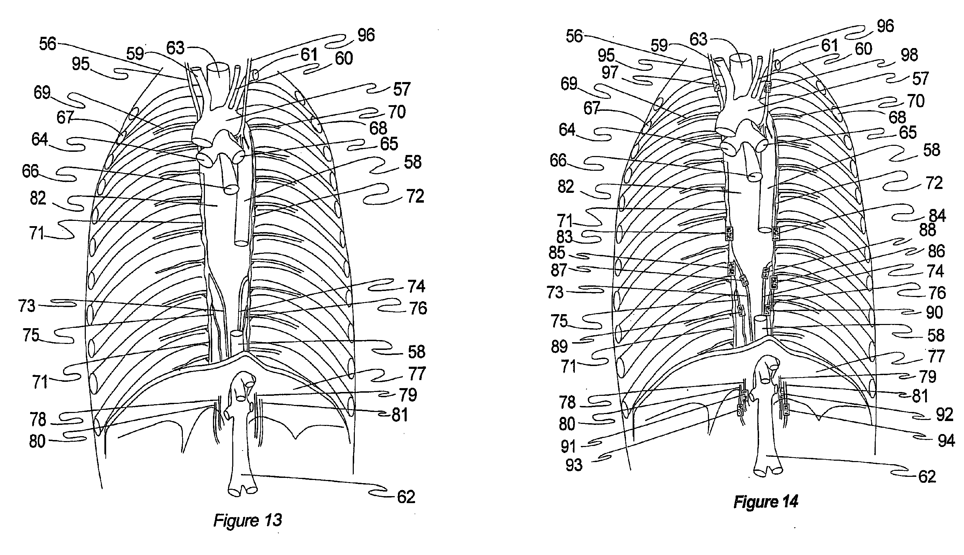

A1b. Sympathetic Afferents - Sympathetic Trunk. The present invention teaches apparatus and method for stimulation of sympathetic afferent fibers using stimulation in the region of the sympathetic trunk. As shown in Figures 14, 15, and 16, sympathetic trunk neuromodulatory interface 83 and 85, positioned on right sympathetic trunk 71, and sympathetic trunk neuromodulatory interface 85, 86 positioned on left sympathetic trunk 72, are used to provide stimulation for afferent as well as for efferent sympathetic nervous system modulation. Modulation of efferent sympathetic nervous system is discussed below, and this is used for metabolic modulation.

A1c. Sympathetic Afferents - Other. The present invention teaches apparatus and method for stimulation of sympathetic afferent fibers using stimulation of nerves arising from the sympathetic trunk. As shown in Figures 14,15, and 16, thoracic splanchnic neuromodulatory interface 87, 89, 88, and 90, positioned on right greater splanchnic nerve 73, right lesser splanchnic nerve 75, left greater splanchnic nerve 74, left lesser splanchnic nerve 76, respectively, and are used to provide stimulation for afferent as well as for efferent sympathetic nervous system modulation. Modulation of efferent sympathetic nervous system is discussed below, and this is used for metabolic modulation.

A2. Gastric Musculature Stimulation. A further object of the present invention is the stimulation of the gastric musculature. This may be performed using either or both of dosed loop and open loop control. In the present embodiment, a combination of open and dosed loop control is employed. The open loop control provides a baseline level of gastric stimulation. This stimulation maintains tone of the gastric musculature. This increases the wall tension the stomach and plays a role in the perception of satiety in the implanted patient. Additionally, stimulation of the gastric musculature causes contraction of the structures, thereby reducing the volume of the stomach. This gastric muscle contraction, and the consequent reduction of stomach volume effectively restricts the amount of food that may be ingested. Surgical techniques have been developed and are known to those practicing in the field of surgical treatment of obesity. Several of these procedures are of the restrictive type, but because of their surgical nature they are fixed in magnitude and difficult if not impossible to reverse. The present invention teaches a technique which employs neural modulation and gastric muscle stimulation which by its nature is the variable and reversible. This offers the advantages postoperative adjustment of magnitude, fine tuning for the individual patient, varying of magnitude to suit the patient's changing needs and changing anatomy over time, and the potential for reversal or termination of treatment. Furthermore, since the gastric wall tension is generated in a physiological manner by the muscle itself, it does not have the substantial risk of gastric wall necrosis and rupture inherent in externally applied pressure, as is the case with gastric banding.

Figures 1, 2, and 3 depict placements of the implantable pulse generator 1 that may be used to stimulate gastric muscle tissue. Stimulation of both longitudinal and circular muscle layers is included in the present invention. Stimulation of gastric circular muscle layer causes circumferential contraction of the stomach, and stimulation of gastric longitudinal muscle layer causes longitudinal contraction of the stomach.

This muscle stimulation and contraction accomplishes several objectives: (1) functional reduction in stomach volume, (2) increase in stomach wall tension, (3) reduction in rate of food bolus flow. All of these effects are performed to induce the sensation of satiety.

A3. Gastric Pylorus Stimulation. Figure 4 depicts implantable pulse generator 1 positioned to perform stimulation of the gastric pylorus 12 to induce satiety by restricting outflow of food bolus material from the stomach 8 into the duodenum 13. Stimulation of the pylorus 12 may be continuous, intermittent, or triggered manually or by sensed event or physiological condition. Figure 4 depicts implantable pulse generator 1 positioned adjacent to the gastric pylorus 12; this position provides secure modulator positioning while eliminating the risk of modulator and wire breakage inherent in other designs in which implantable pulse generator 1 is positioned remote from the gastric pylorus 12.

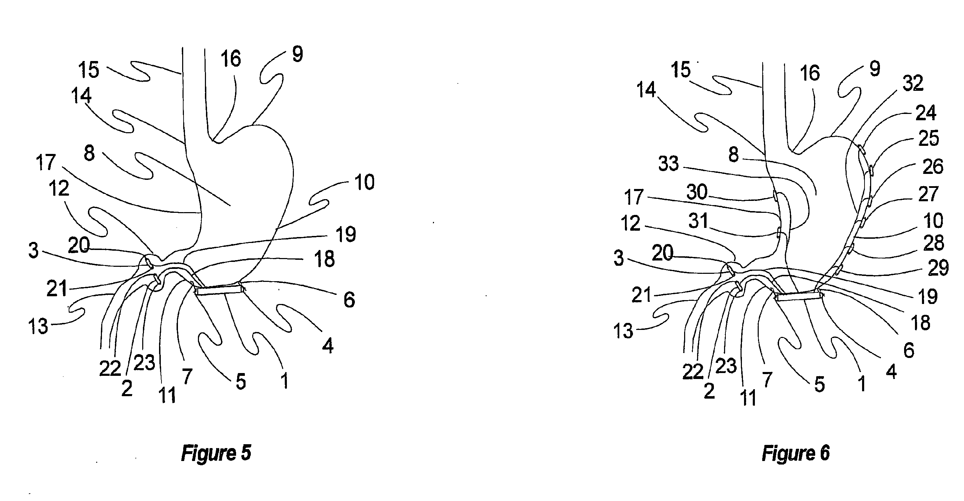

Figure 5 depicts implantable pulse generator 1 positioned to perform stimulation of the gastric pylorus 12 to induce satiety by restricting outflow of food bolus material from the stomach 8 into the duodenum 13. Stimulation of the pylorus 12 may be continuous, intermittent, or triggered manually or by sensed event or physiological condition. Figure 5 depicts implantable pulse generator 1 attached to stomach 8, specifically by the pyloric antrum 11; this position facilitates the use of a larger implantable pulse generator 1. The risk of modulator and wire breakage is minimized by the use of appropriate strain relief and stranded wire designs.

A4. Parasympathetic Stimulation. The parasympathetic nervous system is complementary to the sympathetic nervous system and plays a substantial role in controlling digestion and cardiac activity. Several routes are described in the present invention to modulate activity of the parasympathetic nervous system.

A4a. Parasympathetic Stimulation - Vagus Nerve. Others have advocated the use of vagus nerve stimulation for the treatment of a number of disorders including obesity. Zabara and others have described systems in which the vagus nerve in the region of the neck is stimulated. This is plagued with a host of problems, including life-threatening cardiac complications as well as difficulties with speech and discomfort during stimulation. The present invention is a substantial advance over that discussed by Zabara et al, in which unrestricted fiber activation using epineural stimulation is described. That technique results in indiscriminate stimulation of efferent and afferent fibers. With vagus nerve stimulation, efferent fiber activation generates many undesirable side effects, including gastric and duodenal ulcers, cardiac disturbances, and others.

In the present invention, as depicted in Figure 14, vagus neuromodulatory interface 97 and 98 are implanted adjacent to and in communication with right vagus nerve 95 and left vagus nerve 96. The neuromodulatory interface 97 and 98 overcomes these limitations that have persisted for over a decade with indiscriminate vagus nerve stimulation, by selectively stimulating afferent fibers of the at least one of the vagus nerve, the sympathetic nerves, and other nerves. The present invention includes the selective stimulation of afferent fibers using a technique in which electrical stimulation, is used to block anterograde propagation of action potentials along the efferent fibers. The present invention includes the selective stimulation of afferent fibers using a technique in which stimulation is performed proximal to a nerve transection and in which the viability of the afferent fibers is maintained. One such implementation involves use of at least one of neuromodulatory interface 34 which is of the form shown in at least one of Longitudinal Electrode Neuromodulatory Interface 118, Longitudinal Electrode Regeneration Port Neuromodulatory Interface 119, Regeneration Tube Neuromodulatory Interface 120, neuromodulatory interface array catheter 284 or other design which may become apparent to one skilled in the art, including designs in which a subset of the neuronal polulaiton is modulated.

A.4.a.i. Innovative Stimulation Anatomy. Figure 6 depicts multimodal treatment for the generation of satiety, using sympathetic stimulation, gastric muscle stimulation, gastric pylorus stimulation, and vagus nerve stimulation. This is described in more detail below. Modulators 30 and 31 are positioned in the general region of the lesser curvature of stomach 17. Stimulation in this region results in activation of vagus nerve afferent fibers. Stimulation of other regions may be performed without departing from the present invention. In this manner, selective afferent vagus nerve stimulation may be achieved, without the detrimental effects inherent in efferent vagus nerve stimulation, including cardiac rhythm disruption and induction of gastric ulcers.

A.4.a.ii. Innovative Stimulation Device. The present invention further includes devices designed specifically for the stimulation of afferent fibers.

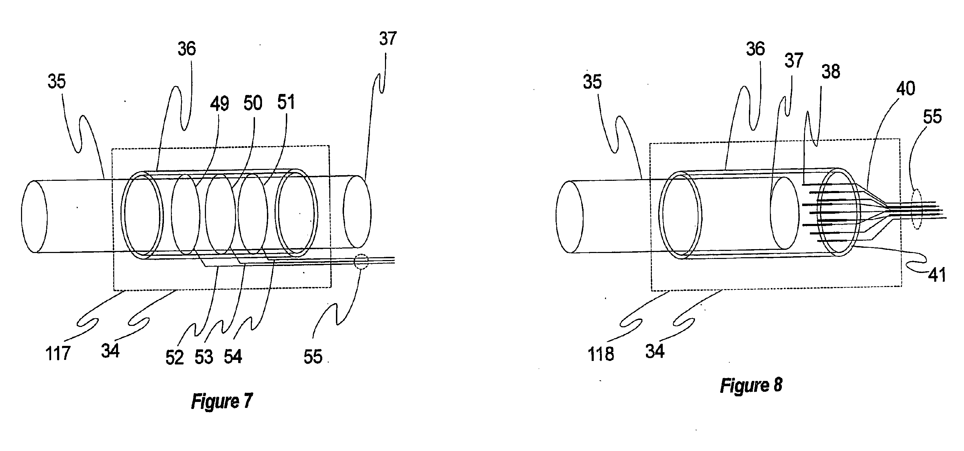

Figure 7 depicts epineural cuff electrode neuromodulatory interface 117, one of several designs for neuromodulatory interface 34 included in the present invention. Nerve 35 is shown inserted through nerve cuff 36. For selective afferent stimulation, the nerve 35 is transected distal to the epineural cuff electrode neuromodulatory interface 117. This case is depicted here, in which transected nerve end 37 is seen distal to epineural cuff electrode neuromodulatory interface 117. Epineural electrode 49, 50, and 51 are mounted along the inner surface of nerve cuff 36 and in contact or dose proximity to nerve 35. Epineural electrode connecting wire 52, 53, 54 are electrically connected on one end to epineural electrode 49, 50, and 51, respectively, and merge together on the other end to form connecting cable 55.

Figure 8 depicts longitudinal electrode neuromodulatory interface 118, one of several designs for neuromodulatory interface 34 included in the present invention. Nerve 35 is shown inserted into nerve cuff 36. For selective afferent stimulation, the nerve 35 is transected prior to surgical insertion into nerve cuff 36. Longitudinal electrode array 38 is mounted within nerve cuff 36 and in contact or dose proximity to nerve 35. Connecting wire array 40 provides electrical connection from each element of longitudinal electrode array 38 to connecting cable 55. Nerve cuff end plate 41 is attached to the distal end of nerve cuff 36. Nerve 35 may be advanced sufficiently far into longitudinal electrode array 38 such that elements of longitudinal electrode array 38 penetrate into nerve 35. Alternatively, nerve 35 may be placed with a gap between transected nerve end 37 and longitudinal electrode array 38 such that neural regeneration occurs from transected nerve end 37 toward and in dose proximity to elements of longitudinal electrode array 38.

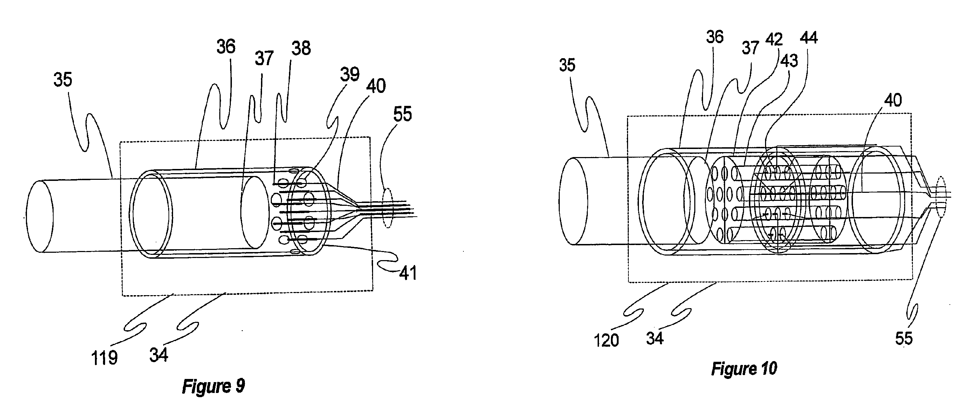

Figure 9 depicts longitudinal electrode regeneration port neuromodulatory interface 119, an improved design for neuromodulatory interface 34 included in the present invention. Nerve 35 is shown inserted into nerve cuff 36. For selective afferent stimulation, the nerve 35 is transected prior to surgical insertion into nerve cuff 36. Longitudinal electrode array 38 is mounted within nerve cuff 36 and in contact or dose proximity to nerve 35. Connecting wire array 40. provides electrical connection from each element of longitudinal electrode array 38 to connecting cable 55. Nerve cuff end plate 41 is attached to the distal end of nerve cuff 36. Nerve 35 may be advanced sufficiently far into longitudinal electrode array 38 such that elements of longitudinal electrode array 38 penetrate into nerve 35. Alternatively, nerve 35 may be placed with a gap between transected nerve end 37 and longitudinal electrode array 38 such that neural regeneration occurs from transected nerve end 37 toward and in dose proximity to elements of longitudinal electrode array 38. At least one of nerve cuff 36 and nerve cuff end plate 41 are perforated with one or a multiplicity of regeneration port 39 to facilitate and enhance regeneration of nerve fibers from transected nerve end 37.

Figure 10 depicts regeneration tube neuromodulatory interface 120, an advanced design for neuromodulatory interface 34 included in the present invention. Nerve 35 is shown inserted into nerve cuff 36. For selective afferent stimulation, the nerve 35 is transected prior to surgical insertion into nerve cuff 36. Regeneration electrode array 44 is mounted within regeneration tube array 42, which is contained within nerve cuff 36. Each regeneration tube 43 contains at least one element of regeneration electrode array 44. Each element of regeneration electrode array 44 is electrically connected by at least one element of connecting wire array 40 to connecting cable 55. Nerve 35 may be surgically inserted into nerve cuff 36 sufficiently far to be adjacent to regeneration tube array 42 or may be placed with a gap between transected nerve end 37 and regeneration tube array 42. Neural regeneration occurs from transected nerve end 37 toward and through regeneration tube 43 elements regeneration tube array 42.

The present invention further includes stimulation of other tissues that influence vagus nerve activity. These include tissues of the esophagus, stomach, small and large intestine, pancreas, liver, gallbladder, kidney, mesentery, appendix, bladder, uterus, and other intraabdominal tissues. Stimulation of one or a multiplicity of these tissues modulates activity of the vagus nerve afferent fibers without significantly altering activity of efferent fibers. This method and the associated apparatus facilitates the stimulation of vagus nerve afferent fibers without activating vagus nerve efferent fibers, thereby overcoming the ulcerogenic and cardiac side effects of nonselective vagus nerve stimulation. This represents a major advance in vagus nerve modulation and overcomes the potentially life-threatening complications of nonselective stimulation of the vagus nerve.

A4b. Parasympathetic Stimulation - Other. The present invention teaches stimulation of the cervical nerves or their roots or branches for modulation of the parasympathetic nervous system. Additionally, the present invention teaches stimulation of the sacral nerves or their roots or branches for modulation of the parasympathetic nervous system.

A5. Multichannel Satiety Modulation. Figure 6 depicts apparatus and method for performing multichannel modulation of satiety. Implantable pulse generator 1 is attached to stomach 8, via attachment means 6 and 7 connected from stomach 8 to attachment fixture 4 and 5, respectively. Implantable pulse generator 1 is electrically connected via modulator cable 32 to modulators 24, 25, 26, 27, 28, and 29, which are affixed to the stomach 8 preferably along the region of the greater curvature of stomach 10. Implantable pulse generator 1 is additionally electrically connected via modulator cable 33 to modulators 30 and 31, which are affixed to the stomach 8 preferably along the region of the lesser curvature of stomach 17. Implantable pulse generator 1 is furthermore electrically connected via modulator cable 18 and 19 to modulators 2 and 3, respectively, which are affixed to the gastric pylorus 12. Modulator 2 is affixed to gastric pylorus via modulator attachment fixture 22 and 23, and modulator 3 is affixed to gastric pylorus via modulator attachment fixture 20 and 21.

Using the apparatus depicted in Figure 6, satiety modulation is achieved through multiple modalities. A multiplicity of modulators, including modulator 30 and 31 facilitate stimulation of vagus and sympathetic afferent fibers directly, as well as through stimulation of tissues, including gastric muscle, that in turn influence activity of the sympathetic and vagus afferent fibers. A multiplicity of modulators, including modulator 24, 25, 26, 27, 28, and 29 facilitate stimulation of sympathetic afferent fibers directly, as well as through stimulation of tissues, including gastric muscle, that in turn influence activity of the sympathetic fibers. Any of these modulators may be used to modulate vagus nerve activity; however, one advancement taught in the present invention is the selective stimulation of sympathetic nerve fiber activation, and this is facilitated by modulators 24, 25, 26, 27, 28, and 29, by virtue of their design for and anatomical placement in regions of the stomach 8 that are not innervated by the vagus nerve or its branches.

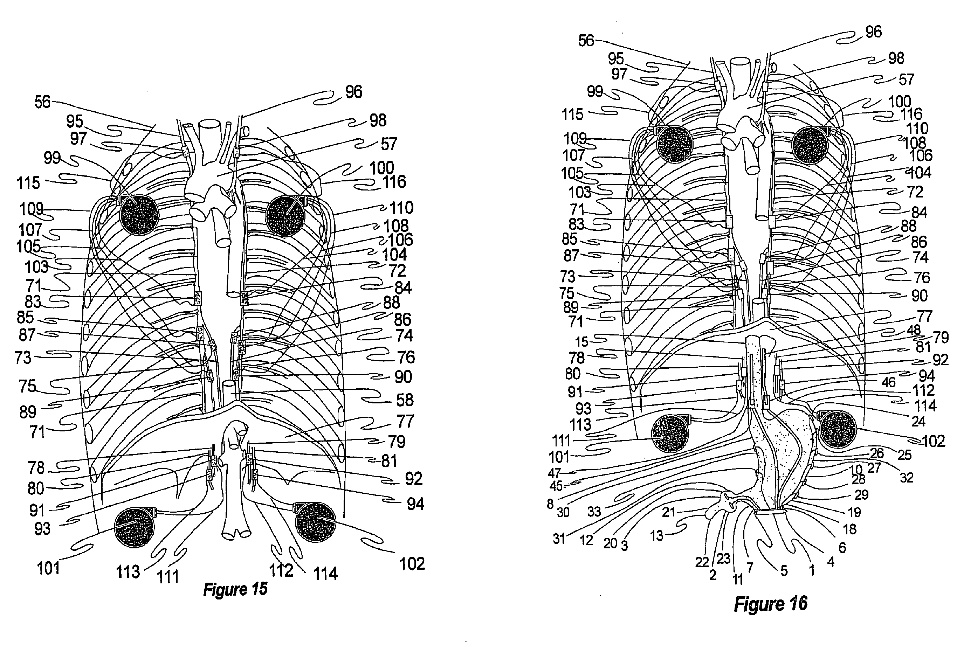

In addition to the apparatus and methods depicted in figure 6 for satiety modulation, the present invention further includes satiety modulation performed with the apparatus depicted in Figure 16, and described previously, using stimulation of right sympathetic trunk 71, left sympathetic trunk 72, right greater splanchnic nerve 73, left greater splanchnic nerve 74, right lesser splanchnic nerve 75, left lesser splanchnic nerve 76 or other branch or the sympathetic nervous system.

B. Metabolic ModulationB.1. Sympathetic Efferent Stimulation. One objective of the modulator configuration employed in the present invention is the selected stimulation of sympathetic efferent nerve fibers. The present invention includes a multiplicity of potential modulator configurations and combinations of thereof. The present embodiment includes modulators placed at a combination of sites to interface with the sympathetic efferent fibers. These sites include the musculature of the stomach, the distal sympathetic branches penetrating into the stomach, postganglionic axons and cell bodies, preganglionic axons and cell bodies, the sympathetic chain and portions thereof, the intermediolateral nucleus, the locus ceruleus, the hypothalamus, and other structures comprising or influencing activity of the sympathetic nervous system.

Stimulation of the sympathetic efferents is performed to elevate the metabolic rate and lipolysis in the adipose tissue, thereby enhancing breakdown of fat and weight loss in the patient.

B.1.a. Sympathetic Efferent Stimulation Sympathetic Trunk. Figures 14, 15, and 16 depict apparatus for stimulation of the sympathetic nervous system. Figure 14 depicts a subset of anatomical locations for placement of neuromodulatory interfaces for modulation of the sympathetic nervous system. Figure 15 depicts the same apparatus with the further addition of a set of implantable pulse generator 1 and connecting cables. Figure 16 depicts the apparatus shown in Figure 15 with the further addition of gastric modulation apparatus also depicted in Figure 6.

Figure 13 reveals the normal anatomy of the thoracic region. Trachea 63 is seen posterior to aortic arch 57. Brachiocephalic artery 59, left common carotid artery 60 arise from aortic arch 57, and left subclavian artery 61 arises from the left common carotid artery 60. Right mainstem bronchus 64 and left mainstem bronchus 65 arise from trachea 63. Thoracic descending aorta 58 extends from aortic arch 57 and is continuous with abdominal aorta 62. Right vagus nerve 95 and left vagus nerve 96 are shown. Intercostal nerve 69 and 70 are shown between respective pairs of ribs, of which rib 67 and rib 68 are labeled.

Right sympathetic trunk 71 and left sympathetic trunk are lateral to mediastinum 82. Right greater splanchnic nerve 73 and right lesser splanchnic nerve 75 arise from right sympathetic trunk 71. Left greater splanchnic nerve 74 and left lesser splanchnic nerve 76 arise from left sympathetic trunk 72. Right subdiaphragmatic greater splanchnic nerve 78, left subdiaphragmatic greater splanchnic nerve 79, right subdiaphragmatic lesser splanchnic nerve 80, and left subdiaphragmatic lesser splanchnic nerve 81 are extensions below the diaphragm 77 of the right greater splanchnic nerve 73, left greater splanchnic nerve 74, right lesser splanchnic nerve 75, and left lesser splanchnic nerve 76, respectively.

B.1.b. Sympathetic Efferent Stimulation - Splanchnics. Figure 14 depicts multichannel sympathetic modulation implanted with relevant anatomical structures. Sympathetic trunk neuromodulatory interface 83 and 85 are implanted adjacent to and in communication with right sympathetic trunk 71. Sympathetic trunk neuromodulatory interface 84 and 86 are implanted adjacent to and in communication with left sympathetic trunk 72. Sympathetic trunk neuromodulatory interface 83,84,85, and 86 are implanted superior to their respective sympathetic trunk levels atwhich the right greater splanchnic nerve 73, left greater splanchnic nerve 74, right lesser splanchnic nerve 75, and left lesser splanchnic nerve 76, arise, respectively.

Thoracic splanchnic nerve interface 87, 88, 89, 90 are implanted adjacent to and in communication with the right greater splanchnic nerve 73, left greater splanchnic nerve 74, right lesser splanchnic nerve 75, and left lesser splanchnic nerve 76, arise, respectively. Abdominal splanchnic nerve interface 91, 92, 93, and 94 are implanted adjacent to and in communication with the right subdiaphragmatic greater splanchnic nerve 78, left subdiaphragmatic greater splanchnic nerve 79, right subdiaphragmatic lesser splanchnic nerve 80, and left subdiaphragmatic lesser splanchnic nerve 81, respectively.

Stimulation of at least one of right sympathetic trunk 71, left sympathetic trunk 72, right greater splanchnic nerve 73, left greater splanchnic nerve 74, right lesser splanchnic nerve 75, and left lesser splanchnic nerve 76, right subdiaphragmatic greater splanchnic nerve 78, left subdiaphragmatic greater splanchnic nerve 79, right subdiaphragmatic lesser splanchnic nerve 80, and left subdiaphragmatic lesser splanchnic nerve 81 enhances metabolism of adipose tissue. Stimulation of these structures may be performed using at least one of electrical energy, electrical fields, optical energy, mechanical energy, magnetic energy, chemical compounds, pharmacological compounds, thermal energy, vibratory energy, or other means for modulating neural activity.

Figure 15 depicts the implanted neuromodulatory interfaces as in Figure 14, with the addition of the implanted pulse generators. Implantable pulse generator 99 is connected via connecting cable 103, 105, 107, 109, 115, to sympathetic trunk neuromodulatory interface 83.and 85, and thoracic splanchnic neuromodulatory interface 87 and 89, and vagus neuromodulatory interface 97, respectively. Implantable pulse generator 100 is connected via connecting cable 104, 106, 108, 110, 116, to sympathetic trunk neuromodulatory interface 83.and 85, and thoracic splanchnic neuromodulatory interface 88 and 90, and vagus neuromodulatory interface 98, respectively. Implantable pulse generator 101 is connected via connecting cable 111 and 113 to abdominal splanchnic neuromodulatory interface 91 and 93, respectively. Implantable pulse generator 102 is connected via connecting cable 112 and 114 to abdominal splanchnic neuromodulatory interface 92 and 94, respectively.

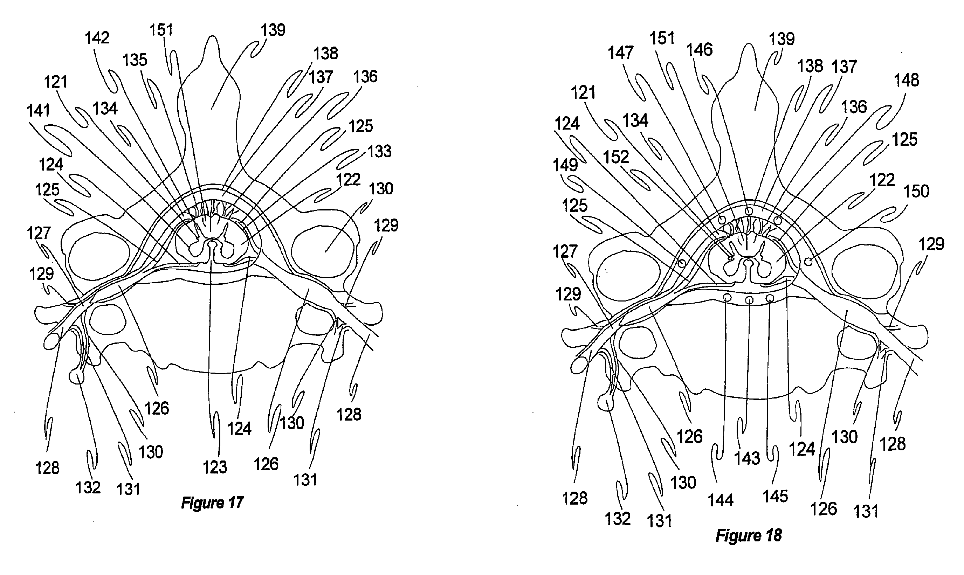

B.1.c. Sympathetic Efferent Stimulation - Spinal Cord. Figures 17 and 18 depicts the normal cross sectional anatomy of the spinal cord 151 and anatomy with implanted neuromodulatory interfaces, respectively.

Figure 17 depicts the normal anatomical structures of the spinal cord 151, including several of its component structures such as the intermediolateral nucleus 121, ventral horn of spinal gray matter 141, dorsal horn ofspinal gray matter 142, spinal cord white matter 122, anterior median fissure 123. Other structures adjacent to or surrounding spinal cord 151 include ventral spinal root 124, dorsal spinal root 125, spinal ganglion 126, spinal nerve 127, spinal nerve anterior ramus 128, spinal nerve posterior ramus 129, gray ramus communicantes 130, white ramus communicantes 131, sympathetic trunk 132, pia mater 133, subarachnoid space 134, arachnoid 135, meningeal layer of dura mater 136, epidural space 137, periosteal layer of dura mater 138, and vertebral spinous process 139, and vertebral facet 140.

Figure 17 depicts the normal anatomy of the spinal cord seen in transverse section. Spinal cord and related neural structures structures include intermediolateral nucleus 121, spinal cord white matter 122, anterior median fissure 123, ventral spinal root 124, dorsal spinal root 125, spinal ganglion 126, spinal nerve 127, spinal nerve anterior ramus 128, spinal nerve posterior ramus 129, grey ramus communicantes 130, white ramus communicantes 131, sympathetic trunk 132, pia mater 133, subarachnoid space 134, arachnoid 135, meningeal layer of dura 136, epidural space 137, periostial layer of dura mater 138, vertebral spinous process 139, vertebral facet 140, ventral horn of spinal gray matter 141, and dorsal horn of spinal gray matter 142.

Figure 18 depicts the spinal neuromodulatory interfaces positioned in the vicinity of spinal cord 151. Neuromodulatory interfaces positioned anterior to spinal cord 151 include anterior central spinal neuromodulatory interface 143, anterior right lateral spinal neuromodulatory interface 144, and anterior left lateral spinal neuromodulatory interface 145. Neuromodulatory interfaces positioned posterior to spinal cord 151 include posterior central spinal neuromodulatory interface 146, posterior right lateral spinal neuromodulatory interface 147, and posterior left lateral spinal neuromodulatory interface 148. Neuromodulatory interfaces positioned lateral to spinal cord 151 include right lateral spinal neuromodulatory interface 149 and left lateral spinal neuromodulatory interface 150. Neuromodulatory interfaces positioned within the spinal cord 151 include intermediolateral nucleus neuromodulatory interface 152.

Stimulation, inhibition, or other modulation of the spinal cord 151 is used to modulate fibers of the sympathetic nervous system, including those in the intermediolateral nucleus 121 and efferent and efferent fibers connected to the intermediolateral nucleus 121. Modulation of at least one of portions of the spinal cord 151, intermediolateral nucleus 121, ventral spinal root 124, dorsal spinal root 125, spinal ganglion 126, spinal nerve 127, gray ramus communicantes 130, white ramus communicantes 131 and other structures facilitates modulation of activity of the sympathetic trunk 132. Modulation of activity of the sympathetic trunk 132, in turn, is used to modulate at least one of metabolic activity, satiety, and appetite. This may be achieved using intermediolateral nucleus neuromodulatory interface 152, placed in or adjacent to the intermediolateral nucleus 121. The less invasive design employing neuromodulatory interfaces (144, 145, 146, 147, 148, 149, 150) shown positioned in the in epidural space 137 is taught in the present invention.

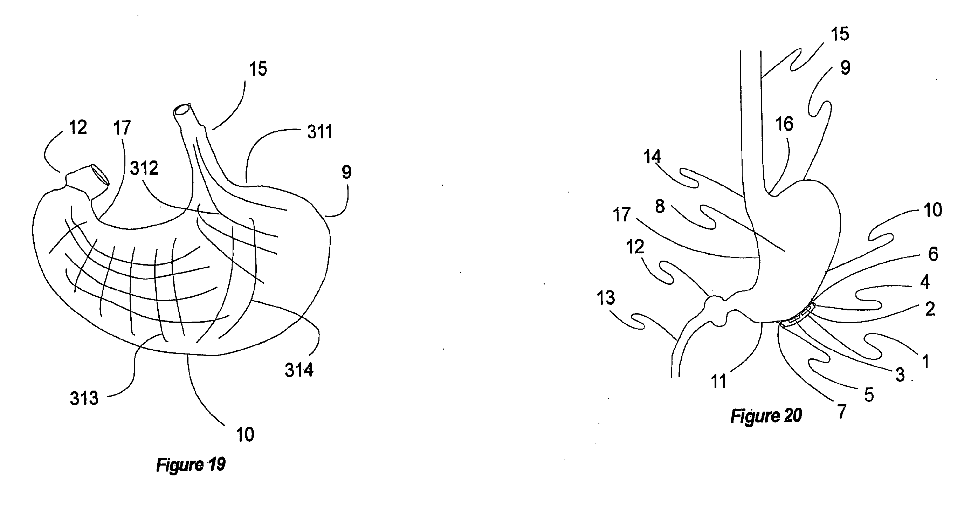

Figure 19 depicts a cut away view of the stomach, revealing the four coats: serous, muscular, aerolar, and mucous. The gastric muscular coat 311 is comprised of 3 layers, the gastric longitudinal fibers 311, gastric circular fibers 312, and gastric oblique fibers 313. Gastric longitudinal fibers 311 are most superficial; they are continuous with the longitudinal fibers of the esophagus 15, radiating in a stellate manner from the cardiac orifice. They are most distinct along the curvatures, especially the lesser, but are very thinly distributed over the surfaces. At the pyloric end, they are more thickly distributed and are continuous with the longitudinal fibers of the small intestine. Gastric circular fibers 313 form a uniform layer over the whole extent of the stomach beneath the gastric longitudinal fibers 311. At the gastric pylorus 12 they are most abundant and are aggregated into a circular ring, which projects into the lumen and forms, with the fold of mucous membrane covering its surface, the pyloric valve. They are continuous with the circular layers of the esophagus 15. The gastric oblique fibers 314 are beneath the gastric circular fibers 313. Stimulation of afferent neural fibers innervating stretch receptors in these muscle layers is taught in the parent case. This figure merely depicts anatomical detail.

B.1.d. Sympathetic Efferent Stimulation - Other. The present invention further includes modulation of all sympathetic efferent nerves, nerve fibers, and neural structures. These sympathetic efferent neural structures include but are not limited to distal sympathetic nerve branches, mesenteric nerves, sympathetic efferent fibers at all spinal levels, rami communicantes at all spinal levels, paravertebral nuclei, prevertebral nuclei, and other sympathetic structures.

B.2. Noninvasive Stimulation. The present invention teaches a device for metabolic control using tactile stimulation. Tactile stimulation of afferent neurons causes alterations in activity of sympathetic neurons which influence metabolic activity of adipose tissue. The present invention teaches tactile stimulation of skin, dermal and epidermal sensory structures, subcutaneous tissues and structures, and deeper tissues to modulate activity of afferent neurons.

This device for metabolic control employs vibratory actuators. Alternatively, electrical stimulation, mechanical stimulation, optical stimulation, acoustic stimulation, pressure stimulation, and other forms of energy that modulate afferent neural activity, are used.

C. Multimodal Metabolic Modulation. To maximize efficacy while tailoring treatment to minimize side effects, the preferred embodiment includes a multiplicity of treatment modalities, including afferent, efferent, and neuromuscular modulation.

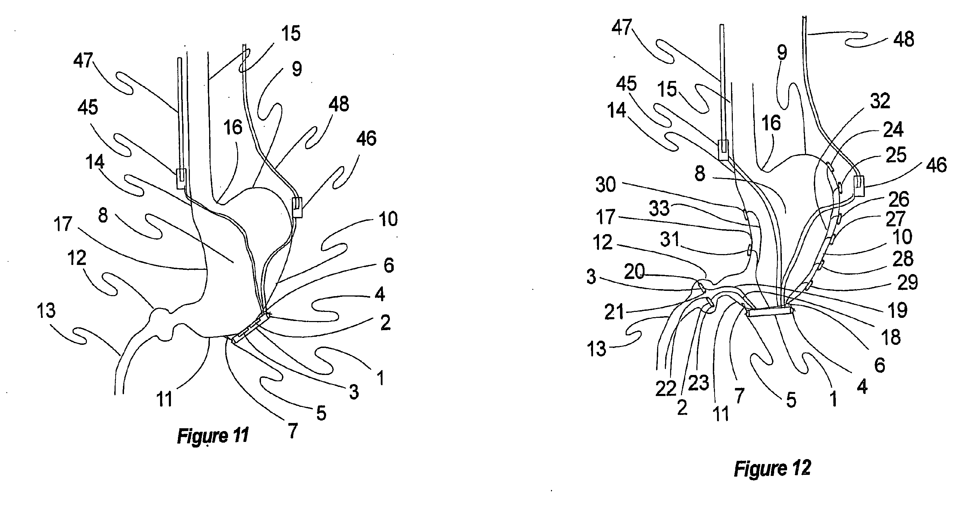

Afferent signals are generated to simulate satiety. This is accomplished through neural, neuromuscular, and hydrostatic mechanisms. Electrical stimulation of the vagus via vagus nerve interface 45 afferents provides one such channel to transmit information to the central nervous system for the purpose of eliciting satiety. Electrical stimulation of the sympathetic afferents via sympathetic nerve interface 46 provides another such channel to transmit information to the central nervous system for the purpose of eliciting satiety. Electrical stimulation of gastric circular muscle layer in Figure 11, multimodal stimulation is depicted, including stimulation of gastric musculature using modulators 2 and 3, as well as stimulation of afferent fibers of the proximal stump of vagus nerve 47 using vagus nerve modulator 45 and stimulation of afferent fibers of sympathetic nerve branch 48.

In Figure 12, expanded multimodal stimulation is depicted, including those modalities shown in Figure 11, including stimulation of gastric musculature using modulators 2 and 3, as well as stimulation of afferent fibers of the proximal stump of vagus nerve 47 using vagus nerve modulator 45 and stimulation of afferent fibers of sympathetic nerve branch 48., in addition to those modalities shown in Figure 6, explained in detail above, including modulation of gastric muscular fibers, sympathetic afferent fibers innervating gastric tissues, and vagus afferent fibers innervating gastric tissues.

In Figure 16, further expanded multimodal modulation is depicted, including modalities encompassed and described above and depicted in Figure 15 and Figure 12. This includes modulation of gastric muscle fibers, fibers of the sympathetic nerve branch 48 and vagus nerve 47 that innervate gastric tissues, and a multiplicity of structures in the sympathetic nervous system and vagus nerve 47.

E. System / Pulse Generator Design. Neuromodulatory interfaces that use electrical energy to modulate neural activity may deliver a broad spectrum of electrical waveforms. One preferred set of neural stimulation parameter sets includes pulse frequencies ranging from 0.1 Hertz to 1000 Hertz, pulse widths from 1 microsecond to 500 milliseconds. Pulses are charge balanced to insure no net direct current charge delivery. The preferred waveform is bipolar pulse pair, with an interpulse interval of 1 microsecond to 1000 milliseconds. Current regulated stimulation is preferred and includes pulse current amplitudes ranging from 1 microamp to 1000 milliamps. Alternatively, voltage regulation may be used, and pulse voltage amplitudes ranging from 1 microamp to 1000 milliamps. These parameters are provided as exemplary of some of the ranges included in the present invention; variations from these parameter sets are included in the present invention.

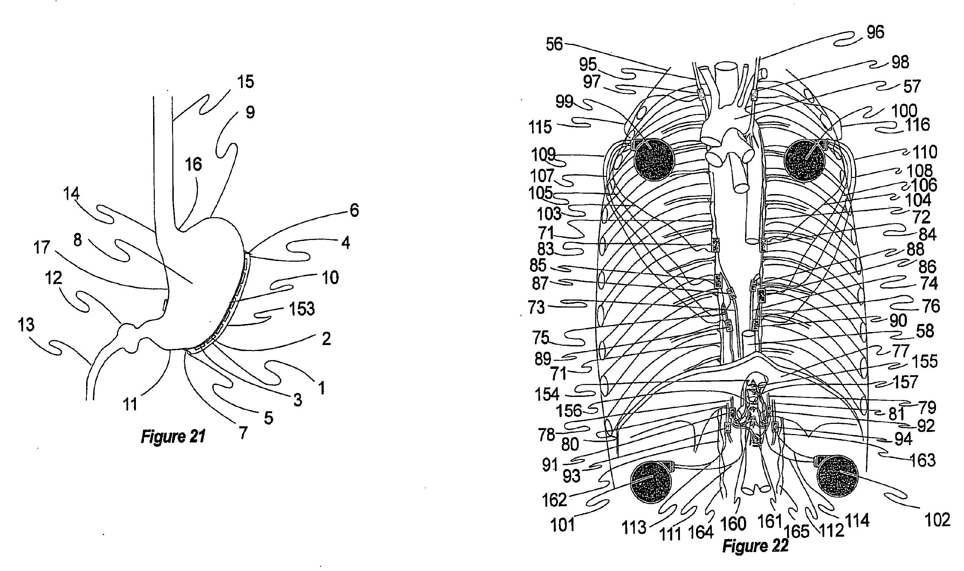

Figure 22 shows the same invention taught in the parent case. In this figure, the distal portion of the sympathetic nervous system is shown in more detail. In the parent case, modulation of the sympathetic nervous system was taught for the treatment of disease. When a portion of the nervous system is modulated, connected neural structures are likewise modulated. Neural structures proximal and distal to the location of the modulator are modulated by the action of the modulator. A multiplicity of locations for neuromodulators are presented in the parent case, and other locations may be selected without departing from the parent case invention. The addition of more detail of the nervous system renders obvious to the reader of the parent application additional locations for placement of neural modulators.

In Figure 22, additional anatomical structures shown include celiac plexus 154, celiac ganglion 155, superior mesenteric plexus 156, superior mesenteric ganglion 157, renal plexus 158, renal ganglion 159, inferior mesenteric plexus 160, iliac plexus 161, right lumbar sympathetic ganglia 162, left lumbar sympathetic ganglia 163, right sacral sympathetic ganglia 164, and left sacral sympathetic ganglia 165.

It is obvious to the reader that modulation of the right greater splanchnic nerve 73, the performance of which is exemplified by Abdominal Splanchnic Neuromodulatory Interface 91, will in turn effect modulation of connected structures, including proximal and distal portions of Right Subdiaphragmatic Greater Splanchnic Nerve 78. Proximal or retrograde conduction of neural signals will effect modulation of Right Greater Splanchnic Nerve 73 and more proximal structures. Distal or anterograde conduction of neural signals will effect modulation of distal structures including but not limited to celiac plexus 154, celiac ganglion 155, superior mesenteric plexus 156, superior mesenteric ganglion 157, renal plexus 158, renal ganglion 159, inferior mesenteric plexus 160, iliac plexus 161, and other structures connected by neural pathways.

It is obvious to the reader that modulation of the left greater splanchnic nerve 74, the performance of which is exemplified by Abdominal Splanchnic Neuromodulatory Interface 92, will in turn effect modulation of connected structures, including proximal and distal portions of Left Subdiaphragmatic Greater Splanchnic Nerve 79. Proximal or retrograde conduction of neural signals will effect modulation of Left Greater Splanchnic Nerve 74 and more proximal structures. Distal or anterograde conduction of neural signals will effect modulation of distal structures including but not limited to celiac plexus 154, celiac ganglion 155, superior mesenteric plexus 156, superior mesenteric ganglion 157, renal plexus 158, renal ganglion 159, inferior mesenteric plexus 160, iliac plexus 161, and other structures connected by neural pathways.

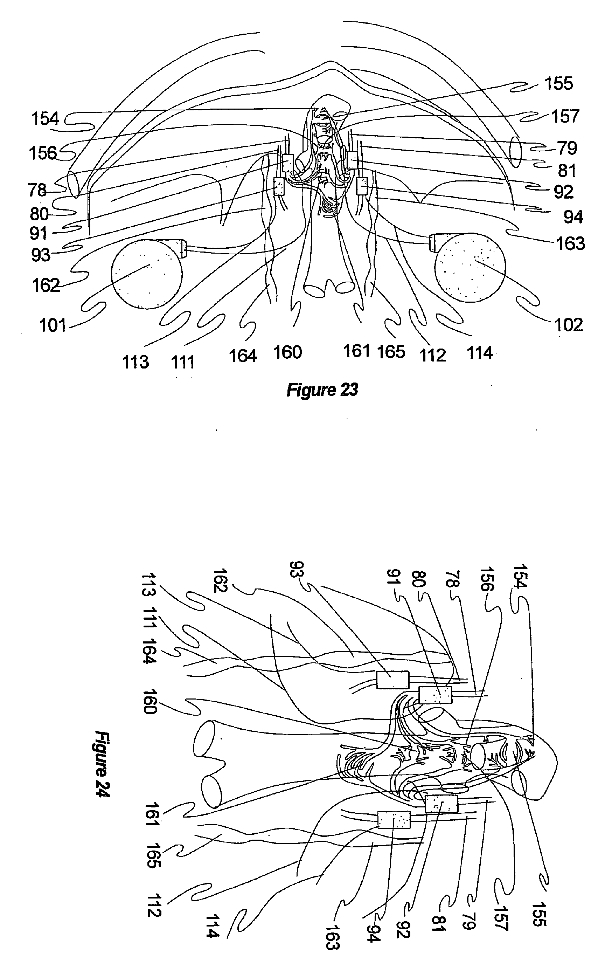

Figure 23 and Figure 24 show Abdominal Splanchnic Neuromodulatory Interface 91, Abdominal Splanchnic Neuromodulatory Interface 92, Abdominal Splanchnic Neuromodulatory Interface 93, Abdominal Splanchnic Neuromodulatory Interface 94 and surrounding anatomical structures, as described above, at larger magnification.

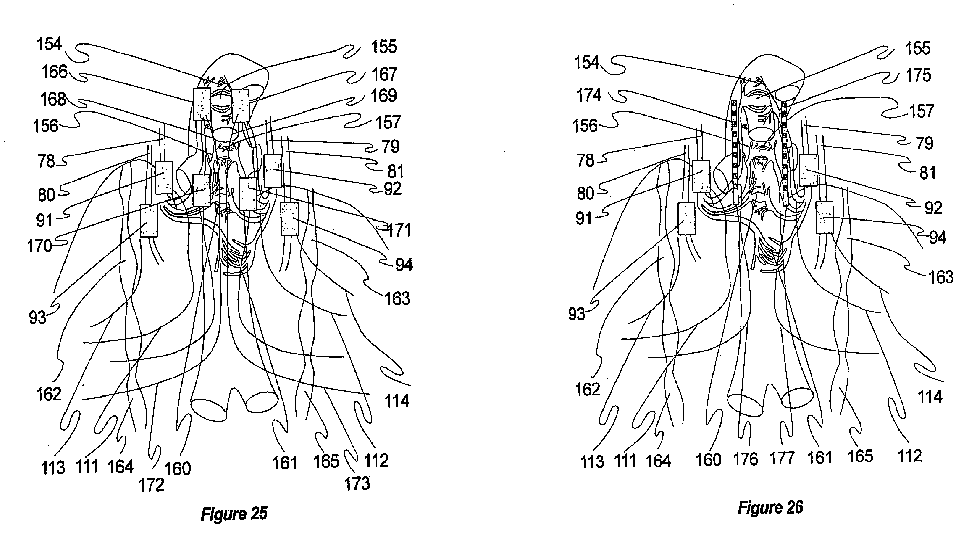

Figure 25 shows Abdominal Splanchnic Neuromodulatory Interface 166, Abdominal Splanchnic Neuromodulatory Interface 167, Abdominal Splanchnic Neuromodulatory Interface 170, and Abdominal Splanchnic Neuromodulatory Interface 171 in proximity to neural structures distal to and in neural communication with each of the right greater splanchnic nerve 73 and left greater splanchnic nerve 73.

Pulse generator 101 generates neuromodulatory signal which is transmitted by connecting cable 168 to abdominal splanchnic neuromodulatory interface 166, which modulates at least one of celiac plexus 154 and celiac ganglion 155. Implantable Pulse generator 102 generates neuromodulatory signal which is transmitted by connecting cable 169 to abdominal splanchnic neuromodulatory interface 167, which modulates at least one of celiac plexus 154 and celiac ganglion 155.

Pulse generator 101 generates neuromodulatory signal which is transmitted by connecting cable 172 to abdominal splanchnic neuromodulatory interface 170, which modulates at least one of superior mesenteric plexus 156, superior mesenteric ganglion 157, renal plexus 158, renal ganglion 159, inferior mesenteric plexus 160, and iliac plexus 161. Pulse generator 102 generates neuromodulatory signal which is transmitted by connecting cable 173 to abdominal splanchnic neuromodulatory interface 171, which modulates at least one of superior mesenteric plexus 156, superior mesenteric ganglion 157, renal plexus 158, renal ganglion 159, inferior mesenteric plexus 160, and iliac plexus 161.

Figure 26 shows neuromodulator array 174 and neuromodulator array 175 in proximity to neural structures distal to and in neural communication with each of the right greater splanchnic nerve 73 and left greater splanchnic nerve 73.

Pulse generator 101 generates neuromodulatory signal which is transmitted by connecting cable 176 to neuromodulator array 174, which modulates at least one of celiac plexus 154, celiac ganglion 155, superior mesenteric plexus 156, superior mesenteric ganglion 157, renal plexus 158, renal ganglion 159, inferior mesenteric plexus 160, and iliac plexus 161.

Pulse generator 102 generates neuromodulatory signal which is transmitted by connecting cable 177 to neuromodulator array 175, which modulates at least one of celiac plexus 154, celiac ganglion 155, superior mesenteric plexus 156, superior mesenteric ganglion 157, renal plexus 158, renal ganglion 159, inferior mesenteric plexus 160, and iliac plexus 161.

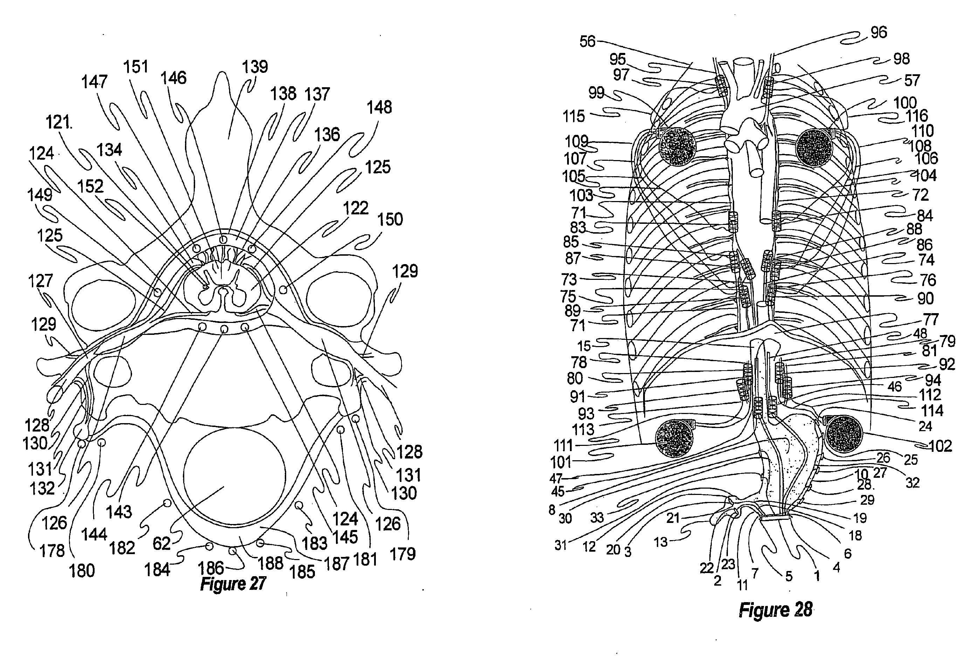

Figure 27 shows a transverse section through the spinal canal, vertebral columns, and adjacent structures in the lumbar region. The components described may be positioned at a higher level, including cervical and thoracic, ora a lover level including sacral and coccygeal, without departing from the present invention. Perispinal neuromodulatory interfaces are described in the description for Figure 18. Abdominal aorta 62 is shown.

Abdominal Splanchnic Neuromodulatory Interface 178 modulate at least one of sympathetic trunk, 132, Right Lumbar Sympathetic Ganglia 162, and Right Sacral Sympathetic Ganglia 164. Abdominal Splanchnic Neuromodulatory Interface 179 modulates at least one of sympathetic trunk, 132, Left Lumbar Sympathetic Ganglia 163, and Left Sacral Sympathetic Ganglia 165

Abdominal Splanchnic Neuromodulatory Interface 180 modulates at least one neural structure in neural connection to sympathetic trunk 132, including but not limited to right greater splanchnic nerve 73, right lesser splanchnic nerve 75, right least splanchnic nerve, or other structure. Abdominal Splanchnic Neuromodulatory Interface 181 modulates at least one neural structure in neural connection to sympathetic trunk 132, including but not limited to left greater splanchnic nerve 74, left lesser splanchnic nerve 76, left least splanchnic nerve, or other structure.

Abdominal Splanchnic Neuromodulatory Interface 182, Abdominal Splanchnic QSNeuromodulatory Interface 183, Abdominal Splanchnic Neuromodulatory Interface 184, Abdominal Splanchnic Neuromodulator Interface 185, and Abdominal Splanchnic Neuromodulatory Interface 186 each modulate abdominal structures including but not limited to celiac plexus 154, celiac ganglion 155, superior mesenteric plexus 156, superior mesenteric ganglion 157, renal plexus 158, renal ganglion 159, inferior mesenteric plexus 160, and iliac plexus 161.

Modulation is performed to modulate metabolic rate, satiety, blood pressure, heart rate, peristalsis, insulin release, CCK release, and other gastrointestinal functions. Modulation using the system and method taught, as well as equivalent modifications and varioations thereof, allows the treatment of disease including obesity, bulimia, anorexia, diabetes, hypoglycemis, hyperglycemia, irritable bowel syndrome, hypertension, hypotension, shock, gastroparesis, and other disorders. Modulation includes at least one of stimulatory and inhibitory effect on neural structures.

Figure 28 shows the same invention taught in the parent case and shown in Figure 16, with detail shown for the nerve cuff electrode implementation for the neuromodulatory interfaces. In this figure, the distal portion of the sympathetic nervous system is shown in more detail. In the parent case, modulation of the sympathetic nervous system was taught for the treatment of disease, and several nerve cuff electrode designs were presented in Figures 7,8,9, and 10 as a subset of many possible implementations of a neuromodulator or neuromodulatory interface. This Figure 28 shows one of many potential arrangements of these components shown in the parent case; numerous other arrangements will be apparent to one skilled in the art upon reading the parent patent specification and figures.

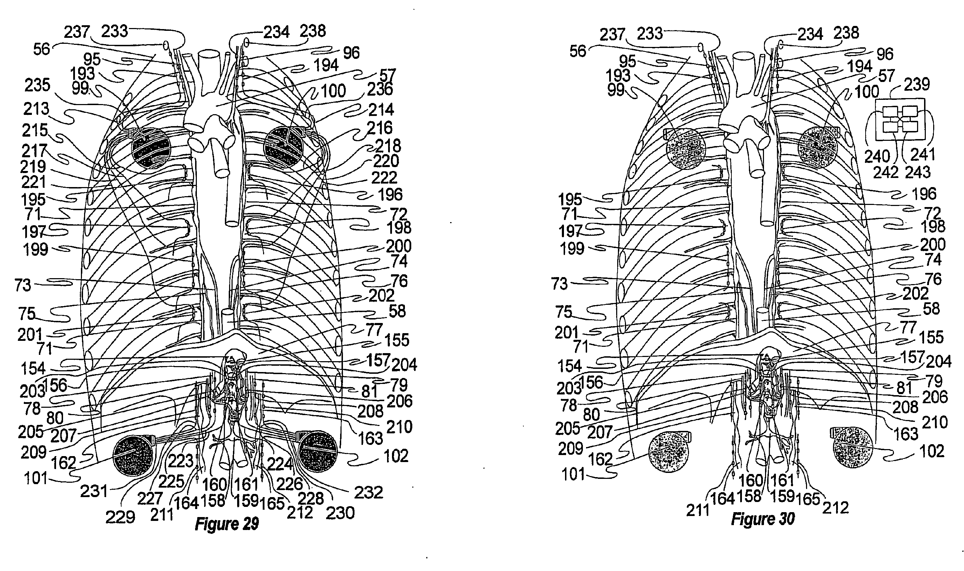

Figure 29 shows the same invention taught in the parent case and shown in Figure 16, with detail shown foran electrode catheter, a linear catheter based electrode implementation for the neuromodulatory interfaces. In this figure, the distal portion of the sympathetic nervous system is shown in more detail. In the parent case, modulation of the sympathetic nervous system was taught for the treatment of disease. This Figure 29 shows another potential arrangement of electrodes that become apparent to one skilled in the art upon reading the parent patent specification and figures.

Implantable pulse generator 99 is connected via connecting cable 213, 215, 217, 219, 221, and 235 to Right Cervical Plexus Neuromodulator Array 193, Right Intercostal Neuromodulator Array 195, Right Intercostal Neuromodulator Array 197, Right Intercostal Neuromodulator Array 199, Right Intercostal Neuromodulator Array 201, and Right Vagal Neuromodulator Array 233, respectively.

Implantable pulse generator 100 is connected via connecting cable 214,216,218,220,222, and 236 to Left Cervical Plexus Neuromodulator Array 194, Left Intercostal Neuromodulator Array 196, Left Intercostal Neuromodulator Array 198, Left Intercostal Neuromodulator Array 200, and Left Intercostal Neuromodulator Array 202, and Left Vagal Neuromodulator Array 234, respectively.

Implantable pulse generator 101 is connected via connecting cable 223, 225, 227, 229, and 231 to Right Abdominal Para Plexus Neuromodulator Array 203, Right Abdominal Greater Splanchnic Neuromodulator Array 205, RightAbdominal Lesser Splanchnic Neuromodulator Array 207, Right Abdominal Sympathetic Trunk Neuromodulator Array 209, and Right Abdominal Sympathetic Trunk Neuromodulator Array 211, respectively

Implantable pulse generator 102 is connected via connecting cable 224, 226, 228, 230, and 232 to Left Abdominal Para Plexus Neuromodulator Array 204, Left Abdominal Greater Splanchnic Neuromodulator Array 206, Left Abdominal Lesser Splanchnic Neuromodulator Array 208, Left Abdominal Sympathetic Trunk Neuromodulator Array 210, and Left Abdominal Sympathetic Trunk Neuromodulator Array 212, respectively

Right Cervical Plexus Neuromodulator Array 193 modulates neural activity in Right Cervical Plexus 237. Right Intercostal Neuromodulator Array 195, Right Intercostal Neuromodulator Array 197, Right Intercostal Neuromodulator Array 199, and Right Intercostal Neuromodulator Array 201 each modulate neural activity in at least one of Right Sympathetic Trunk 71, Right Greater Splanchnic Nerve 73, and Right Lesser Splanchnic Nerve 75. Right Vagal Neuromodulator Array 233 modulates neural activity in Right Vagus Nerve 95.

Left Cervical Plexus Neuromodulator Array 194 modulates neural activity in Left Cervical Plexus 238. Left Intercostal Neuromodulator Array 196, Left Intercostal Neuromodulator Array 198, Left Intercostal Neuromodulator Array 200, and Left Intercostal Neuromodulator Array 202 each modulate neural activity in at least one of Left Sympathetic Trunk 72, Left Greater Splanchnic Nerve 74, and Left Lesser Splanchnic Nerve 76. Left Vagal Neuromodulator Array 234 modulates neural activity in Left Vagus Nerve 96.

Right Abdominal Para Plexus Neuromodulator Array 203 modulates at least one of Celiac Plexus 154, Celiac Ganglion 155, Superior Mesenteric Plexus 156, Superior Mesenteric Ganglion 157, Renal Plexus 158, Renal Ganglion 159, inferior Mesenteric Plexus 160, and Iliac Plexus 161. Right Abdominal Greater Splanchnic Neuromodulator Array 205 modulates Right Subdiaphragmatic Greater Splanchnic Nerve 78. Right Abdominal Lesser Splanchnic Neuromodulator Array 207 modulates Right Subdiaphragmatic Lesser Splanchnic Nerve 80. Right Abdominal Sympathetic Trunk Neuromodulator Array 209 and RightAbdominal Sympathetic Trunk Neuromodulator Array 211 each modulate at least one of Right Lumbar Sympathetic Ganglia 162, Right Sacral Sympathetic Ganglia 164, and Right Sympathetic Trunk 71.

Left Abdominal Para Plexus Neuromodulator Array 204 modulates at least one of Celiac Plexus 154, Celiac Ganglion 155, Superior Mesenteric Plexus 156, Superior Mesenteric Ganglion 157, Renal Plexus 158, Renal Ganglion 159, Inferior Mesenteric Plexus 160, and Iliac Plexus 161. Left Abdominal Greater Splanchnic Neuromodulator Array 206 modulates Left Subdiaphragmatic Greater Splanchnic Nerve 79. Left Abdominal Lesser Splanchnic Neuromodulator Array 208 modulates Left Subdiaphragmatic Lesser Splanchnic Nerve 81. Left Abdominal Sympathetic Trunk Neuromodulator Array 210 and Left Abdominal Sympathetic Trunk Neuromodulator Array 212 each modulate at least one of Left Lumbar Sympathetic Ganglia 163, Left Sacral Sympathetic Ganglia 165, and Left Sympathetic Trunk 72.

Elements comprising neuromodulators and neuromodulator arrays provide at least one of activating or inhibiting influence on neural activity of respective neurological target structures. Additional or fewer connecting cables and neuromodulator arrays may be employed without departing from the present invention.

These connections provided by connecting cables may facilitate communication and/or power transmission via electrical energy, ultrasound energy, optical energy, radiofrequency energy, electromagnetic energy, thermal energy, mechanical energy, chemical agent, pharmacological agent, or other signal or power means without departing from the parent or present invention.

Neuromodulator and neuromodulatory interface may be used interchangeably in this specification. Neuromodulator is a subset of modulator and modulates neural tissue.

Figure 30 shows the same invention taught in the parent case and shown in Figure 16, with detail shown fora a telemetrically powered linear catheter based electrode implementation for the neuromodulatory interfaces. In this Figure 30, the distal portion of the sympathetic nervous system is shown in more detail. In the parent case, modulation of the sympathetic nervous system was taught for the treatment of disease. This Figure 30 shows the same neuromodulator configuration shown in Figure 29, which is a potential arrangement of electrodes that becomes apparent to one skilled in the art upon reading the parent patent specification and figures. Each of the neuromodulator arrays includes a means for bidirectional transmission of information and power to and from at least one of an implantable pulse generator 99. 100, 101, and 102, and an External Transmitting and Receiving Unit 239. Each of the neuromodulator arrays includes a telemetry module, which serves as a means for bidirectional transmission of information and power to and from at least one of an implantable pulse generator 99. 100, 101, and 102 and External Transmitting and Receiving Unit 239. Each of the neuromodulator arrays includes a means for bidirectional transmission of information and power to and from at least one of an External Transmitting and Receiving Unit 239. Each of the implantable pulse generator 99.100,101, and 102 includes a means for bidirectional transmission of information and power to and from at least one of an External Transmitting and Receiving Unit 239.

External Transmitting and Receiving Unit 239 comprises modules including Controller 240, Memory 241, Bidirectional Transceiver 242, and User Interface 243. Additional or fewer modules may be included without departing from the present invention.

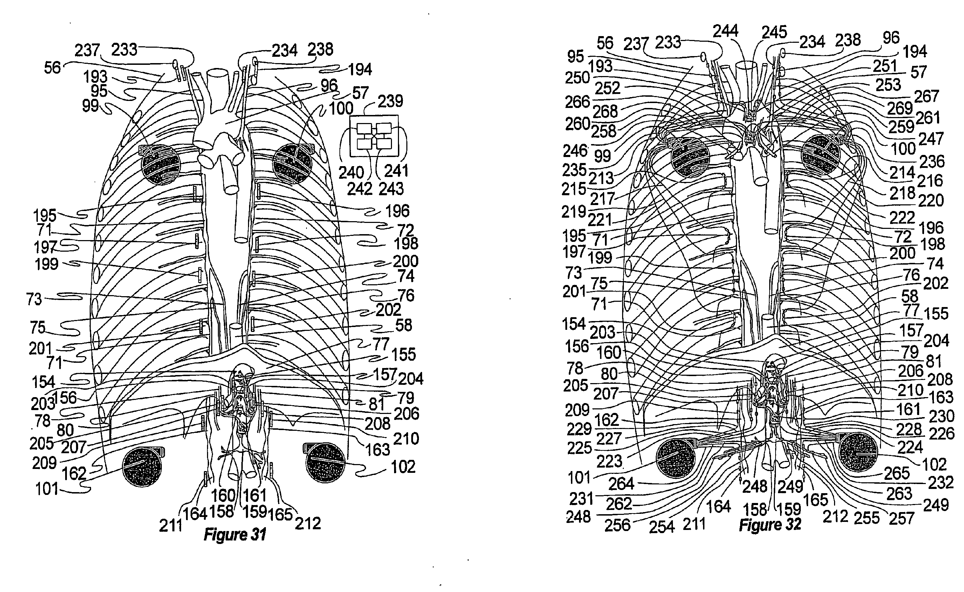

Figure 31 shows the same invention taught in the parent case and shown in Figure 16, with detail shown fora a telemetrically powered miniature enclosure based electrode implementation for the neuromodulatory interfaces. In one preferred embodiment, the neuromodulatory interfaces are implemented as injectable cylinders. These may have other cross sectional shapes, including flat meshes, paddles, or grid arrays, without departing from this invention. These may have other longitudinal profiles, including rectangular, tapered, serrated, convex, biconcave, or disk shapes, without departing from this invention. In this Figure 31, the distal portion of the sympathetic nervous system is shown in more detail. In the parent case, modulation of the sympathetic nervous system was taught for the treatment of disease. This Figure 31 shows the same neuromodulator configuration shown in Figure 29, which is a potential arrangement of electrodes that becomes apparent to one skilled in the art upon reading the parent patent specification and figures. Each of the neuromodulator arrays includes a means for bidirectional transmission of information and power to and from at least one of an implantable pulse generator 99. 100, 101, and 102, and an External Transmitting and Receiving Unit 239. The cylindrical enclosure based electrode implementation for the neuromodulatory interfaces may further be injectable or implantable via laparoscopic procedure, to facilitate minimally invasive implantation.

Neuromodulatory interfaces include an energy storage element, such as capacitor, battery, or inductor, for storage of power for delivery to at least one of tissue and on board electronic components.

External Transmitting and Receiving Unit 239 comprises modules including Controller 240, Memory 241, Bidirectional Transceiver 242, and User Interface 243. Additional or fewer modules and additional or fewer neuromodulatory interfaces may be included without departing from the present invention.

Figure 32: shows the same invention taught in the parent case and shown in Figure 16, with more anatomic detail shown for the autonomic nervous system and with placement of neuromodulatory interfaces for modulation of these structures.

In addition to the thoracic anatomical structures shown on Figure 29, the superficial cardiac plexus 244, deep cardiac plexus 245, right anterior pulmonary nerve 246, and left anterior pulmonary nerve 247 are depicted in Figure 32.

In addition to the abdominal anatomical structures shown on Figure 29, the renal plexus 158 and renal ganglion 159 are shown with more branches, including the right renal nerve branch 248, and left renal nerve branch 249.

The activity of these structures are modulated by corresponding neuromodulatory interfaces. Any of the previously described neuromodulatory interfaces in the parent case and the present case may be positioned to modulate these neural structures. Additional or alternate designs for neuromodulatory interfaces may be employed without departing from the present or parent invention.

Implantable pulse generator 99 is connected via connecting cable 213, 215, 217, 219, 221, 235, 258, 260, and 268 to Right Cervical Plexus Neuromodulator Array 193, Right Intercostal Neuromodulator Array 195, Right Intercostal Neuromodulator Array 197, Right Intercostal Neuromodulator Array 199, Right Intercostal Neuromodulator Array 201, and Right Vagal Neuromodulator Array 233, Right Superficial Cardiac Plexus Neuromodulator Array 250, Right Deep Cardiac Plexus Neuromodulator Array 252, Right Anterior Pulmonary Nerve Neuromodulator Array 266, respectively.

Implantable pulse generator 100 is connected via connecting cable 214, 216, 218, 220, 222, 236, 259, 261, and 269 to Left Cervical Plexus Neuromodulator Array 194, Left Intercostal Neuromodulator Array 196, Left Intercostal Neuromodulator Array 198, Left Intercostal Neuromodulator Array 200, and Left Intercostal Neuromodulator Array 202, and Left Vagal Neuromodulator Array 234, Left Superficial Cardiac Plexus Neuromodulator Array 251, Left Deep Cardiac Plexus Neuromodulator Array 253, Left Anterior Pulmonary Nerve Neuromodulator Array 267, respectively.

Implantable pulse generator 101 is connected via connecting cable 223, 225, 227, 229, 231, 262, and 264 to Right Abdominal Para Plexus Neuromodulator Array 203, Right Abdominal Greater Splanchnic Neuromodulator Array 205, Right Abdominal Lesser Splanchnic Neuromodulator Array 207, Right Abdominal Sympathetic Trunk Neuromodulator Array 209, and Right Abdominal Sympathetic Trunk Neuromodulator Array 211, Right Renal Plexus Neuromodulator Array 254, and Right Renal Nerve Branch Neuromodulator Array 256, respectively.

Implantable pulse generator 102 is connected via connecting cable 224, 226, 228, 230, 232, 263, and 265 to Left Abdominal Para Plexus Neuromodulator Array 204, Left Abdominal Greater Splanchnic Neuromodulator Array 206, LeftAbdominal Lesser Splanchnic Neuromodulator Array 208, Left Abdominal Sympathetic Trunk Neuromodulator Array 210, and LeftAbdominal Sympathetic Trunk Neuromodulator Array 212, LeftRenal Plexus Neuromodulator Array 255, and Left Renal Nerve Branch Neuromodulator Array 257, respectively

Right Cervical Plexus Neuromodulator Array 193 modulates neural activity in RightCervical Plexus 237. Right Intercostal Neuromodulator Array 195, Right Intercostal Neuromodulator Array 197, Right Intercostal Neuromodulator Array 199, and Right Intercostal Neuromodulator Array 201 each modulate neural activity in at least one of Right Sympathetic Trunk 71, Right Greater Splanchnic Nerve 73, and Right Lesser Splanchnic Nerve 75. Right Vagal Neuromodulator Array 233 modulates neural activity in Right Vagus Nerve 95.

Right Superficial Cardiac Plexus Neuromodulator Array 250 modulates neural activity in at least one of Superficial Cardiac Plexus 244 and other structures. Right Deep Cardiac Plexus Neuromodulator Array 252 modulates neural activity in at least one of Deep Cardiac Plexus 245 and other structures. Right Anterior Pulmonary Nerve Neuromodulator Array 266 modulates neural activity in at least one of Right Anterior Pulmonary Nerve 246 and other structures.

Left Cervical Plexus Neuromodulator Array 194 modulates neural activity in Left Cervical Plexus 238. Left Intercostal Neuromodulator Array 196, Left Intercostal Neuromodulator Array 198, Left Intercostal Neuromodulator Array 200, and Left Intercostal Neuromodulator Array 202 each modulate neural activity in at least one of Left Sympathetic Trunk 72, Left Greater Splanchnic Nerve 74, and Left Lesser Splanchnic Nerve 76. Left Vagal Neuromodulator Array 234 modulates neural activity in Left Vagus Nerve 96.

Left Superficial Cardiac Plexus Neuromodulator Array 251 modulates neural activity in at least one of Superficial Cardiac Plexus 244 and other structures. Left Deep Cardiac Plexus Neuromodulator Array 253 modulates neural activity in at least one of Deep Cardiac Plexus 245 and other structures. Left Anterior Pulmonary Nerve Neuromodulator Array 267 modulates neural activity in at least one of Left Anterior Pulmonary Nerve 247 and other structures.

Right Abdominal Para Plexus Neuromodulator Array 203 modulates neural activity in at least one of Celiac Plexus 154, Celiac Ganglion 155, Superior Mesenteric Plexus 156, Superior Mesenteric Ganglion 157, Renal Plexus 158, Renal Ganglion 159, Inferior Mesenteric Plexus 160, and Iliac Plexus 161. Right Abdominal Greater Splanchnic Neuromodulator Array 205 modulates neural activity in Right Subdiaphragmatic Greater Splanchnic Nerve 78. Right Abdominal Lesser Splanchnic Neuromodulator Array 207 modulates neural activity in Right Subdiaphragmatic Lesser Splanchnic Nerve 80. Right Abdominal Sympathetic Trunk Neuromodulator Array 209 and Right Abdominal Sympathetic Trunk Neuromodulator Array 211 each modulate neural activity in at least one of Right Lumbar Sympathetic Ganglia 162, Right Sacral Sympathetic Ganglia 164, and Right Sympathetic Trunk 71.

Right Renal Plexus Neuromodulator Array 254 modulates neural activity in at least one of Right Renal Nerve Branch 248, Renal Plexus 158, Renal Ganglion 159, and other structures. Right Renal Nerve Branch Neuromodulator Array 256 modulates neural activity in at least one of Right Renal Nerve Branch 248, Renal Plexus 158, Renal Ganglion 159, and other structures.

Left Abdominal Para Plexus Neuromodulator Array 204 modulates neural activity in at least one of Celiac Plexus 154, Celiac Ganglion 155, Superior Mesenteric Plexus 156, Superior Mesenteric Ganglion 157, Renal Plexus 158, Renal Ganglion 159, Inferior Mesenteric Plexus 160, and Iliac Plexus 161. Left Abdominal Greater Splanchnic Neuromodulator Array 206 modulates neural activity in Left Subdiaphragmatic Greater Splanchnic Nerve 79. Left Abdominal Lesser Splanchnic Neuromodulator Array 208 modulates neural activity in Left Subdiaphragmatic Lesser Splanchnic Nerve 81. Left Abdominal Sympathetic Trunk Neuromodulator Array 210 and Left Abdominal Sympathetic Trunk Neuromodulator Array 212 each modulate neural activity in at least one of Left Lumbar Sympathetic Ganglia 163, Left Sacral Sympathetic Ganglia 165, and Left Sympathetic Trunk 72.

Left Renal Plexus Neuromodulator Array 255 modulates neural activity in at least one of Left Renal Nerve Branch 249, Renal Plexus 158, Renal Ganglion 159, and other structures. Left Renal Nerve Branch Neuromodulator Array 257 modulates neural activity in at least one of Left Renal Nerve Branch 249, Renal Plexus 158, Renal Ganglion 159, and other structures.

Elements comprising neuromodulators and neuromodulator arrays provide at least one of activating or inhibiting influence on neural activity of respective neurological target structures. Additional or fewer connecting cables and neuromodulator arrays may be employed without departing from the present invention.

These connections provided by connecting cables may facilitate communication and/or power transmission via electrical energy, ultrasound energy, optical energy, radiofrequency energy, electromagnetic energy, thermal energy, mechanical energy, chemical agent, pharmacological agent, or other signal or power means without departing from the parent or present invention.

Neuromodulators and neuromodulatory interfaces may be used interchangeably in this specification.

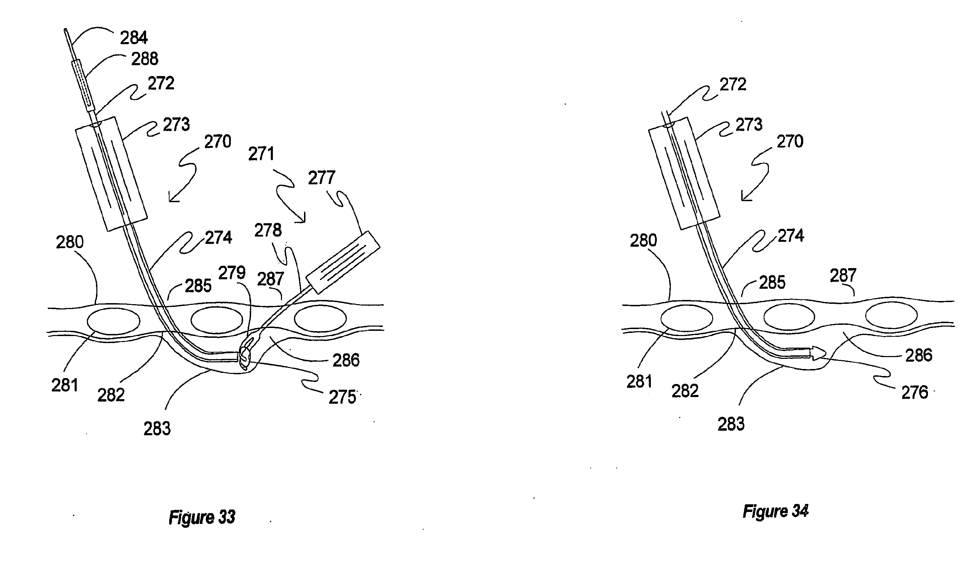

Figure 33 and 34: show the catheter insertion trocar 270 during intraoperative use for placement of neuromodulatory interface array catheter 284. Surgeon or assistant makes incision in skin 280, at entry point 285 in the cerivical, thoracic, lumbar, or sacral region. Figure 33 and 34 depict a skin incision at an entry point 285 which is shown in a representative site in the thoracic or lumbar region. Surgeon grasps catheter insertion trocar handle 273 and applies force which is transmitted through catheter insertion trocar shaft 274 to advance catheter insertion trocar bulb tip 275 through skin 280 and parietal pleura 282 into the potential space labeled pleural space 286 which is expanded by this procedure. Entry point 285 and exit point 287 are shown adjacent to but not directly overlying any of rib 281; however, either or both of entry point 285 and exit point 287 may overly any of rib 281, in which case tunneling under skin or through rib may be performed.

Care is taken to avoid perforating visceral pleura 283. Skin incision is made at entry point 285 through the majority of the thickness of skin 280 close to parietal pleura 282 to assist in minimizing the amount of force required to enter pleural space 286, thereby minimizing the velocity and acceleration of catheter insertion trocar bulb tip 275 during this procedure and reducing the risk of perforation of visceral pleura 283. A novelty of the present invention, shown in Figure 33, is the shape of catheter insertion trocar bulb tip 275, which is curved to further reduce the risk of perforation of visceral pleura 283.

Catheter insertion retriever 271 is inserted through an incision in skin 280 at the site of exit point 287. Surgeon or assistant grasps catheter insertion retriever handle 277, and with catheter insertion retriever shaft 286 penetrating skin 280, positions catheter insertion retriever grasper 279 to grasp catheter insertion trocar bulb tip 275 and to pull or guide attached catheter 272 through incision in skin 280 at exit point 287.

As shown in Figure 33, catheter insertion trocar bulb tip 275 may be part of catheter 272. Tensile and shear force applied through catheter insertion retriever grasper 279 is applied to pull and guide, respectively, catheter 272 in its advancement through pleural space 286 and through parietal pleura 282 and skin 280 at the site of exit point 287. Catheter attachment means 288 at the trailing end of catheter 272 enables neuromodulatory interface array catheter 284 to be pulled through skin 280 and parietal pleura 282 at entry point 285, through pleural space 286, and through parietal pleura 282 and skin 280 at exit point 287. Depending on the design, catheter insertion trocar 270 may be withdrawn prior to attachment of catheter 272 to neuromodulatory interface array catheter 284. Alternately, if said catheter attachment means 288 is sufficiently small relative to the internal diameter of catheter insertion trocar shaft 274, catheter insertion trocar 270 may be withdrawn after attachment of catheter 272 to neuromodulatory interface array catheter 284 and advancement of neuromodulatory interface array catheter 284 through skin 280 at exit point 287.

Figure 34 depicts a pointed design which facilitates advancement of catheter insertion trocar 270 into pleural space 286 and back through parietal pleura 282 and skin 280 at the site of exit point 287. As shown in this figure, pointed tip 276 is attached to or part of catheter 272. Alternatively, pointed tip 276 may be attached to or part of catheter insertion trocar shaft 274, without departing from the present invention.

In both Figure 33 and Figure 34, catheter 272 may serve as a guide to facilitate advancement of neuromodulatory interface array catheter 284 into position, as described above. Alternately, to save time and to reduce procedural complexity, catheter 272 may be replaced with neuromodulatory interface array catheter 284, without departing form the present invention. In this latter configuration, neuromodulatory interface array catheter 284 is advanced into position by catheter insertion trocar 270 in either of the two methods described and shown in Figure 33 and Figure 34.

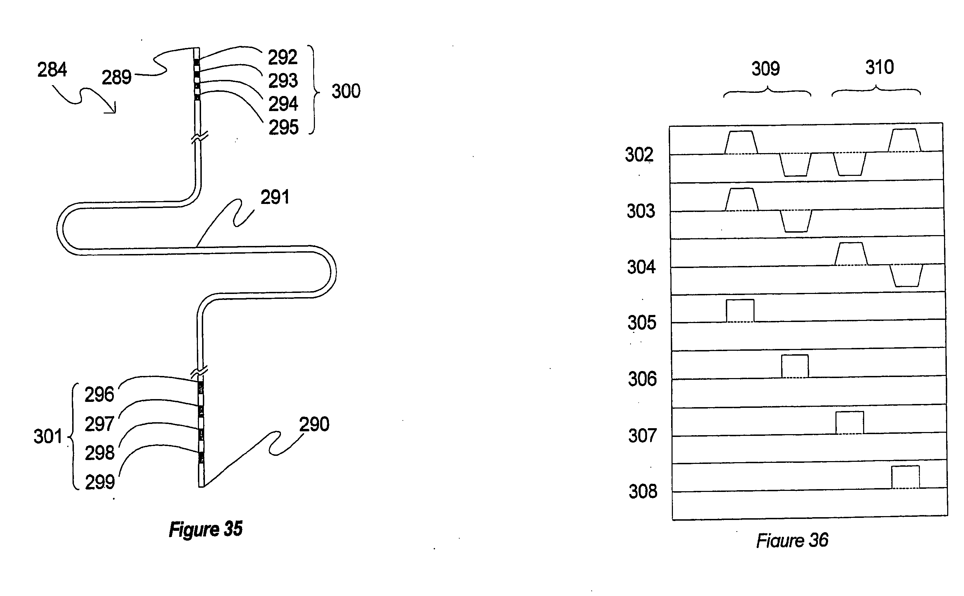

Figure 35 shows the neuromodulatory interface array catheter 284 which represent another implementation of the neuromodulatory interface 34 taught in the parent case and shown in multiple forms in Figure 16. In this embodiment, at least one neuromodulatory interface 34 is implemented as a single or plurality of neuromodulatory interface array catheter 284.

Neuromodulatory interface array catheter 284 comprises a connector contact array 300 located near connector end 289, a neuromodulatory interface array 301 located near neuromodulatory interface end 290, and catheter body 291, which provides mechanical connection and signal transmisison between connector contact array 300 and neuromodulatory interface array 301. Said signal transmission may be in the form of electrical fields or energy, electrical voltage, electrical current, optical energy, magnetic fields or energy, electromagnetic fields or energy, mechanical force or energy, vibratory force or energy, chemical agent or activation, pharmacological agent or activation, or other signal transmission means.

Neuromodulatory interface array 301 is comprised of at least one of neuromodulatory interface 296, 297, 298, and 299. Additional orfewer numbers of neuromodulatory interface may comprise neuromodulatory interface array 301 without departing from the present invention. Neuromodulator interface 296,297,298,299 modulate activity of neural structures using at least one of electrical fields or energy, electrical voltage, electrical current, optical energy, magnetic fields or energy, electromagnetic fields or energy, mechanical force or energy, vibratory force or energy, chemical agent or activation, pharmacological agent or activation, or other neural modulation means.

Connector contact array 300 is comprised of at least one of connector element 292, 293, 294, and 295. Additional or fewer numbers of connector element may comprise connector contact array 300 without departing from the present invention.

Figure 36 shows the effects of modulation of the autonomic nervous system, including periods of sympathetic modulation 309 and parasympathetic modulation 310. Sympathetic modulation 309 may be performed by stimulating or inhibiting activity in a portion of the sympathetic nervous system. Parasympathetic modulation 310 may be performed by stimulating or inhibiting activity in a portion of the parasympathetic nervous system.

Tracings showing the level of sympathetic stimulation 305 and sympathetic inhibition 306 are shown. During the time window in which sympathetic stimulation 305 is active, the sympathetic index 303 is seen to be increased and the autonomic index 302 is increased. During the time window in which sympathetic inhibition 306 is active, the sympathetic index 303 is seen to be decreased and the autonomic index 302 is decreased.

Tracings showing the level of parasympathetic stimulation 307 and parasympathetic inhibition 308 are shown. During the time window in which parasympathetic stimulation 307 is active, the parasympathetic index 304 is seen to be increased and the autonomic index 302 is decreased. During the time window in which parasympathetic inhibition 308 is active, the parasympathetic index 304 is seen to be decreased and the autonomic index 302 is increased.

Sympathetic and parasympathetic inhibition is accomplished by blockage of neural fibers. This is be performed using high frequency stimulation, with a best mode involving biphasic charge balanced waveforms delivered at frequencies over 100 Hz, though significantly higher as well as lower frequencies may be employed without departing form the present invention.

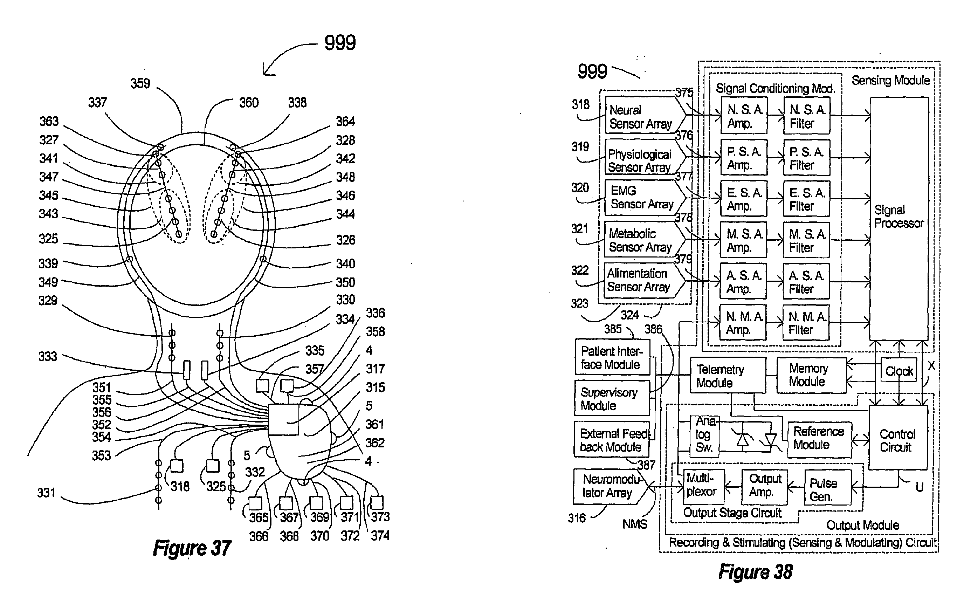

E. Intracranial - Subclavicular components. Figure 37 Shows a closed-loop stimulator circuit placed in a aubslcavicular pocket with intracranial and peripheral components..

Figure 37 is a schematic diagram of one embodiment of the neurological control system 999 of the present invention shown implanted in a human patient. The neurological control system 999 could be external or implanted as shown. A single or plurality of neurological control system 999, including bilateral application, may be used. Each neurological control system 999 includes a stimulating and recording unit 315 and one or more intracranial and extracranial components described below. As described in this illustrative embodiment, the intracranial components preferably include a neuromodulator array 316. These may be implemented as stimulating electrodes or as other elements designed to impart signals to neural structures and thereby modulate neural activity, including optical, ultrasound, electromagnetic sources as well as pharmacological or chemical emitters, or other means to alter neural activity. However, it should become apparent to those of ordinary skill in the relevant art after reading the present disclosure that the stimulating electrodes may also be extracranial; that is, attached to a peripheral nerve or autonomic neural structure in addition to or in place of being located within the cranium. As shown in Figure 37, stimulating and recording unit 315 of neurological control system 999 is preferably implanted in a subclavicular pocket. Alternately it may be implanted in a pericranial location, such as being recessed in the calvarum. Header 317 facilitates signal communication between stimulating and recording unit 315 and other components of neurological control system 999, such as neuromodulator array 316 and other sensors, modulators, communications modules, and other components. Some or al of the connections facilitated by header 317 may alternately be implemented using wireless technology.

As one skilled in the relevant art would find apparent from the following description, the configuration illustrated in Figure 37 is just one example of the present invention. Many other configurations are contemplated. For example, in alternative embodiments of the present invention, the stimulating and recording unit 315 is implanted ipsilateral or bilateral to particular intracranial or extracranial components. It should also be understood that the stimulating and recording unit 315 can receive ipsilateral, contralateral or bilateral inputs from sensors and deliver ipsilateral, contralateral, or bilateral outputs to a single or a plurality of intracranial or extracranial neuromodulator arrays 316, including stimulating and recording electrode arrays. Preferably, these inputs are direct or preamplified signals from at least one of sensor array 323, including neural sensor array 318, physiological sensor array 319, EMG sensor array 320, metabolic sensor array 321, alimentation sensor array 322, or other sensor array. Physiological sensor array 319 includes single and multiple modality sensor arrays, including but not limited to accelerometer array, acoustic transducer array, gastrointestinal pressure sensor array, gastrointestinal strain sensor array, gastrointestinal stress sensor array, temperature sensor array, glucose sensor array, heart rate sensor array, blood pressure sensor array, respiratory rate sensor array, respiratory pressure sensor array, respiratory acoustic sensor array, patient inpur sensor array, or other sensor array. Neural sensor array 318 includes any senros which generates a signal representative of neural activity, including but not limited to peripheral nerve electrode array, intracranial recording electrode array, other electrode array, neuromodulator array, or other neural sensing device. The signals input from these sensors will be referred to herein as "sensory input modalities" 324. The outputs include but are not limited to one or more signals, such as stimulating current signals or stimulating voltage signals or stimulating optical signals, to neuromodulator array 316.

Neuromodulator array 316 includes but is not limited to neuromodulator array 318, 319, 320, 321, 322, 323, modulator 2, 3, 24, 25, 26, 27, 28, 29, 30, 31, neuromodulatory interface 34, nerve cuff 36, longitudinal electrode array 38, regeneration electrode array 44, vagus nerve interface 45, sympathetic nerve interface 46, epineural electrode 49, 50, 51, sympathetic trunk neuromodulatory interface 83, 84, 85, 86, thoracic splanchnic neuromodulatory interface 87, 88,89,90, abdominal splanchnic neuromodulatory interface 91, 92, 93, 94, vagus neuromodulatory interface 97, 98, epineural cuff electrode neuromodulatory interface 117, longitudinal electrode neuromodulatory interface 118, 119, regeneration tube neuromodulatory interface 120, anterior central spinal neuromodulatory interface 143, anterior right lateral spinal neuromodulatory interface144, anterior left lateral spinal neuromodulatory interface 145, posterior central spinal neuromodulatory interface 146, posterior right lateral spinal neuromodulatory interface147, posterior left lateral spinal neuromodulatory interface 148, right lateral spinal neuromodulatory interface149, left lateral spinal neuromodulatory interface 150, intermediolateral nucleus neuromodulatory interface152, abdominal splanchnic neuromodulatory interface 170,171, neuromodulator array 174, 175, abdominal splanchnic neuromodulatory interface 178, 179, 180, 181, 182, 193, 184, 185, 186, right cervical plexus neuromodulatory array 193, left cervical plexus neuromodulatory array 194, right intercostal neuromodulatory array 195, 197, 199, 201, left intercostal neuromodulatory array 196, 198, 200, 202, right abdominal para plexus neuromodulatory array 203, left abdominal para plexus neuromodulatory array 204, right abdominal superior splanchnic neuromodulatory array 205, left abdominal superior splanchnic neuromodulatory array 206, right abdominal inferior splanchnic neuromodulatory array 207, left abdominal inferior splanchnic neuromodulatory array 208, right abdominal sympathetic trunk neuromodulatory array 209,211, left abdominal sympathetic trunk neuromodulatory array 210, 212, right vagal neuromodulator array 233, left vagal neuromodulator array 234, right superficial cardiac plexus neuromodulator array 250, left superficial cardiac plexus neuromodulator array 251, right deep cardiac plexus neuromodulator array 252, left deep cardiac plexus neuromodulator array 253, right renal plexus neuromodulator array 254, left renal plexus neuromodulator array 255, right renal nerve branch neuromodulator array 256, left renal nerve branch neuromodulator array 257, right anterior pulmonary nerve neuromodulator array 266, left anterior pulmonary nerve neuromodulator array 267, neuromodulatory interface 296, 297, 298, 299, neuromodulatory interface array 301, neuromodulator array 316, 325, 326, 327, 328, 329, 330, 331, 332, and other apparatus or methods which modulate neural activity. A single or plurality of elements of neuromodulator array 316 may also be used as a elements of a sensor array instead of or in addition to their function in modulating neural activity.

In the embodiment illustrated in Figure 37, neurological control system 999 is shown to receive bilateral sensory inputs and to deliver outputs through bilateral instances of neuromodulator array 316. In the illustrative embodiment, neurological control system 999 also receives sensory inputs from neuromodulator array316 and sensory input modalities 324, including neural sensor array 318, physiological sensor array 319, EMG sensor array 320, metabolic sensor array 321, alimentation sensor array 322, and other sensors arrays 323. Neural sensor array 321 comprises all neuromodulators 316 (including neuromodulator arrays 325, 326, 327, 328, 329, 330, 331, and 332) and neural sensors and electrodes, including EEG electrodes 337, 338, 339, and 340. Physiological Sensor Array 319 comprises physiological sensor array 333,334, and 335. physiological sensor array 333, 334, and 335 are connected to stimulating and recording circuit 315 via physiological sensor array connecting cable 355, 356, and 357, respectively.

Physiological sensor array 319 senses at least one of any physiological parameter comprising temperature, hear rate, heart rate variability, any cardiac parameter, blood pressure, respiratory rate, respiratory function parameters and pressures, metabolic rate, respiratory quotient, glucose level, insulin level, organ perfusion, or other physiological parameter. Additional or fewer sensors and/or neuromodulators may be used without departing from the present invention.

Superficial intracranial electrode array 341 and 342 modulate and sense activity from superficial regions of the nervous system, including the cortex, subdural space, epidural space, calvaral space, subgaleal space, subcutaneous space and/or scalp region. Deep intracranial electrode array 343 and 344 modulate and sense activity from deep brain regions, including but not limited to subcortical nuclei and white matter tracts, brainstem structures, and medial and lateral and other components of the hypothalamus and all satiety centers.

Neural sensor array 318 generates neural signals representative of neural activity, including but not limited to signals from cortical, white matter, and deep brain nuclear signals. Neural activity to be sensed and neural activity to be modulated includes but is not limited to that found in the sympathetic nervous system, parasympathetic nervous system, autonomic nervous system, baroreceptor neural circuit components, primary motor cortex, premotor cortex, supplementary motor cortex, other motor cortical regions, somatosensory cortex, other sensory cortical regions, Broca's area, Wernickie's area, other cortical regions, white matter tracts associated with these cortical areas, otherwhite matter tracts, the globus pallidus internal segment (GPi, GPi,e, GPi,e), the globus pallidus external segment, the caudate, the putamen, locus ceruleus, and other cortical and subcortical areas, ventral medial Vim thalamic nucleus, other portion of the thalamus, subthalamic nucleus (STN), caudate, putamen, other basal ganglia components, cingulate gyrus, other subcortical nuclei, nucleus locus ceruleus, pedunculopontine nuclei of the reticular formation, red nucleus, substantia nigra, other brainstem structure, cerebellum, internal capsule, external capsule, corticospinal tract, pyramidal tract, ansa lenticularis, other central nervous system structure, other peripheral nervous system structure, other intracranial region, other extracranial region, other neural structure, sensory organs, muscle tissue, or other non-neural structure.

This is one embodiment. Numerous permutations of electrode stimulation site configuration may be employed, including more or fewer electrodes in each of these said regions, without departing from the present invention. Electrodes may be implanted within or adjacent to other regions in addition to or instead of those listed above without departing from the present invention.

As one of ordinary skill in the relevant art will find apparent, the present invention may include additional or different types of sensors that sense neural responses for the type and particular patient. Such sensors generate sensed signals that may be conditioned to generate conditioned signals, as described below. Examples of the placement of these electrodes is described above with reference to the embodiment illustrated in these figures. Many others are contemplated by the present invention.

Neural sensor array 318 is connected to recording and stimulating circuit 315 with neural sensor array connecting cable 375. In one embodiment, neural sensor array 318 comprises, at least one of neuromodulatory interface 34, nerve cuff 36, longitudinal electrode array 38, regeneration electrode array 44, vagus nerve interface 45, sympathetic nerve interface 46, epineural electrode 49, 50, and 51, sympathetic trunk neuromodulatory interface 83,84, 85, and 86, thoracic splanchnic neuromodulatory interface 87,88,89, and 90, abdominal splanchnic neuromodulatory interface 91, 92, 93, and 94, vagus neuromodulatory interface 97 and 98, epineural cuff electrode neuromodulatory interface 117, longitudinal electrode neuromodulatory interface 118, longitudinal electrode regeneration port neuromodulatory interface 119, regeneration tube neuromodulatory interface 120, and any other potential component comprising neuromodulator array 316, which is described above. A single or multiplicity of peripheral nerve interface 380, comprising vagus neuromodulatory interface 97 and 98, vagus nerve interface 45, sympathetic nerve interface 46, or other neural interface may be located in the cervical region, thoracic region, lumbar region, sacral region, abdominal region, pelvic region, the head, cranial nerves, neck, torso, upper extremities, and lower extremities, without departing from the present invention. Peripheral nerve interface 380, when located in the neck region, can interface with the vagus nerve, sympathetic ganglia, spinal accessory nerve, or nerve arising from cervical roots.

In one embodiment, peripheral nerve interface 380 are each comprised of three epineural platinum-iridium ring electrodes, each in with an internal diameter approximately 30% larger than that of the epineurium, longitudinally spaced along the nerve. Electrodes of differing dimensions and geometries and constructed from different materials may alternatively be used without departing from the present invention. Alternative electrode configurations include but are not limited to epineural, intrafascicular, or other intraneural electrodes; and materials include but are not limited to platinum, gold, stainless steel, carbon, and other element or alloy.

As will become apparent from the following description, signals representing various sensory input modalities 324 from sensor arrays 323 may provide valuable feedback information.

It should be understood that this depiction is for simplicity only, and that any combination of ipsilateral, contralateral or bilateral combination of each of the multiple sensory input modalities and multiple stimulation output channels may be employed. In addition, neurological control system 999 may be a single device, multiple communicating devices, or multiple independent devices. Accordingly, these and other configurations are considered to be within the scope of the present invention. It is anticipated that neurological control system 999, if implemented as distinct units, would likely be implanted in separate procedures (soon after clinical introduction) to minimize the likelihood of drastic neurological complications.

In the exemplary embodiment illustrated in Figure 37, intracranial components 345 and 346 include intracranial catheter 347 and 348, one preferred embodiment of which comprise a plurality of intracranial stimulating and recording electrodes. Superficial intracranial electrode array 341 and 342 may, of course, have more or fewer electrodes than that depicted in Figure 37. These intracranial stimulating electrodes may be used to provide stimulation to a predetermined nervous system component. The electrical stimulation provided by the intracranial stimulating electrodes may be excitatory or inhibitory, and this may vary in a manner which is preprogrammed, varied in real-time, computed in advance using a predictive algorithm, or determined using another technique now or latter developed.

Intracranial catheters 347 and 348 include neuromodulator arrays 325, 326, 327, and 328, which may comprise intracranial recording electrodes and/or intracranial stimulating electrodes. In accordance with one embodiment of the present invention, intracranial recording electrodes are used to record cortical activity as a measure of response to treatment and as a predictor of impeding treatment magnitude requirements. In the illustrative embodiment, neuromodulator arrays 327 and 328, which may be implemented as superficial intracranial electrode array 341 and 342 are depicted in a location superficial to neuromodulator arrays 325 and 326, which may be implemented as deep intracranial electrode arrays 343 and 344.

In the illustrative embodiment, an intracranial catheters 347 and 348 are provided to mechanically support and facilitate communication of electrical, optical, or other signal and/or power modality between intracranial and extracranial structures. In this embodiment, intracranial catheters 347 and 348 contain one or more wires, optical fibers, telemetry links or other means facilitating connecting stimulating and recording circuit 315 to the intracranial components 345 and 346, including but not limited to neuromodulator array 316, which may comprise intracranial stimulating electrodes, intracranial recording electrodes, as well as extracranial stimulating electrodes and extracranial recording electrodes, and other sensors and modulators. The wires contained within intracranial catheters 347 and 348 transmit neuromodulation signal (NMS) 998 or stimulating electrode output signal (SEOS) to superficial intracranial electrode arrays 341 and 342 and to deep intracranial electrode arrays 343 and 344. Wires are understood to also include other communications medium, comprising optical fibers, ultrasound conduits, wireless telemetry modules, and the like. Such wires additionally transmit stimulating electrode input signal (SEIS) and recording electrode input signal (REIS), to and from superficial intracranial electrode arrays 341 and 342 and to and from deep intracranial electrode arrays 343 and 344. Other recording and stimulating or modulating modalities may be used in addition to or instead of electrode arrays without departing from the present invention.

Stimulating and recording circuit 315 is protected within circuit enclosure 361. Circuit enclosure 361 and contained components, including stimulating and recording circuit 315 comprise stimulating and recording unit 362. It should be understood that more or fewer of either type of electrode as well as additional electrode types and locations may be incorporated or substituted. Furthermore, stimulating and recording circuit 315 can be placed extra cranially in a subclavian pocket as shown in Figure 37, or it may be placed in other extracranial, intracranial, or nonimplanted locations.

Connecting cable 349 and 350 generally provide electrical, optical, chemical or other signal connection between intracranial or intracranial locations. A set of electrical wires is one mans which provides the for electrical communication between the intracranial and extracranial components; however, it should be understood that alternate systems and techniques such as radiofrequency links, optical (including infrared) links with transcranial optical windows, magnetic links, and electrical links using the body components as conductors, may be used without departing from the present invention. Specifically, in the illustrative embodiment, connecting cable 349 and 350 provide electrical connection between intracranial components 345 and 346 and stimulating and recording circuit 315. In embodiments wherein stimulating and recording circuit 315 has an intracranial location, connecting cable 349 and 350 would likely be entirely intracranial. Alternatively, connecting in embodiments wherein stimulating and recording circuit 315 is implanted under scalp 359 or within or attached to calvarum 360, connecting cable 349 and 350 may be confined entirely to subcutaneous region under the scalp 359.

A catheter anchor 363 and 364 provide mechanical connection between intracranial catheter 347 and 348 and calvarum 360. Catheter anchor 363 and 364 are preferably deep to the overlying scalp 359. Such a subcutaneous connecting cable 349 and 350 provides connection between stimulating and recording circuit 26 and at least one of superficial intracranial electrode array 341 and 342, deep intracranial electrode array 343 and 344, other neuromodulator array 316, neural sensor array 318, physiological sensor array 319, metabolic sensorarray 321, orother sensor array 323. Connecting cable 349 and 350 may also connect any other sensors, including but not limited to any of sensory input modalities 324, or other stimulating electrodes, neuromodulators, medication dispensers, or actuators with stimulating and recording circuit 315.

Sensory feedback is provided to stimulating and recording circuit 315 from a multiplicity of sensors, collectively referred to as sensory input modalities 324. Neural sensor array 318 comprises superficial intracranial electrode array 341 and 342, deep intracranial electrode array 343 and 344, and other intracranial and extracranial recording electrode arrays and other neural sensors and neuromodulators. Additional sensors, some of which are located extracranially in the embodiment, comprise the remainder of sensory input modalities 324. Sensory input modalities 324 provide information to stimulating and recording circuit 315. As will be described in greater detail below, such information is processed by stimulating and recording circuit 315 to deduce the disease state and progression and its response to therapy.

For example, disease state comprises metabolic state, cardiovascular parameters, respiratory parameters, affect qualities or parameters, psychosis qualities or parameters, insulin and glucose levels or parameters, irritable bowel syndrome qualities or parameters, or any quality or metric related to a disease, disorder, condition, neurological, psychiatric, or physiological state.

In one embodiment of the invention, physiological sensor array 319 comprises an acoustic transducer array 336 to monitor any number of vibratory characteristics such as high frequency head or body vibration, muscle vibration, speech production, blood flow, airflow, and/or other physiological parameter. Acoustic transducer array 336 comprises at least one of an acoustic sensor or an acoustic transducer and is connected to stimulating and recording circuit 315 with acoustic transducer array connecting cable 358.

In one embodiment of the invention, physiological sensor array 319 comprises temperature sensor array 365 to monitor local temperature, body temperature, or ambient temperature. Temperature sensor array 365 is connected to stimulating and recording circuit 315 with temperature sensor array connecting cable 366.

In one embodiment of the invention, physiological sensor array 319 comprises respiratory sensor array 367 to monitor at least one of pulmonary pleura pressure, inter-bronchial pressure, inter-alveolar pressure, transpleural pressure, transbronchial pressure, thransthoracic pressure, other pressure related to pulmonary or respiratory function, bronchial air flow, alveolar airflow, tracheal airflow, or other airflow or blood flow related to pulmonary or respiratory function. Respiratory sensor 367 may be implemented as at least one of a pressure sensor, flow sensor, Doppler transceiver and/or sensor, acoustic sensor and/or transducer, electrical impedance sensor and/or transducer, mechanical impedance sensor and/or transducer, or other sensor or transducer. Respiratory sensor array 367 is connected to stimulating and recording circuit 315 with respiratory sensor array connecting cable 368.

In one embodiment of the invention, physiological sensor array 319 comprises pressure sensor array 369 to monitor a pressure related to function of at least one of pulmonary function, respiratory function, cardiac function, cardiovascular function, vascular function, gastrointestinal function, alimentary function, gastricfunction, pyloric function, duodenun function, jejunum function, ileum function, small intestinal function, large intestine function, cecum function, sigmoid function, rectum function, bladder function, ovulatory function, ejaculatory function, other pressure listed in this specification, or other physiological function. Pressure sensor array 369 is connected to stimulating and recording circuit 315 with pressure sensor array connecting cable 370.

In one embodiment of the invention, physiological sensor array 319 comprises cardiovascular sensor array 371 to monitor at least one parameter related to cardiac, cardiovascular, or vascular function or physiology. Example parameters sensed by cardiovascular sensor array 371 comprise intracardiac pressure, right atrium pressure, left atrium pressure, right ventricle pressure, left ventricle pressure, intramural pressure, transmural pressure, pericardial pressure, intraluminal pressure, transvalvular pressure, transthoracic pressure, aortic pressure, pulmonary arterial pressure, central venous pressure, pulmonary venous pressure, arterial pressure, venous pressure, left ventricular end diastolic pressure, LVEDP, intracardiac blood flow, aortic blood flow, pulmonary arterial blood flow, or other pressure or flow related to cardiac function, cardiovascular function, or vascular function. Cardiovascular sensor array 371 may be implemented as at least one of a pressure sensor, flow sensor, Doppler transceiver and/or sensor, acoustic sensor and/or transducer, electrical impedance sensor and/or transducer, mechanical impedance sensor and/or transducer, or other sensor or transducer. Cardiovascular sensor array 371 is connected to stimulating and recording circuit 315 with cardiovascular sensor array connecting cable 372.

In one embodiment of the invention, physiological sensor array 319 comprises glucose sensor array 373 to monitor at least one parameter related to glucose, glycogen, and insulin level and metabolism. Example parameters sensed by glucose sensor array 373 comprise blood glucose level, tissue glucose level, other fluid glucose level, blood glycogen level, tissue glycogen level, otherfluid glycogen level, blood insulin level, tissue insulin level, otherfluid insulin level, other substance level reflective of levels or metabolism of glucose, glycogen, or insulin. Glucose sensor array 373 may be implemented using chemical, biological, optical, electronic, affinity array, or other known or new technologies for sensing such levels. Glucose sensor array 373 is connected to stimulating and recording circuit 315 with glucose sensor array connecting cable 374.

In one embodiment of the invention, physiological sensor array 319 comprises an to monitor head or body position and movement with respect to gravity. Accelerometer may be mounted to any structure or structures that enables it to accurately sense a position or movement. Such structures include, for example, the skull base, calvarum, clavicle, mandible, extraocular structures, soft tissues and vertebrae. Accelerometer is connected to stimulating and recording circuit 315 with an accelerometer connecting cable. Accelerometer may be used to sense body position, such as recumbancy, and provide information useful to determine circadian rhythm and sleep-wake cycle.

An electromyography (EMG) sensor array 320 is also included in certain embodiments of the invention. EMG sensor array 320 preferably includes a positive proximal EMG electrode, a reference proximal EMG electrode, and a negative proximal EMG electrode. As one skilled in the relevant art would find apparent, EMG sensor array may include any number of type of electrodes. EMG sensor array 320 is non-implanted overlying muscle tissue or is implanted in or adjacent to muscle tissue. EMG electrode array 320 may be located to sense activity of skeletal muscle, smooth muscle, or cardiac muscle and may therefore be used for many sensory modalities comprising motor function, visceral function including gastrointestinal and alimentary function, respiratory function, cardiac function, and other physiological function.

Acoustic transducer array 336 may also be implemented in the present invention. Acoustic transducer array 336 senses muscle vibration and may be used to augment, supplement or replace EMG recording. Also, acoustic transducer array 336 may be used to sense movement, including tremor and voluntary activity. Acoustic transducer array 336 may be used to sense respiratory function, including onset of symptoms of asthma.

It should also be understood from the preceding description that the number of each type of sensor may also be increased or decreased, some sensor types may be eliminated, and other sensor types may be included.

### F. System / Pulse Generator Design.

Figure 38 is an architectural block diagram of one embodiment of the neurological control system 999 of the present invention for modulating the activity of at least one nervous system component in a patient. As used herein, a nervous system component includes any component or structure comprising an entirety or portion of the nervous system, or any structure interfaced thereto. In one preferred embodiment, the nervous system component that is controlled by the present invention includes the sympathetic nervous system. In another preferred embodiment, the controlled nervous system component is the parasympathetic nervous system. In yet another preferred embodiment, the controlled nervous system component is at least one component of the hypothalamus. In an additional preferred embodiment, the controlled nervous system component is at least one component of the pituitary.

Stimulating and recording unit 362, comprises stimulating and recording circuit 315, circuit enclosure 361, header 317, and a single or plurality of attachment fixture 4 and 5. Stimulating and recording unit 362 is also a preferred embodiment of implantable pulse generator 99, 100, 101, 102, which are understood to be implanted or alternatively nonimplanted.

The neurological control system 999 includes one or more implantable or noninvasive components including one or more sensors each configured to sense a particular characteristic indicative of a neurological, psychiatric, or metabolic condition.

### G. Stimulation Parameters

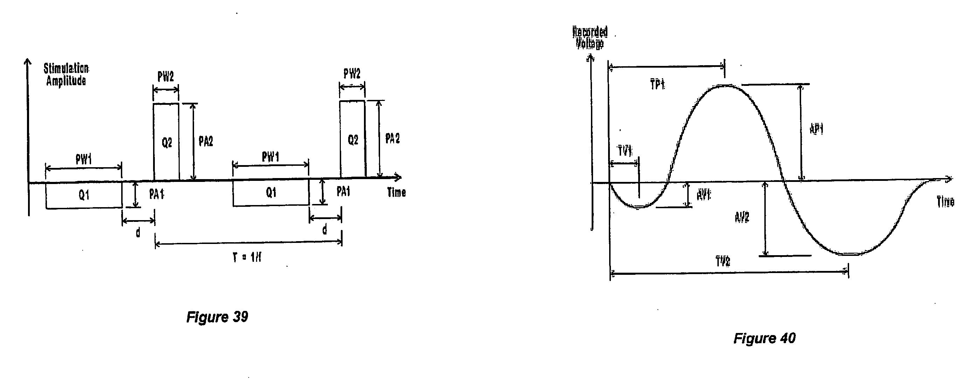

Figure 39 is a schematic diagram of electrical stimulation waveforms for neural modulation. The illustrated ideal stimulus waveform is a charge balanced biphasic current controlled electrical pulse train. Two cycles of this waveform are depicted, each of which is made of a smaller cathodic phase followed, after a short delay, by a larger anodic phase. In one preferred embodiment, a current controlled stimulus is delivered; and the "Stimulus Amplitude" represents stimulation current. A voltage controlled or other stimulus may be used without departing from the present invention. Similarly, other waveforms, including an anodic phase preceding a cathodic phase, a monophasic pulse, a triphasic pulse, multiphasic pulse, or the waveform may be used without departing from the present invention.

The amplitude of the first phase, depicted here as cathodic, is given by pulse amplitude 1 PA1; the amplitude of the second phase, depicted here as anodic, is given by pulse amplitude 2 PA2. The durations of the first and second phases are pulse width 1 PW1 and pulse width 1 PW2, respectively. Phase 1 and phase 2 are separated by a brief delay d. Waveforms repeat with a stimulation period T, defining the stimulation frequency as f = 1/T.

The area under the curve for each phase represents the charge Q transferred, and in the preferred embodiment, these quantities are equal and opposite for the cathodic (Q1) and anodic (Q2) pulses, i.e. Q = Q1 = Q2. For rectangular pulses, the charge transferred per pulse is given by Q1 = PA1 * PW1 and Q2 = PA2 * PW2. The charge balancing constraint given by -Q1 = Q2 imposes the relation PA1 * PW1 = -PA2 * PW2. Departure from the charge balancing constraint, as is desired for optimal function of certain electrode materials, in included in the present invention.

The stimulus amplitudes PA1 and PA2, durations PW1 and PW2, frequency f, or a combination thereof may be varied to modulate the intensity of the said stimulus. A series of stimulus waveforms may be delivered as a burst, in which case the number of stimuli per burst, the frequency of waveforms within the said burst, the frequency at which the bursts are repeated, or a combination thereof may additionally be varied to modulate the stimulus intensity.

Typical values for stimulation parameters include f = 100-300 Hz, PA1 and PA2 range from 10 microamps to 10 milliamps, PW1 and PW2 range from 50 microseconds to 100 milliseconds. These values are representative, and departure from these ranges is included in the apparatus and method of the present invention.

Safe stimulation current waveforms may be achieved for stimulus waveforms which satisfy charge injection limits. For stimulation of peripheral nerves, sympathetic nerves, sympathetic trunk, sympathetic plexus, vagus nerve, and other neural structures, such as may be performed using peripheral nerve interface 380, charge injection limits may be selected to be approximately or less than 50 microcoulombs per square centimeter for stainless steel electrodes and approximately or less than 25 microcoulombs per square centimeter for Platinum-Iridium (Pt/Ir)electrodes.

For a design as shown in Figures 8 and 9, in which exposed electrode wire tips comprise the active electrode site, an example set of dimensions for a stainless steel implementation of this electrode are a diameter of 50 microns and an exposed length of 2,000 microns (2 mm), resulting in a gross surface area of 314,000 square microns. This may increase substantially if the surface is roughened. The 50 microcoulomb per square centimeter charge injection limit for such a stainless steel electrode would be 0.157 microcoulombs, which would be satisfied by stimulation waveform of amplitude 1.57 milliamperes and pulse width 100 microseconds. For an example electrode resistance of 4,000 ohms, the required stimulation voltage would be 6.28 volts.

An example set of dimensions for a Platinum-Iridium implementation of this electrode are a diameter of 127 microns and an exposed length of 2,000 microns (2 mm), resulting in a gross surface area of 797,560 square microns. This may increase substantially if the surface is roughened. The 25 microcoulomb per square centimeter charge injection limit for such a Platinum-Iridium electrode would be 0.199 microcoulombs, which would be satisfied by stimulation waveform of amplitude 1.99 milliamperes and pulse width 100 microseconds. For an example electrode resistance of 4,000 ohms, the required stimulation voltage would be 7.97 volts.

These dimensions are for example only, and much larger or smaller electrode dimensions and configurations, including those shown in Figures 7, 8, 9, and 10, and other figures in the present invention, and other electrode designs without departing from the present invention.

### H. Recording Signals

Figure 40 is a schematic diagram of electrical recording waveforms from neural or muscular structures. These are sensed by any of sensor array 323 and transmitted to recording and stimulation circuit 315 for processing and disease state estimation.

### I. Control

In one preferred embodiment, sympathetic index is modulated to control at least one of metabolic rate, body temperature, food intake, blood pressure, heart rate, respiratory gas flow, pulmonary function parameters, cardiac parameters, cardiovascular parameters, vascular parameters, and other parameters.

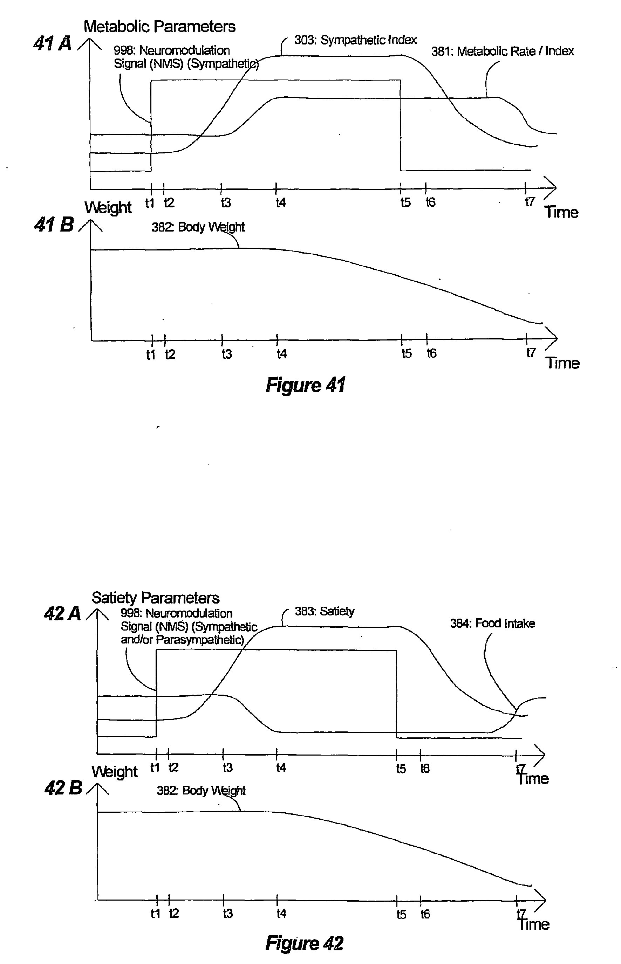

Figure 41 is a diagram depicting metabolic modulation. Neuromodulation Signal (NMS) 998 is delivered to the sympathetic nervous system, in the sympathetic trunk, splanchnic nerves, celiac plexus, other nerves or plexi, and/or intracranial locations including hypothalamus. NMS 998 causes an increase in sympathetic index 303, which results in an increase in metabolic rate 381 or metabolic index, which results in a decline in body weight 382, achieving therapeutic effect in the treatment of obesity.

Figure 42 is a diagram depicting satiety modulation or appetite modulation. Neuromodulation Signal (NMS) 998 is delivered to the autonomic nervous system. The autonomic nervous system includes components of the sympathetic nervous system, including the sympathetic trunk, splanchnic nerves, celiac plexus, other nerves or plexi, and/or intracranial locations including hypothalamus. The autonomic nervous system includes components of the parasympathetic nervous system, including the vagus nerve and parasympathetic afferents, and portions of the solitary nucleus. NMS 998 may causes an increase in sympathetic index 303 and/or in parasympathetic index 304, and causes an increase in satiety 383, which results in a decrease in food intake 384, which results in a decline in body weight 382, achieving therapeutic effect in the treatment of obesity.

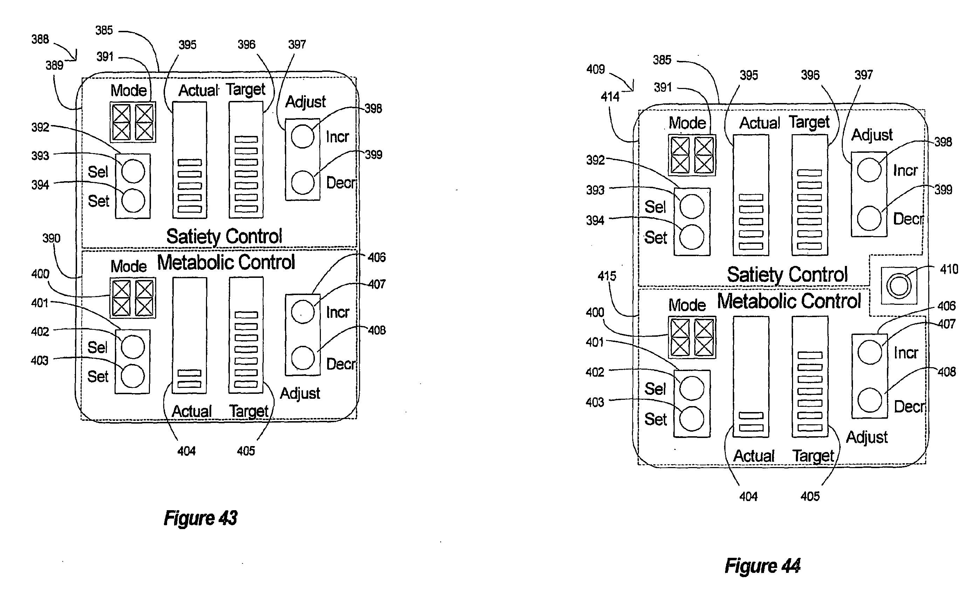

Figure 43 depicts one implementation of an Autonomic Neuromodulation Programmer 388. Numerous other implementations of this and the other interfaces described herein may be conceived and designed without departing from the present invention. This may be implemented using other input devices, buttons, switches, toggles, output displays, arrangements thereof, display technologies, liquid crystal displays (LCDs), light emitting diode (LED) displays, plasma displays, touch screens, software and hardware, and other existing orfuture technologies without departing from the present invention. Autonomic neuromodulation programmer 388 may comprise a portion of at least one of Patient Interface Module 385, Supervisory Module 386, External Feedback Module 387, or other device which communicates with any portion of the neurological control system 999 which may be implanted or attached or in proximity to the body of the user.

Autonomic Neuromodulation Programmer 388 typically comprises at least one of Satiety Control Interface 389 and Metabolic Control Interface 390. Autonomic Neuromodulation Programmer 388 may comprise additional components or fewer components arranged in any manner without departing from the present invention.

Satiety Control Interface 389 facilitates the setting of control parameters for the neurological control system 999 for the control of satiety 383, which is inversely related to the sensation of hunger, which may also be called hunger pains. Satiety control interface 389 facilitates entry of a singularity or plurality of satiety control inputs, which may comprise a vector of values, a set of scalars, a set of vectors, a collection of different parameters relating to various quantifications, qualities, or parameters related to satiety, hunger, hunger pains, cravings, or other subjective or objective experiences related to food intake. Satiety Mode Display 391, depicted as an alphanumeric display, communicates to the user the satiety control mode being programmed. This may specify a particular parameter for the autonomic modulation, including but not limited to stimulation waveform parameter, autonomic index, sympathetic index, parasympathetic index, metric of satiety, a magnitude parameter relating to satiety control, a temporal parameter relating to satiety control, a parameter relating to timing of meals, a parameter relating to timing of stimulus relative to timing of meals, a parameter relating to magnitude of stimulation related to meals, or other parameter related to the control or modulation of satiety or hunger sensations or hunger pains.

Satiety control inputs may specify a singularuty or plurality of inputs relating to the planned or selected regimen for ameliorating hunger and achieving satiety. Satiety control inputs may include timing and magnitude of neuromodulation signal (NMS) or related parameter specifying timing, magnitude, or other parameter for autonomic modulation, including but not limited to stimulation waveform parameter, autonomic index, sympathetic index, parasympathetic index to induce satiety. This may include a singularity or plurality of satiety target levels, and corresponding satiety actual levels, which may comprise baseline satiety level, preprandial satiety level, postprandial satiety level, periprandial satiety level, daytime satiety level, nighttime satiety level, satiety level between meal times, or other satiety level. By modulating satiety levels, neurological control system 999 reduces food intake. By modulating at least one of preprandial satiety levels, postprandial satiety levels, periprandial satiety levels, neurological control system 999 reduces food intake at mealtime, enabling the user to achieve satiety on a smaller meal size. By modulating at least one of daytime satiety levels, night time satiety levels, inter-prandial satiety levels (between meal times), neurological control system 999 reduces food intake between mealtimes, enabling the user to achieve a reduction in snacking behavior and overall food intake. Satiety control inputs may also specify a singularity or plurality of a parameters relating to timing of meals, parameters relating to timing of stimulus relative to timing of meals, parameters relating to magnitude of stimulation related to meals, or other parameter related to the control or modulation of satiety which may comprise a quantification of at least one of degree of satiety, degree of hunger suppression, degree of hunger pain, degree of food craving, or other related parameter, other metric related to satiety, and combination of metrics or parameters related to satiety. Satiety control inputs may comprise target satiety levels and actual satiety levels relating to a variety of satiety states including but not limited to resting satiety level, preprandial satiety level (before meals), periprandial satiety level (around meal time), postprandial satiety level (after meal time), inter-prandial satiety level (between meals), daytime satiety levels, night time satiety levels, or other satiety levels.

Satiety Mode Adjuster 392 facilitates the selection of a satiety mode to program and the setting of parameters for control of satiety 383. Satiety Mode Adjuster 392 comprises Satiety Mode Select Input 393, which enables the user to select among at least one satiety control mode or satiety control parameter or other parameter or mode related to satiety control. Satiety Mode Adjuster 392 further comprises Satiety Mode Set Input 394, which enables the user to set or program the satiety control mode or satiety control parameter or other parameter or mode related to satiety control.

Satiety Actual Level Display 395 displays a current or actual metric of or function of satiety. This may be estimated from physiological parameter such as glucose level, insulin level, gastrointestinal physiological parameter, other parameter related to autonomic activity, including cardiac parameters such as heart rate and blood pressure, and respiratory parameters such as respiratory rate and carbon dioxide production and respiratory exchange ratio. Actual satiety level may also be estimated from time since prior meal or time until next anticipated meal, or other parameter related to at least one of meal pattern, time since last meal, time until next expected meal, size of last meal, nutritional content of prior meals, caloric content of past meals, carbohydrate content of last meals, insulin response to prior meals, insulin level, history of prior insulin levels, cortisol level, history of prior cortisol levels, level of other hormones, history of prior levels of other hormones, other endocrinological parameters, history of other prior endocrinological parameters, circadian cycle, or other parameter or sets of parameters.

Satiety Target Level Display 396 displays the target satiety level, satiety control parameter value, or other parameter related to satiety being adjusted by Satiety Target Level Adjuster 397. Satiety Target Level Adjuster 397 facilitates the adjustment of the target value of the selected satiety control parameter or other parameter related to satiety 398 which is selected for adjustment. Satiety Target Level Adjuster 397 may be implemented in any of numerous ways without departing from the present invention; this includes the use of one or more knobs, rollers, dials, touch screens, check boxes, drop down menus, or other input means for selecting values or parameters or sets of values or parameters. Satiety Target Level Adjuster 397 is depicted comprising Satiety Target Level Adjuster Increase Input 398 and Satiety Target Level Adjuster Decrease Input 399, which facilitate the increase and decrease, respectively, of the selected satiety control parameter or other parameter related to satiety 398 which is being adjusted.

Metabolic Control Interface 390 facilitates the setting of control parameters for the neurological control system 999 for the control of metabolic rate / index 381, which is a quantification of at least one of metabolic rate, metabolic index, respiratory exchange ratio, heat production, carbon dioxide production, oxygen consumption, rate of weight change, rate of weight loss, other metric related to metabolism, and combination of metrics or parameters related to metabolism. Metabolic Control Interface 390 facilitates entry of a singularity or plurality of metabolic control inputs, which may comprise a vector of values, a set of scalars, a set of vectors, a collection of different parameters relating to various quantifications, qualities, or parameters related to metabolism, metabolic rate / index, energy expenditure, energy consumption, heat generation, food utilization, glucose consumption, glucose oxidation, oxygen consumption, carbon dioxide production, glycogen consumption, or other subjective or objective parameters or metrics related to metabolism. Metabolic Mode Display 400, depicted as an alphanumeric display, communicates to the user the metabolic control mode being programmed. This may specify a particular parameter for the autonomic modulation, including but not limited to stimulation waveform parameter, autonomic index, sympathetic index, parasympathetic index, metric of metabolism, a magnitude parameter relating to metabolism control, a temporal parameter relating to metabolism control, a parameter relating to timing of meals, a parameter relating to timing of stimulus relative to timing of meals, a parameter relating to magnitude of stimulation related to meals, or other parameter related to the control or modulation of metabolism which may comprise a quantification of at least one of metabolic rate, metabolic index, respiratory exchange ratio, heat production, carbon dioxide production, oxygen consumption, rate of weight change, rate of weight loss, other metric related to metabolism, and combination of metrics or parameters related to metabolism.

Metabolic Mode Adjuster 401 facilitates the selection of a metabolic mode to program and the setting of parameters for control of Metabolic Rate / Index 381. Metabolic Mode Adjuster 401 comprises Metabolic Mode Select Input 402, which enables the user to select among at least one metabolic control mode or metabolic control parameter or other parameter or mode related to metabolic control. Metabolic Mode Adjuster 401 further comprises Metabolic Mode Set Input 403, which enables the user to set or program the metabolic control mode or metabolic control parameter or other parameter or mode related to metabolic control.

Metabolic Actual Level Display 404 displays a current or actual metric of or function of metabolism or metabolic rate / index 381. This may be estimated from physiological parameter such as glucose level, insulin level, gastrointestinal physiological parameter, other parameter related to autonomic activity, including cardiac parameters such as heart rate and blood pressure, and respiratory parameters such as respiratory rate and carbon dioxide production and respiratory exchange ratio. Actual metabolic level or metabolic rate / index 381 may also be estimated from time since prior meal or time until next anticipated meal, or other parameter related to at least one of meal pattern, time since last meal, time until next expected meal, size of last meal, nutritional content of prior meals, caloric content of past meals, carbohydrate content of last meals, insulin response to prior meals, insulin level, history of prior insulin levels, cortisol level, history of prior cortisol levels, level of other hormones, history of prior levels of other hormones, other endocrinological parameters, history of other prior endocrinological parameters, circadian cycle, or other parameter or sets of parameters.

Metabolic Target Level Display 405 displays the target metabolic level, metabolic rate / index 381, metabolic control parameter value, or other parameter related to metabolism being adjusted by Metabolic Target Level Adjuster 406. Metabolic Target Level Adjuster 406 facilitates the adjustment of the target value of the selected metabolic control parameter or other parameter related to metabolism or to metabolic rate / index 381 which is selected for adjustment. Metabolic Target Level Adjuster 406 may be implemented in any of numerous ways without departing from the present invention; this includes the use of one or more knobs, rollers, dials, touch screens, check boxes, drop down menus, or other input means for selecting values or parameters or sets of values or parameters. Metabolic Target Level Adjuster 406 is depicted comprising Metabolic Target Level Adjuster Increase Input 407 and Metabolic Target Level Adjuster Decrease Input 408, which facilitate the increase and decrease, respectively, of the selected satiety control parameteror other parameter related to metabolism or metabolic rate / index 381which is being adjusted.

Metabolic Control Interface 390 allows setting of a single or plurality of target metabolic rates. Target metabolic rates and corresponding actual metabolic rates may comprise a control vector, with singularity or plurality of elements relating to various metabolic rates including but not limited to basal metabolic rate, postprandial metabolic rate, preprandial metabolic rate, daytime metabolic rate, night-time metabolic rate, preprandial (before meal) metabolic rate, postprandial (after meal) metabolic rate, periprandial (around meal time) metabolic rate, intermittent metabolic rate (set for a specific period of time), patterned metabolic rate, exercise metabolic rate, periodic elevated metabolic rate (for periods of high caloric burn), resting metabolic rate, or other times and patterns for metabolic rates. Neuromodulation signal (NMS) 998 is adjusted by Neurological control system 999, using open-loop control or closed-loop control or other control methodology such that actual metabolic rate is controlled to approach or be within a specified error of the target metabolic rate or to achieve the desired metabolic effect including but not limited to level of weight reduction, desired metabolic rate, Metabolic Target Level, and other parameter.

Other implementations for a Autonomic Neuromodulation Programmer 388 or equivalent module are encompassed within the present invention. These will become apparent to one skilled in the art. Variations include but are not limited to supersets or subsets of the modules and input and output interfaces described. Alternate means, apparatus, and methods for inputting and displaying this data using existing and future technologies which may become apparent to one skilled in the art and are included in tthe present invention. The input and display devices may be integrated and multiplexed to provide for a simpler and smaller interface, and this is include without departing form the present invention.

Figure 44 depicts one implementation of an Autonomic Neuromodulation Patient Interface 409, functionality of which is described under Figure 43, which is an implementation of at least one of Patient Interface Module 385, Supervisory Module 386, External Feedback Module 387, or other device which communicates with any portion of the neurological control system 999.

In the context of Figure 44, which is the Autonomic Neuromodulation Patient Interface 409 as one implementation of Patient Interface Module 385, Physician Lock 410 enables Physician to access Autonomic Neuromodulation Patient Interface 409 to program parameters, including the selection of which parameters the patient or the user or other caregiver may program or adjust, termed patient programmable parameters. The patient or user programmable parameters may be the full set or a subset of the physician programmable parameters. All or some of patient or user programmable parameters may further include limited ranges in which the patient or other caregiver may make adjustments for each of the parameters. This enables the physician to specify safe and efficacious parameter ranges and for the patient or other user or caregiver to make finer adjustments without requiring direct physician contact. Autonomic Neuromodulation Patient Interface 409 comprises Satiety Control User Interface 414, Metabolic Control User Interface 415, Physician Lock 410, and other components.

Autonomic Neuromodulation Patient Interface 409 enables the patient to program in anticipated meal times and expected levels of hunger. Additionally, Autonomic Neuromodulation Patient Interface 409 enables patient to specify current, recent, and anticipates level and patterns of satiety, hunger, or hunger pains. This can serve as an input to satiety control system to regulate satiety and sensation of hunger or hunger pains, in an open loop or a closed-loop manner. Patient and user are used interchangeable in this context.

Additionally, Autonomic Neuromodulation Patient Interface 409 enables patient to specify current, recent, and anticipates level and patterns of food or energy intake. This can serve as an input to metabolic control system to regulate metabolism and energy expenditure, energy consumption, oxygen consumption, lipolysis, fat metabolism, glucose metabolism, and other parameters of interest in an open loop or a closed-loop manner. Patient and user are used interchangeable in this context.

Numerous other implementations of this and the other interfaces described herein may be conceived and designed without departing from the present invention. This may be implemented using other input devices, buttons, switches, toggles, output displays, arrangements thereof, display technologies, liquid crystal displays (LCDs), light emitting diode (LED) displays, plasma displays, touch screens, software and hardware, and other existing or future technologies without departing from the present invention.

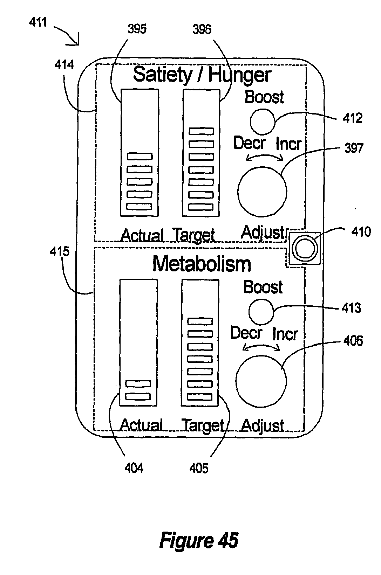

Figure 45 depicts an implementation of an Autonomic Neuromodulation Patient Interface 411, functionality of which is described under Figure 43, which is an implementation of at least one of Patient Interface Module 385, Supervisory Module 386, External Feedback Module 387, or other device which communicates with any portion of the neurological control system 999. Neuromodulation Patient Interface 411 comprises Satiety Control User Interface 414, Metabolic Control User Interface 415, Physician Lock 410, and other components

In the context of Figure 45, which is the Autonomic Neuromodulation Patient Interface 411 as one implementation of Patient Interface Module 385, Physician Lock 410 enables Physician to access Autonomic Neuromodulation Patient Interface 409 to program parameters, including the selection of which parameters the patient or the user or other caregiver may program or adjust, termed patient programmable parameters. The patient programmable parameters may be the full set or a subset of the physician programmable parameters. All or some of patient programmable parameters may further include limited ranges in which the patient or other caregiver may make adjustments for each of the parameters. This enables the physician to specify safe and efficacious parameter ranges and for the patient or other caregiver to make finer adjustments without requiring direct physician contact.

Autonomic Neuromodulation Patient Interface 411 enables the patient to program in anticipated meal times and expected levels of hunger. Additionally, Autonomic Neuromodulation Patient Interface 411 enables patient to specify current, recent, and anticipates level and patterns of satiety, hunger, or hunger pains. This can serve as an input to satiety control system to regulate satiety and sensation of hunger or hunger pains, in an open loop or a closed-loop manner. Patient and user are used interchangeable in this context.

Additionally, Autonomic Neuromodulation Patient Interface 411 enables patient to specify current, recent, and anticipates level and patterns of food or energy intake. This can serve as an input to metabolic control system to regulate metabolism and energy expenditure, energy consumption, oxygen consumption, lipolysis, fat metabolism, glucose metabolism, and other parameters of interest in an open loop or a closed-loop manner. Patient and user are used interchangeable in this context.

Further, Autonomic Neuromodulation Patient Interface 411 comprises Satiety/ Hunger Control Boost Input 412 which enables patient or user or caregiver to select, specify, or activate a preprogrammed or adaptive component of modulation to control satiety, sensation of hunger, or hunger pains, the latter two terms of which are interchangeable. With this functionality, if the patent feels a sensation of hunger in between meals or does not feel sufficient satiety or satiation following a meal, the patient may activate the Satiety / Hunger Control Boost Input 412 which may trigger neurological control system 999 to provide additional modulation to control satiety or achieve satiety or ameliorate the sensation of hunger or hunger pains. This may be accomplished by increasing or providing augmented modulation of sympathetic afferents as well as by increasing or providing augmented modulation of sympathetic efferents which may increase adrenergic stimulation and increase glucose levels, which also may induce satiety or ameliorate the hunger sensation or pains.

Further, Autonomic Neuromodulation Patient Interface 411 comprises Metabolic Control Boost Input 413 which enables patient or user or caregiver to select, specify, or activate a preprogrammed or adaptive component of modulation to control metabolism. With this functionality, if the patent feels a lack of energy, fatigue, lethargy, postprandial sleepiness, or otherwise wishes to select an increase in or augmentation of metabolism at any time including between meals, prior to a meal, following a meal, during a meal, during the daytime or during night time or at any other time, the patient may activate the Metabolic Control Boost Input 413, which may trigger neurological control system 999 to provide additional modulation to control or increase metabolism or metabolic rate or energy expenditure, energy consumption, oxygen consumption, lipolysis, fat metabolism, glucose metabolism, or other parameters of interest in an open loop or a closed-loop manner. Patient and user are used interchangeable in this context.

This may be accomplished by increasing or providing augmented modulation of sympathetic afferents as well as by increasing or providing augmented modulation of sympathetic efferents which may increase adrenergic stimulation and increase glucose levels, increase lipolysis, increase energy consumption, increase oxygen consumption, increase subjective energy level, ameliorate fatigue, ameliorate lethargy, ameliorate tiredness or sleepiness, ameliorate postprandial tiredness or sleepiness, or achieve other desired objective with respect to metabolic control.

Numerous other implementations of this and the other interfaces described herein may be conceived and designed without departing from the present invention. This may be implemented using other input devices, buttons, switches, toggles, output displays, arrangements thereof, display technologies, liquid crystal displays (LCDs), light emitting diode (LED) displays, plasma displays, touch screens, software and hardware, and other existing or future technologies without departing from the present invention. User may be the patient, family member, caregiver, nurse, physician, or other person acting on behalf of the user or patient to assist in the operation of neurological control system or a component thereof. User input means generally comprise input / output devices which facilitate user entry and reading of data. For the user, who is typically not a clinician, labeling and input / output means are generally simplified and are accompanied with labeling on the device and instruction manuals which are more easily understood by the non clinician lay person. The user input means may also be designed to be more rugged and able to withstand use in environments which may include temperature ad humidity extremes as well as environments which may include mechanical stresses and strains, vibration, accelerations and decelerations, mechanical shock, electrical shocks, exposure to water, exposure to corrosive substances, exposure to solids, liquids, gasses, biological agents, living organisms, and forms of energy or force which may require measures to for protection of the input device or interface module.

It will be appreciated by those skilled in the art that while the invention has been described above in connection with the particular embodiments and examples, the invention is not necessarily so limited, and that numerous other embodiments, examples uses, modifications, and departures from the embodiments, examples, and uses are intended to be encompassed by the claims attached hereto.

## Claims

1. Apparatus for the treatment of obesity, said apparatus comprising:
a neurological control system (999) configured to modulate the sympathetic nervous system, wherein said neurological control system (999) comprises a stimulating and recording unit (315) and a neuromodulator array (316);
a device for user control or operation of said neurological control system (999), said device comprising:
an autonomic neuromodulation programmer (388) comprising at least one of a metabolic control interface (390) and a satiety control interface (389); and
a communication link, whereby at least one of metabolic control input and satiety control input is communicated to the neurological control system (999) from said autonomic neuromodulation programmer (388);
wherein said satiety control interface (389) comprises:
a satiety mode adjuster (392) which facilitates the selection of a satiety mode; and
a satiety target level adjuster (397) which facilitates the adjustment of the target level of the selected satiety control parameter; and
wherein said metabolic control interface (390) comprises:
a metabolic mode adjuster (401) which facilitates the selection of a metabolic mode; and
a metabolic target level adjuster (406) which facilitates the adjustment of the target level of the selected metabolic control parameter;
wherein said stimulation and recording unit (315) generates at least one of a neuromodulation signal (998) which modulates metabolic rate and a neuromodulation control signal which modulates satiety;
wherein said neuromodulator array (316) is configured to communicate with a portion of the sympathetic nervous system.

2. The device of claim 1, wherein the autonomic neuromodulation programmer comprises said metabolic control interface and said satiety control interface, wherein said metabolic control interface facilitates at least one of the selection of metabolic rate and control of metabolic rate.

3. The device of claim 1, wherein the autonomic neuromodulation programmer comprises said metabolic control interface, and wherein the metabolic control interface facilitates input of at least one of:
basal metabolic rate;
at least one of daytime metabolic rate and nighttime metabolic rate;
at least one of postprandial metabolic rate, intermittent metabolic rate, elevated metabolic rate, and resting metabolic rate.

4. The device of claim 1, wherein the autonomic neuromodulation programmer comprises said satiety control interface,
wherein the satiety control interface facilitates input of at least one of:
resting satiety level;
at least one of preprandial satiety level, postprandial satiety level, periprandial satiety level, and inter-prandial satiety level;
at least one of daytime satiety level and night time satiety level;
wherein said satiety control interface facilitates selection of at least one of stimulation parameters and stimulation patterns for modulation of at least one afferent neural pathway by said neurological control system.

5. The device of any one of claims 1 to 4, wherein the autonomic neuromodulation programmer generates a metabolic control input which specifies modulation of autonomic efferent neurons, which modulate metabolic rate.

6. The device of any one of claims 1 to 4, wherein the autonomic neuromodulation programmer generates a metabolic control input which specifies modulation of sympathetic efferent neurons, which modulate metabolic rate.

7. The device of any one of claims 1 to 4, wherein the autonomic neuromodulation programmer generates a satiety control input which specifies modulation of autonomic efferent neurons, which modulate the sensation of satiety.

8. The device of any one of claims 1 to 4, wherein the autonomic neuromodulation programmer generates a satiety control input which specifies modulation of sympathetic afferent neurons.

9. The device of any one of claims 1 to 4, wherein the autonomic neuromodulation programmer generates at least one of:
a satiety control input, which comprises at least one of satiety boost input, hunger control boost input, input to trigger control of sensation of hunger, input to trigger control of hunger pains, and input to trigger the neurological control system to reduce craving for food, and
a metabolic control input, which comprises at least one of metabolic rate and metabolic boost input.

10. The device of any one of the preceding claims, wherein the autonomic neuromodulation programmer generates a metabolic control input, which comprises a parameter related to at least one of metabolic rate and energy expenditure.

11. The device of any one of the preceding claims, wherein the autonomic neuromodulation programmer generates a metabolic control input, which comprises a parameter related to at least one of glucose metabolism.

12. The device of any one of the preceding claims, wherein said metabolic control interface (390) comprises a metabolic actual level display (404) which displays an actual metric of or function of metabolism, metabolic rate or metabolic index that has been estimated from a physiological parameter;
wherein said satiety control interface (389) comprises a satiety actual level display (395) which displays an actual metric of or function of satiety that has been estimated from a physiological parameter.

13. The device of any one of the preceding claims, wherein said device is configured to modulate sympathetic index to control at least one of metabolic rate, body temperature, food intake, blood pressure, heart rate, respiratory gas flow, pulmonary function parameters, cardiac parameters, cardiovascular parameters and vascular parameters.

14. The device of any one of the preceding claims, wherein said metabolic control interface (390) comprises:
a metabolic target level display (405) which displays the target metabolic level; wherein said satiety control interface (389) comprises:
a satiety target level display (396) which displays the target satiety level.

15. The device of any one of the preceding claims, wherein said device is configured to be programmed with meal times and/or anticipated levels or patterns of hunger, such that the satiety control system can regulate satiety in an open loop or a closed loop manner.

16. The device of any one of the preceding claims in combination with a neurological control system (999), wherein said neurological control system (999) comprises a stimulating and recording unit (315) that includes a physiological sensor array (319).

17. The device of claim 16, wherein said physiological sensor array (319) is configured to sense a physiological parameter comprising at least one of temperature, heart rate, heart rate variability, blood pressure, respiratory rate, respiratory function parameters and pressures, metabolic rate, respiratory quotient, glucose level, insulin level, organ perfusion.

## Patentansprüche

1. Vorrichtung zur Behandlung von Adipositas, wobei die Vorrichtung umfasst:
ein neurologisches Steuersystem (999), das so konfiguriert ist, dass es das sympathische Nervensystem moduliert, wobei das neurologische Steuersystem (999) eine Stimulations- und Aufzeichnungseinheit (315) und eine Neuromodulatoranordnung (316) umfasst;
eine Einrichtung für die Benutzersteuerung oder den Betrieb des neurologischen Steuersystems (999), wobei die Einrichtung umfasst:
einen autonomen Neuromodulationsprogrammierer (388), der zumindest eines von einer Stoffwechselsteuerschnittstelle (390) und einer Sättigungssteuerschnittstelle (389) umfasst; und
eine Kommunikationsverbindung, wobei zumindest eines einer Stoffwechselsteuereingabe und einer Sättigungssteuereingabe vom autonomen Neuromodulationsprogrammierer (388) an das neurologische Steuersystem (999) kommuniziert wird;
wobei die Sättigungssteuerschnittstelle (389) umfasst:
einen Sättigungsmoduseinsteller (392), der das Auswählen eines Sättigungsmodus erleichtert; und
einen Zielsättigungshöheneinsteller (397), der das Einstellen der Zielhöhe des ausgewählten Sättigungssteuerparameters erleichtert; und
wobei die Stoffwechselsteuerschnittstelle (390) umfasst:
einen Stoffwechselmoduseinsteller (401), der das Auswählen eines Stoffwechselmodus erleichtert; und
einen Zielstoffwechselhöheneinsteller (406), der das Einstellen der Zielhöhe des ausgewählten Stoffwechselsteuerparameters erleichtert;
wobei die Stimulations- und Aufzeichnungseinheit (315) zumindest eines von einem Neuromodulationssignal (998), das die Stoffwechselrate moduliert, und einem Neuromodulationssteuersignal, das die Sättigung moduliert, erzeugt;
wobei die Neuromodulatoranordnung (316) so konfiguriert ist, dass sie mit einem Teil des sympathischen Nervensystems kommuniziert.

2. Einrichtung nach Anspruch 1, wobei der autonome Neuromodulationsprogrammierer die Stoffwechselsteuerschnittstelle und die Sättigungssteuerschnittstelle umfasst, wobei die Stoffwechselsteuerschnittstelle zumindest eines des Auswählen der Stoffwechselrate und des Steuerns der Stoffwechselrate erleichtert.

3. Einrichtung nach Anspruch 1, wobei der autonome Neuromodulationsprogrammierer die Stoffwechselsteuerschnittstelle umfasst und wobei die Stoffwechselsteuerschnittstelle eine Eingabe von zumindest einem des Folgenden erleichtert:
der Stoffwechselbasisrate;
zumindest einem einer Stoffwechselrate am Tag und einer Stoffwechselrate in der Nacht;
zumindest einem einer postprandialen Stoffwechselrate, einer zwischenzeitlichen Stoffwechselrate, einer erhöhten Stoffwechselrate und einer Stoffwechselrate im Ruhezustand.

4. Einrichtung nach Anspruch 1, wobei der autonome Neuromodulationsprogrammierer die Sättigungssteuerschnittstelle umfasst,
wobei die Sättigungssteuerschnittstelle die Eingabe von zumindest einem des Folgenden erleichtert:
einer Sättigungshöhe im Ruhezustand;
zumindest einem einer präprandialen Sättigungshöhe, einer postprandialen Sättigungshöhe, einer periprandialen Sättigungshöhe und einer interprandialen Sättigungshöhe;
zumindest einem einer Sättigungshöhe am Tag und einer Sättigungshöhe in der Nacht;
wobei die Sättigungssteuerschnittstelle das Auswählen von zumindest einem von Stimulationsparametern und Stimulationsmustern für eine Modulation zumindest eines afferenten neuronalen Pfads durch das neurologische Steuersystem erleichtert.

5. Einrichtung nach einem der Ansprüche 1 bis 4, wobei der autonome Neuromodulationsprogrammierer eine Stoffwechselsteuereingabe erzeugt, die eine Modulation von autonomen efferenten Neuronen spezifiziert, die die Stoffwechselrate modulieren.

6. Einrichtung nach einem der Ansprüche 1 bis 4, wobei der autonome Neuromodulationsprogrammierer eine Stoffwechselsteuereingabe erzeugt, die eine Modulation von sympathischen efferenten Neuronen spezifiziert, die die Stoffwechselrate modulieren.

7. Einrichtung nach einem der Ansprüche 1 bis 4, wobei der autonome Neuromodulationsprogrammierer eine Sättigungssteuereingabe erzeugt, die eine Modulation von autonomen efferenten Neuronen spezifiziert, die das Sättigungsgefühl modulieren.

8. Einrichtung nach einem der Ansprüche 1 bis 4, wobei der autonome Neuromodulationsprogrammierer eine Sättigungssteuereingabe erzeugt, die eine Modulation von sympathischen afferenten Neuronen spezifiziert.

9. Einrichtung nach einem der Ansprüche 1 bis 4, wobei der autonome Neuromodulationsprogrammierer zumindest eines des Folgenden erzeugt:
eine Sättigungssteuereingabe, die zumindest eines von einer Sättigungsschubeingabe, Hungersteuerschubeingabe, Eingabe zum Auslösen der Steuerung des Hungergefühls, Eingabe zum Auslösen der Steuerung von Hungerschmerzen und Eingabe zum Auslösen des neurologischen Steuersystems, um das Verlangen nach Nahrung zu verringern, und
eine Stoffwechselsteuereingabe, die zumindest eines von Stoffwechselrate und Stoffwechselschubeingabe umfasst.

10. Einrichtung nach einem der vorstehenden Ansprüche, wobei der autonome Neuromodulationsprogrammierer eine Stoffwechselsteuereingabe erzeugt, die einen Parameter umfasst, der sich auf zumindest eines von Stoffwechselrate und Energieverbrauch bezieht.

11. Einrichtung nach einem der vorstehenden Ansprüche, wobei der autonome Neuromodulationsprogrammierer eine Stoffwechselsteuereingabe erzeugt, die einen Parameter umfasst, der sich auf zumindest eines von Glukosestoffwechsel bezieht.

12. Einrichtung nach einem der vorstehenden Ansprüche, wobei die Stoffwechselsteuerschnittstelle (390) eine Anzeige (404) für die tatsächliche Stoffwechselhöhe umfasst, die eine tatsächliche Kennzahl oder Funktion von Stoffwechsel, Stoffwechselrate oder Stoffwechselindex anzeigt, die anhand eines physiologischen Parameters geschätzt wurde;
wobei die Sättigungssteuerschnittstelle (389) eine Anzeige (395) für die tatsächliche Sättigungshöhe umfasst, die eine tatsächliche Kennzahl oder Funktion der Sättigung anzeigt, die anhand eines physiologischen Parameters geschätzt wurde.

13. Einrichtung nach einem der vorstehenden Ansprüche, wobei die Einrichtung so konfiguriert ist, dass sie den sympathischen Index moduliert, um zumindest eines von Stoffwechselrate, Körpertemperatur, Nahrungsaufnahme, Blutdruck, Herzfrequenz, Atemgasströmung, Lungenfunktionsparametern, Herzparametern, Herz-Kreislauf-Parametern und Gefäßparametern zu steuern.

14. Einrichtung nach einem der vorstehenden Ansprüche, wobei die Stoffwechselschnittstelle (390) umfasst:
eine Anzeige (405) für die Zielstoffwechselhöhe, die die Zielstoffwechselhöhe anzeigt;
wobei die Sättigungssteuerschnittstelle (389) umfasst:
eine Anzeige (396) für die Zielsättigungshöhe, die die Zielsättigungshöhe anzeigt.

15. Einrichtung nach einem der vorstehenden Ansprüche, wobei die Einrichtung so konfiguriert ist, dass sie mit Essenszeiten und/oder antizipierten Hungerhöhen oder -mustern programmiert wird, so dass das Sättigungssteuersystem die Sättigung auf die Weise eines offenen Regelkreises oder eines geschlossenen Regelkreises regulieren kann.

16. Einrichtung nach einem der vorstehenden Ansprüche in Kombination mit einem neurologischen Steuersystem (999), wobei das neurologische Steuersystem (999) eine Stimulations- und Aufzeichnungseinheit (315) umfasst, die eine physiologische Sensoranordnung (319) enthält.

17. Einrichtung nach Anspruch 16, wobei die physiologische Sensoranordnung (319) so konfiguriert ist, dass sie einen physiologischen Parameter erfasst, der zumindest eines von Temperatur, Herzfrequenz, Variabilität der Herzfrequenz, Blutdruck, Atemfrequenz, Atemfunktionsparametern und -drücken, Stoffwechselrate, Atemquotient, Glukosespiegel, Insulinspiegel bzw. Organperfusion umfasst.

## Revendications

1. Appareil pour le traitement de l'obésité, ledit appareil comprenant :
un système de régulation neurologique (999) configuré pour moduler le système nerveux sympathique, ledit système de régulation neurologique (999) comprenant une unité de stimulation et d'enregistrement (315) et un réseau neuromodulateur (316) ;
un dispositif pour la commande ou l'activation par l'utilisateur dudit système de régulation neurologique (999), ledit dispositif comprenant :
un programmateur de neuromodulation autonome (388) comprenant au moins une interface de régulation métabolique (390) et une interface de régulation de satiété (389) ; et
une liaison de communication, grâce à laquelle au moins l'une d'une entrée de régulation métabolique et d'une entrée de régulation de satiété est communiquée au système de régulation neurologique (999) depuis ledit programmateur de neuromodulation autonome (388) ;
ladite interface de régulation de satiété (389) comprenant :
un ajusteur de mode de satiété (392) qui facilite la sélection d'un mode de satiété ; et
un ajusteur de niveau cible de satiété (397) qui facilite l'ajustement du niveau cible du paramètre de régulation de satiété sélectionné ; et
ladite interface de régulation métabolique (390) comprenant :
un ajuster de mode métabolique (401) qui facilite la sélection d'un mode métabolique ; et
un ajusteur niveau cible métabolique (406) qui facilite l'ajustement du niveau cible du paramètre de régulation métabolique sélectionné ;
dans lequel ladite unité de stimulation et d'enregistrement (315) génère au moins l'un d'un signal de neuromodulation (998) qui module le rythme métabolique et d'un signal de commande de neuromodulation qui module la satiété ;
dans lequel ledit réseau neuromodulateur (316) est configuré pour communiquer avec une partie du système nerveux sympathique.

2. Dispositif de la revendication 1, dans lequel le programmateur de neuromodulation autonome comprend ladite interface de régulation métabolique et ladite interface de régulation de satiété, ladite interface de régulation métabolique facilitant au moins l'une de la sélection du rythme métabolique et de la commande du rythme métabolique.

3. Dispositif de la revendication 1, dans lequel le programmateur de neuromodulation autonome comprend ladite interface de régulation métabolique, et dans lequel l'interface de régulation métabolique facilite l'entrée d'au moins l'un des éléments suivants :
le rythme métabolique de base ;
au moins l'un du rythme métabolique diurne et du rythme métabolique nocturne ;
au moins l'un du rythme métabolique postprandial, du rythme métabolique intermittent, du rythme métabolique élevé et du rythme métabolique au repos.

4. Dispositif selon la revendication 1, dans lequel le programmateur de neuromodulation autonome comprend ladite interface de régulation de satiété,
l'interface de régulation de satiété facilitant l'entrée d'au moins un des éléments suivants :
le niveau de satiété au repos ;
au moins l'un du niveau de satiété préprandial, du niveau de satiété postprandial, du niveau de satiété périprandial et du niveau de satiété interprandial ;
au moins l'un du niveau de satiété diurne et du niveau de satiété nocturne ;
ladite interface de régulation de satiété facilitant au moins la sélection d'au moins les uns des paramètres de stimulation et de configurations de stimulation pour la modulation d'au moins une voie neuronale afférente par ledit système de régulation neurologique.

5. Dispositif de l'une quelconque des revendications 1 à 4, dans lequel le programmateur de neuromodulation autonome génère une entrée de régulation métabolique qui spécifie la modulation de neurones efférents autonomes, qui modulent le rythme métabolique.

6. Dispositif de l'une quelconque des revendications 1 à 4, dans lequel le programmateur de neuromodulation autonome génère une entrée de régulation métabolique qui spécifie la modulation de neurones sympathiques efférents, qui modulent le rythme métabolique.

7. Dispositif de l'une quelconque des revendications 1 à 4, dans lequel le programmateur de neuromodulation autonome génère une entrée de régulation métabolique qui spécifie la modulation de neurones efférents autonomes, qui modulent la sensation de satiété.

8. Dispositif de l'une quelconque des revendications 1 à 4, dans lequel le programmateur de neuromodulation autonome génère une entrée de régulation métabolique qui spécifie la modulation de neurones sympathiques efférents.

9. Dispositif de l'une quelconque des revendications 1 à 4, dans lequel le programmateur de neuromodulation autonome génère au moins l'un des éléments suivants :
une entrée de régulation de satiété, qui comprend au moins l'une d'une entrée de stimulation de satiété, d'une entrée de stimulation de régulation de la faim, d'une entrée permettant de déclencher une régulation de la sensation de faim, d'une entrée permettant de déclencher une régulation des douleurs dues à la faim, et d'une entrée permettant de déclencher la le système de régulation neurologique afin de réduire la fringale, et
une entrée de régulation métabolique, qui comprend au moins l'un du rythme métabolique et d'une entrée de stimulation métabolique.

10. Dispositif selon l'une quelconque des revendications précédentes, dans lequel le programmateur de neuromodulation autonome génère une entrée de régulation métabolique, qui comprend un paramètre lié à au moins l'un du rythme métabolique et d'une dépense d'énergie.

11. Dispositif selon l'une quelconque des revendications précédentes, dans lequel le programmateur de neuromodulation autonome génère une entrée de régulation métabolique, qui comprend un paramètre lié à au moins l'un du métabolisme du glucose.

12. Dispositif selon l'une quelconque des revendications précédentes, dans lequel ladite interface de régulation métabolique (390) comprend un affichage de niveau métabolique effectif (404) qui affiche une mesure effective ou fonction du métabolisme, du rythme métabolique ou de l'indice métabolique qui a été estimé à partir d'un paramètre physiologique ;
dans lequel ladite interface de régulation de satiété (389) comprend un affichage de niveau de satiété effectif (395) qui affiche une mesure effective ou fonction de la satiété qui a été estimée à partir d'un paramètre physiologique.

13. Dispositif selon l'une quelconque des revendications précédentes, dans lequel ledit dispositif est configuré pour moduler un indice sympathique afin de réguler au moins l'un du rythme métabolique, de la température corporelle, de l'ingestion de nourriture, de la tension artérielle, du rythme cardiaque, du débit respiratoire, des paramètres de la fonction pulmonaire, des paramètres cardiaques, des paramètres cardiovasculaires et des paramètres vasculaires.

14. Dispositif selon l'une quelconque des revendications précédentes, dans lequel ladite interface de régulation métabolique (390) comprend :
un affichage de niveau cible métabolique (405) qui affiche le niveau métabolique cible ;
dans lequel ladite interface de régulation de satiété (389) comprend :
un affichage de niveau cible de satiété (396) qui affiche le niveau de satiété cible.

15. Dispositif selon l'une quelconque des revendications précédentes, dans lequel ledit dispositif est configuré pour être programmé avec les heures de repas et/ou des niveaux ou configurations anticipés de faim, de sorte que le système de régulation de satiété puisse réguler la satiété en boucle ouverte ou en boucle fermée.

16. Dispositif selon l'une quelconque des revendications précédentes en combinaison avec un système de régulation neurologique (999), ledit système de régulation neurologique (999) comprenant une unité de stimulation et d'enregistrement (315) qui inclut un réseau de capteurs physiologiques (319).

17. Dispositif selon la revendication 16, dans lequel le réseau de capteurs physiologiques (319) est configuré pour détecter un paramètre physiologique comprenant au moins l'un de la température, du rythme cardiaque, d'une variabilité du rythme cardiaque, de la tension artérielle, de la fréquence respiratoire, de paramètres et pressions de la fonction respiratoire, du rythme métabolique, du quotient respiratoire, de la glycémie, du taux d'insuline, d'une perfusion d'organe.
